# EUROPEAN PATENT APPLICATION

(11) **EP 4 480 961 A1**
(43) Date of publication of application: **25.12.2024**
(21) Application number: 23382646.0
(22) Date of filing: 23.06.2023
(51) Int. Cl.: C07K 14/47, C07K 14/005, C07K 14/195, C12N 9/14, C12N 15/62, A01K 63/04

(54) **PATHOGEN TRAPPING SYSTEMS AND RELATED AQUACULTURAL USES**

(71) Applicant: Consejo Superior De Investigaciones Científicas, 28006 Madrid (ES); Smartwater Planet S. L., 28232 Las Rozas, Madrid (ES)
(72) Inventor: Carrión Vázquez, Mariano Sixto, Madrid (ES); Vaquero Morales, María Eugenia, Madrid (ES); Pardo Pardo, Wilmer Alfonso, Madrid (ES)
(74) Representative: Pons

(57) **Abstract**

The present invention refers to a chimeric protein and related systems, based on two different components: first, a polypeptide comprising a low-complexity region and second, a binder polypeptide comprising a pathogen recognition region, as well as pathogen detection and pathogen control related methods and uses, and their applications in aquaculture.

## Description

The invention belongs to the field of protein engineering and pathogen detection. In particular, the present invention relates to a chimeric protein based on two different polypeptide elements, which takes advantage of their functional properties for trapping pathogens in water. Related systems and applications, including other aquacultural uses are also described.

### BACKGROUND ART

Aquatic biodiversity is essential to ensure food and nutritional security of humankind. Overfishing poses a major threat to aquatic ecosystems and still results insufficient to meet the current fish consumption needs of a rapidly growing global population. In this context, aquaculture has emerged as a more sustainable alternative, which has experienced an exponential growth of resources invested in the recent years. In 2018, of all global fish consumption, farmed fish accounted for 45.81%, and by 2030 this figure is expected to grow by 30%. This means that in less than 10 years, aquaculture will be the main supplier of fish for human consumption as it has been envisioned by the so-called "Blue Revolution", which is focused on promoting the farming of marine species and that is increasingly dependent on digital tools and new technologies.

Although fish farm production possesses many advantages over capture fisheries, there are still many challenges to overcome. In order to meet the fast-growing demand, large-scale intensive farming is necessary. However, in these conditions fish are often overcrowded in high concentrations under relatively artificial environments, which favors the emergence and spread of pathogens outbreaks. Moreover, climate change worsens the situation by encouraging the continued rise of new pathogenic species. Disease outbreaks impact drastically not only on fish health and production, but also on the environment and on the profitability of aquaculture. Annual loses due to this cause are estimated in more than 6 billions (USD), which positions pathogen outbreaks as the main challenge to overcome.

The sustainable solutions to prevent and control the outbreaks that are used in ecological aquaculture (e.g., quarantines, lower fish density, better health management) cannot be transferred to intensive farming without a drastic and unaffordable reduction of fish production. In intensive aquaculture the general strategies to control and prevent pathogen infections are based mainly on the use of vaccines and antibiotics, which also have their own important drawbacks. Vaccines are expensive and target individual pathogenic strains, becoming useless as infectious microorganisms mutate and evolve. On the other hand, antibiotics pose a potential risk to both fish and consumer health, as they contribute to the emergence of super-resistant bacteria. Thus, the current technological paradigm focuses mainly on disease prevention through the direct action on fish by using either vaccines or broad-spectrum antibiotics.

Lately, alternative approaches for the prevention of pathogen diseases based on the use of multi-functional systems and fusion proteins have been developed.

CN1 13683707A discloses an antigen fusion protein that can be used for subsequent development of triple subunit vaccines and diagnostic kits comprising three proteins from three different bacteria.

US2004258664A1 discloses a multi-functional targeting complex for inducing specific phagocytosis of, at least, one target pathogenic bacteria causing ulcerative disease in fish infected by said bacteria, wherein said complex comprises at least one target recognition component comprising a molecule which specifically binds to *Aeromonas salmonicida* pathogenic bacteria, and an active component comprising phagocytosis inducing agent. Therapeutic and prevention uses for pathogenic disorders, such as furunculosis in fish, based on this complex are also described in said document.

To ensure the longevity and sustainability of aquaculture, the development of new strategies to control and prevent pathogen infections is of great relevance. Therefore, in view of the state of the art, alternative systems for pathogen detection or pathogen control are needed, in particular with regards to aquaculture.

### DESCRIPTION OF THE INVENTION

Inventors have designed innovative protein engineering systems, which trap or block targeted pathogens taking advantage of properties related to two polypeptides: (i) a first polypeptide comprising a low-complexity region (LCR) with self-assembling properties that drives either liquid-liquid phase separation (LLPS), forming liquid droplets, or liquid-solid phase separation (LSPS), forming hydrogels; (ii) a second polypeptide comprising a recognition region for a pathogen (also named in the present document as "binder element"), driving recognition of a targeted pathogen. The combination of the two polypeptides as a fusion protein in the chimera is not just additive since the two components could influence each other. The selected chimeras are the ones able to recognize and trap the targeted pathogens in the desire conditions (marine and/or fresh water).

Based on this chimera design, several related products have been developed by the inventors of the invention with application in pathogen detection or pathogen control, including related uses in aquaculture.

Basically, the inventors have developed two types of formulations. One product formulation takes advantage of the liquid-liquid phase separation phenomenon, where a protein moiety forming liquid droplets acts as a flocculant solution added directly to the water to block the targeted pathogen by the recognition and binding mediated by the protein binder moiety of the chimera. Pathogens are either trapped into liquid-droplets or coated by the chimera, and then can flocculate and can later be eliminated, having application in closed system aquaculture (CSA). The second formulation takes advantage of liquid to solid phase separation (LSPS) where a hydrogel is formed by another chimeric protein being able to retain pathogens as water flows over it, having application in CSA and in open system aquaculture (OSA).

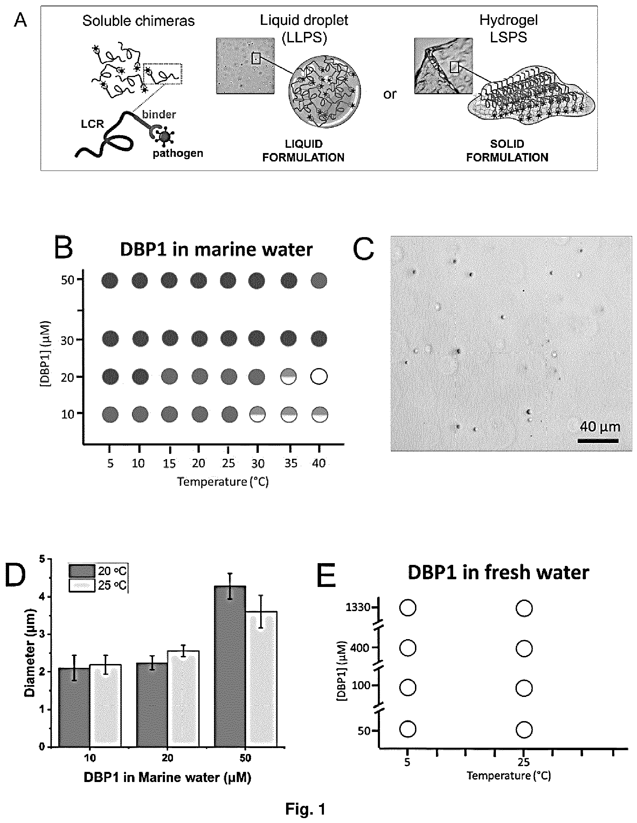

Inventors have generated some specific chimeric proteins, based on the above-mentioned approach, as proof of concepts, as shown in Fig. 1A.

Different aspects of the invention have been developed by the inventors of the invention, which will be described below.

### Chimeric protein of the invention

An aspect of the invention relates to a chimeric protein, hereinafter "the chimeric protein of the invention" comprising:
- a first polypeptide which comprises a low-complexity region (LCR), preferably wherein said first polypeptide is DBP1 or HP1α; and
- a second polypeptide which comprises a recognition region for a pathogen or for a toxin.

The term "chimeric protein", which can also be referred to as "fusion protein", can be defined as proteins created through the joining of two or more gene products which originally code for different proteins. Translation of this recombinant gene results in a single fusion protein comprising two or more polypeptides, with functional properties derived from each of the original polypeptides. In the present invention, the chimeric protein of the invention comprises two polypeptides, whose combination in a single protein allows pathogen trapping or coating as described previously.

### First polypeptide of the chimeric protein of the invention

As mentioned above, the first polypeptide comprises an LCR, preferably wherein said first polypeptide is DBP1 or HP1α.

The term "low-complexity region (LCR)" as used in the present invention, refers to protein sequences that containing little diversity in their amino acid composition deviate from the common residues found in proteins and usually contain a higher proportion of specific amino acids. The degree of diversity shown by these regions may vary, ranging from regions comprising few different amino acids, to those comprising just one, the amino acid positions within these regions being either loosely clustered, irregularly spaced, or periodic. Regarding protein structure, LCRs mostly have a disordered conformation and they are believed to play pivotal roles across a wide range of biological functions, some of whose mechanisms have been extensively documented. In the present invention, the LCR drives liquid-liquid phase separation (LLPS) or liquid-to-solid phase separation (LSPS) forming hydrogels.

The criteria followed to identify LCRs is mainly based on the diversity of its amino acid composition. They can be computationally identified from their sequences using various algorithms. The first one developed to specifically detect LCRs was SEG based on their so-called Shannon entropy (Wootton J.C., Federhen S. Statistics of local complexity in amino acid sequences and sequence databases. Comput. Chem. 1993; 17:149-163) and since then many more have been established. Lately some comprehensive low complexity tools such as LCR-eXXXplorer and PlaToLoCo have been developed to integrate the previously existing platforms. Regarding LCRs able to perform LLPS or LSPS some databases based on empirical results such as LLPSDB are valuable tools.

LLPS is a mechanism used by eukaryotic cells to efficiently optimize their biochemical reactions through the spatio-temporal separation into membraneless organelles based on biomolecular condensates. It is based on specific biomolecules that experience a phase transition similar to the well-known oil-in-water phenomenon. This phase separation is driven by multivalent homo and heterotypic interactions between small repeating protein motifs and often with nucleic acids. Many of these condensates are highly enriched in proteins containing both structurally undefined domains called intrinsically disordered proteins/regions (IDPs/IDRs) and tracts with lower diversity of residues, i.e. the so-called LCRs. These proteins comprise polar, charged and aromatic amino acids that stablish weak multivalent interactions which trigger phase separation. LLPS dynamics starts with the monomeric proteins self-assembling into a dense phase forming liquid droplets that start fusing and coalescing into bigger and fewer dynamic droplets. Eventually, droplets can mature into less mobile structures that undergo liquid-to-solid transition evolving into hydrogels, i.e. solid-like states where molecular diffusion is limited by a stronger network of interactions.

In a preferred embodiment of the chimeric protein of the invention, alone or in combination with other preferred embodiments, the first polypeptide is an intrinsically disordered protein (IDP).

Inventors have tested LLPS properties of DBP1 and HP1α, which are proteins comprising LCR regions, showing both of them LLPS in fish farm conditions (i.e. marine and fresh water, respectively).

Thus, in a more preferred embodiment of the chimeric protein of the invention, the first polypeptide is DBP1.

In the present invention, DBP1 (DEAD-box helicase 1) refers to a polypeptide which comprises, or consists of, an amino acid sequence with a sequence similarity of, at least, 80, 81, 82, 83, 84, 85, 86, 87, 88, 89, 90, 91, 92, 93, 94, 95, 96, 97, 98, or 99% to SEQ ID NO: 1, wherein sequence similarity is computed according to algorithm EMBOSS Needle using the BLOSUM62 matrix, having a Gap Open penalty of 10.0 and a Gap Extend penalty of 0.5.

SEQ ID NO 1:

MADLPQKVSNLSINNKENGGGGGKSSYVPPHLRSRGKPSFERRSPKQKDKVTGG
DFFRRAGRQTGNNGGFFGFSKERNGGTSANYNRRGSSNYKSSGNRWVNGKHIP
GPKNAKLQKAELFGVHDDPDYHSSGIKFDNYDNIPVDASGKDVPEPILGGRTRGG
GGFFNSRNNGSRDYRKHGGNGSFGSTRPRNTGTSNWGSIGGGFRNDNEKNGYG
NSNASWW

More preferably, the first polypeptide comprises, or consists of, an amino acid sequence with a sequence identity of at least, 80, 81, 82, 83, 84, 85, 86, 87, 88, 89, 90, 91, 92, 93, 94, 95, 96, 97, 98, or 99% to SEQ ID NO: 1.

SEQ ID NO 1 corresponds to DBP1 amino acid sequence in *Saccharomyces cerevisae.* The polypeptide comprises 222 residues of the accession number CAA39465.1 in the GenBank database. In a still more preferred embodiment of the chimeric protein of the invention, the first polypeptide comprises, or consists of, sequence SEQ ID NO 1.

In another more preferred embodiment of the chimeric protein of the invention, the first polypeptide is HP1α.

In the present invention, HP1α (Heterochromatin Protein 1α) refers to a polypeptide which comprises, or consists of, an amino acid sequence with a sequence similarity of, at least, 80, 81, 82, 83, 84, 85, 86, 87, 88, 89, 90, 91, 92, 93, 94, 95, 96, 97, 98, or 99% to SEQ ID NO: 100. Wherein sequence similarity is computed according to algorithm EMBOSS Needle using the BLOSUM62 matrix, having a Gap Open penalty of 10.0 and a Gap Extend penalty of 0.5.

More preferably, the first polypeptide comprises an amino acid sequence with a sequence identity of at least, 80, 81, 82, 83, 84, 85, 86, 87, 88, 89, 90, 91, 92, 93, 94, 95, 96, 97, 98, or 99% to SEQ ID NO: 100.

SEQ ID NO: 100:

GKKTKRTADSSSSEDEEEYVVEKVLDRRVVKGQVEYLLKWKGFSEEHNTWEPEK
NLDCPELISEFMKKYKKMKEGENNKPREKSESNKRKSNFSNSADDIKSKKKREQS
NDIARGFERGLEPEKIIGATDSCGDLMFLMKWKDTDEADLVLAKEANVKCPQIVIAF
YEERLTWHAYPEDAENKEKETAKS

SEQ ID NO: 100 corresponds to HP1α amino acid sequence in *Homo sapiens* (accession number: AAC50553.1 in the GenBank database).

In a particular embodiment when the first polypeptide is HP1α, alone or in combination with other preferred embodiments, said first polypeptide comprises, or consists of, an amino acid sequence with a sequence similarity of, at least, 80, 81, 82, 83, 84, 85, 86, 87, 88, 89, 90, 91, 92, 93, 94, 95, 96, 97, 98, or 99% to SEQ ID NO: 2, wherein sequence similarity is computed according to algorithm EMBOSS Needle using the BLOSUM62 matrix, having a Gap Open penalty of 10.0 and a Gap Extend penalty of 0.5. More preferably, the first polypeptide comprises, or consists of, an amino acid sequence with a sequence identity of, at least, 80, 81, 82, 83, 84, 85, 86, 87, 88, 89, 90, 91, 92, 93, 94, 95, 96, 97, 98, or 99% to SEQ ID NO: 2.

SEQ ID NO: 2
MGKKTKRTADSSSSEDEEEYVVEKVLDRRMVKGQVEYLLKWKGFSEEHNTWEPE
KNLDCPELISEFMKKYKKMKEGENNKPREKSEGNKRKSSFSNSADDIKSKKKREQ

SNDIARGFERGLEPEKIIGATDSCGDLMFLMKWKDTDEADLVLAKEANVKCPQIVIA
FYEERLTWHAYPEDAENKEKESAKS

SEQ ID NO: 2 corresponds to HP1α amino acid sequence in *Mus musculus* (also called in the present document as "mHP1α"). The polypeptide comprises 191 residues of the accession number NP_001070257.1. In a still more preferred embodiment of the chimeric protein of the invention, the first polypeptide comprises, or consists of, amino acid sequence SEQ ID NO 2.

Inventors have also performed the phosphorylation of human HP1α (HP1αPhos). Human HP1α differs from the murine version (SEQ ID NO: 2) in 5 residues (deletion of M1, V29M, S86G, N92S, T187S), and as previously mentioned, its accession number is AAC50553.1 in the GenBank database. The phosphorylation process led to a version of the chimeric protein able to experience liquid-solid phase separation (LSPS).

Thus, in another particular embodiment when the first polypeptide is HP1α, alone or in combination with other preferred embodiments, said first polypeptide is a phosphorylated HP1α (hereinafter, also called as "the phosphorylated version of HP1α of the invention", "the phosphorylated HP1α of the invention", "HP1αPhos of the invention" or simply "HP1αPhos").

Techniques and methods to phosphorylate polypeptides are well known in the state of the art, such as, but without limitation to, the use of kinase enzymes after a protein purification step or the co-transformation before production stage, in *E. coli.* In the present invention, a co-transformation approach was used, which consists in introducing two plasmids into the expression host organism, *E. coli.* One of those plasmids contains a sequence of the suited kinase, particularly a casein kinase II. It was also used a pRSTDuet-1 plasmid with the two subunits of that kinase with co-expression (simultaneously) with the protein of interest by performing the phosphorylation process into the bacteria. However, other techniques and methods to phosphorylate polypeptides can be used.

In order to avoid phosphorylation events on unwanted amino acid residues, some amino acid residues may be mutated or changed compared to amino acid sequence SEQ ID NO: 100, particularly, mutations S91A, S94A, S96A obtaining SEQ ID NO 3.

Thus, a preferred embodiment, alone or in combination with other preferred embodiments, provides the chimeric protein of the invention, wherein the first polypeptide is a phosphorylated HP1α, and wherein the HP1αPhos comprises, or consists of, an amino acid sequence with a sequence similarity and/or identity of at least, 80, 81, 82, 83, 84, 85, 86, 87, 88, 89, 90, 91, 92, 93, 94, 95, 96, 97, 98, or 99% to SEQ ID NO: 3, wherein sequence similarity is computed according algorithm EMBOSS Needle using the BLOSUM62 matrix, having a Gap Open penalty of 10.0, a Gap Extend penalty of 0.5.

Therefore, in this more preferred embodiment the HP1αPhos of the invention comprises, or consists of, an amino acid sequence with a sequence similarity and/or identity of at least, 80, 81, 82, 83, 84, 85, 86, 87, 88, 89, 90, 91, 92, 93, 94, 95, 96, 97, 98, or 99% to SEQ ID NO: 3, wherein said amino acid sequence is phosphorylated.

More preferably, the HP1αPhos of the invention comprises, or consists of, amino acid sequence SEQ ID NO: 3, wherein said amino acid sequence is phosphorylated.

SEQ ID NO: 3
GKKTKRTADSSSSEDEEEYVVEKVLDRRVVKGQVEYLLKWKGFSEEHNTWEPEK
NLDCPELISEFMKKYKKMKEGENNKPREKSESNKRKANFANAADDIKSKKKREQS
NDIARGFERGLEPEKIIGATDSCGDLMFLMKWKDTDEADLVLAKEANVKCPQIVIAF
YEERLTWHAYPEDAENKEKETAKS

In an even more preferred embodiment, alone or in combination with other preferred embodiments, the HP1α of the invention is phosphorylated in, at least, 4 serine residues of its amino acid sequence. More preferably, HP1α of the invention is phosphorylated in serine residues at positions 10, 11, 12 and 13 of amino acid sequence SEQ ID NO: 3.

In the present invention, "sequence similarity" means the degree of similarity (similar residues non identical) between two amino acid sequences obtained by aligning the two sequences. The term "sequence identity" means the degree of identity (common or identical residues) between two amino acid or nucleotide sequences. Thus, the term similarity, in the present invention, is broader than the term identity, since it allows conservative substitutions of amino acid residues having similar physicochemical properties over a defined length of a given alignment.

Depending on the number of similar/identical residues between the aligned sequences, a degree of sequence similarity/identity expressed as a percentage will be obtained. The degree of similarity/identity between two nucleotide or amino acid sequences can be determined by conventional methods, *e.g.* by standard sequence alignment algorithms known in the state of the art, such as BLAST [Altschul S.F. et al. Basic local alignment search tool. J Mol Biol. 1990 Oct 5; 215(3):403-10]. BLAST programs, e.g. BLASTN, BLASTX, and TBLASTX, BLASTP and TBLASTN, are in the public domain on the website of The National Center for Biotechonology Information (NCBI). Other algorithms for pairwise alignment are CLUSTALΩ or EMBOSS Needle

Sequence identity and sequence similarity in the context of the present invention are determined according to the EMBOSS "needle" algorithm. This algorithm is a standard algorithm for pairwise amino acid sequence alignments covering the whole length of both sequences. The algorithm implements the Needleman-Wunsch algorithm (Needleman S.B. and Wunsch C.D., 1970 J. Mol. Biol. 48, 443-453), wherein a penalty for a gap of n positions is computed according to the formula gap opening penalty + n - 1 × gap extension penalty. The entire length of each amino acid sequence is aligned, and there is no penalty for hanging ends of the overlap.

Gap Open penalty in the context of the present invention is 10.0. The gap extension penalty in the context of the present invention is 0.5. The scoring matrix for comparing amino acid similarities in the context of the present invention is the "Blosum62" matrix. The Gap Open penalty, gap extend penalty and Blosum62 matrix are standard parameters used in the art. Sequence alignments can be performed with these parameters, e.g. via publicly available free tools. For example, the EBI offers a free pairwise sequence alignment service with the parameters of the present invention via its assortment of internet tools.

### Second polypeptide of the chimeric protein of the invention

As it has been previously mentioned, the second polypeptide of the chimeric protein of the invention (which is also referred in the present document as "binder element", or "binder element of the invention") comprises a recognition region for a pathogen.

The "recognition region for a pathogen" can be defined as the amino acid residues responsible for the recognition and binding of the pathogen of interest. The term "pathogen" may be defined as a microorganism or infectious agent that causes or can cause a disease or infection, including viruses, bacteria, protozoans, algae, prions, viroids or any other infectious agent or injurious molecules to living organisms. Thus, in the present invention, the term "pathogen" may also encompass toxins. The term "toxin" may be defined as a naturally occurring molecule that is injurious to some living organism. Thus, the term "recognition region for a toxin" can be defined as the amino acid residues responsible for the recognition and binding of a toxin of interest.

In particular, the recognition region for a pathogen/toxin binds to the pathogen or toxin of interest, for instance by its interaction with a targeted pathogen antigen in the case of the pathogen. A parameter to assess the strength of the pathogen recognition/binding by the recognition region or by a polypeptide comprising the recognition region is the equilibrium dissociation constant (K_{d}), which gives information related to the affinity between a ligand and a binding site, in the present invention, the affinity between the pathogen and the recognition region or the polypeptide comprising the recognition region. Similar parameters can be used for the toxin. K_{d} is inversely proportional to affinity, so the lower the K_{d} value the higher the affinity. Dissociation constant of the recognition region can be calculated by different techniques, most used are ELISA-based methods, biolayer interferometry (BLI) and surface plasmon resonance (SPR) among others. Since the individual binders are expressed attached to the yeast surface during the library screening, the binding affinity was assessed in a yeast display format by flow cytometry (Traxlmayr MW, Kiefer JD, Srinivas RR, Lobner E, Tisdale AW, Mehta NK, Yang NJ, Tidor B, Wittrup KD. Strong Enrichment of Aromatic Residues in Binding Sites from a Charge-neutralized Hyperthermostable Sso7d Scaffold Library. J Biol Chem. 2016. 291:22496-22508). Titration of the recombinant biotinylated soluble antigens (at concentrations between 0.01 nM to 3 µM) against the displayed binders, both differential fluorescence labelled, was performed by analytical flow cytometry. Previous studies have demonstrated that the K_{d} values determined using yeast surface display are in agreement with those measured using alternative techniques such as fluorescence polarization, SPR and BLI (Cherf GM, Cochran JR. Applications of Yeast Surface Display for Protein Engineering. Methods Mol Biol. 2015.1319:155-75. doi: 10.1007/978-1-4939-2748-7_8).

Usually, antibodies isolated from naïve or synthetic libraries have dissociation constants in the micromolar range while the ones generated *in vivo* can reach affinities up to 100 pM. Affinity maturation (a process aimed to increase protein affinity) could improve affinities up to 500-fold (K.L. Wark, P.J. Hudson. Latest technologies for the enhancement of antibody affinity. Adv. Drug Deliv. Rev., 58 (5-6) (2006), pp. 657-670).

The values obtained for the binders able to recognize the virus antigen after the first selection falled in the nanomolar range (4.5-110 nM) although others binders showed values between 1-2 µM, being discarded since better ones were available. Albeit a lower K_{d} value is preferred, values in the low micromolar range, preferably, a K_{d} of less than 10 µM, more preferably, 1-3 µM a indicates recognition and binding of the pathogen/toxin of interest. In the present invention, after the affinity maturation process, K_{d} values were situated in the low nanomolar and high picomolar range (0.65 nM-12.6 nM). In the case of the bacterial antigen, the measured K_{d} values, failed initially in the high nanomolar range (284-440 nM) and after affinity maturation process dissociation constants were improved although they were still within the nanomolar range (78-176 nM).

Preferably, when the second polypeptide comprises a recognition region for a toxin, said toxin is produced by an aquatic organism, including aquatic pathogens.

In a preferred embodiment, alone or in combination with other preferred embodiments, the second polypeptide comprises a recognition region for an aquatic pathogen.

The term "aquatic pathogen", as used herein refers to any pathogenic organism or toxin which may be found in any water environment including, without limitation to, marine water, fresh water, brackish water, sewage, ground and even drinking water. The term "aquatic pathogen" is well understood by an expert in the field, and is defined in guidelines from international organizations like the World Health Organization (for instance the Guidelines for Drinking-water Quality, 4rd ed. WHO; Geneva, Switzerland: 2017), the European Environment Agency (EEA) or the U.S. Environmental Protection Agency (EPA), as well as many other European or National legislation.

Examples of aquatic pathogens, include, without limitation to, virus: like the Adenovirus, Salmonid Alphavirus, amphibian ranavirus, Astroviruses, reoviruses (Aquareoviridae member Atlantic halibut reovirus (AHRV)), betanodavirus (also known as nodavirus), Duplodnaviria, Enteroviruses, fish herpesvirus, Hepatitis A virus, Hepatitis E virus, hepatopancreatic parvovirus (HPV), infectious hypodermal and hematopoietic necrosis virus (IHHNV), Infectious pancreatic necrosis virus (IPNV), iridoviruses, lymphoidal parvo-like virus (LPV), Monodnaviria, nimavirus (like shrimp and crayfish nimaviruses), Norovirus, orthomyxoviruses (e.g. infectious salmon anemia virus, ISAV), rhabdoviruses, Riboviria, Rotavirus, Sapoviruses, spawner-isolated mortality virus (SMV), Tilapia lake virus (TiLV), Varidnaviria, and microorganisms like: *Acanthamoeba spp., Actinobacillus pleuropneumoniae, Aerococcus viridans sp., Aeromonas hydrophila sp., Aeromonas salmonicida sp, Arthrobacter spp., Burkholderia pseudomallei, Campylobacter spp., Campylobacter jejuni, Cryptosporidium parvum, Cryptosporidium cayetanensis, Edwardsiella ictalurii sp., Edwardsiella tard sp., Entamoeba histolytica, Escherichia coli* (particularly enterohemorragic *E. coli* (EHEC), enteropathogenic (EPEC), enterotoxigenic (ETEC), and enteroinvasive (EIEC)), *Flavobacterium columnare sp., Giardia spp (Giardia intestinalis), Helicobacter pylori, Legionella pneumophila* , *Mycobacterium marinum sp., Naegleria fowleri, Piscirickettsia salmonis sp., Salmonella enterica serotype Typhi, Shigella spp., Pasteurella spp., Streptococcus agalactiae sp., Streptococcus iniae sp., Toxoplasma gondii, Vibrio sp. (particularly Vibrio alginolyticus sp., Vibrio cholerae sp., Vibrio ordalii sp., Vibrio salmonicida sp., Vibrio vulnificus sp.) Yersinia enterocolitica, Yersinia ruckeri, sp.*

Examples of toxins as aquatic pathogens include, but are not limited to, cyanotoxins such as anatoxin-a (produced by genera *Anabaena, Cylindrospermum, Microcystis, Oscillatoria, Raphidiopsis, Planktothix, Aphanizomenon, etc)* homoanatoxin-a (produced by genera *Oscillatoria, Anabaena, Raphidiopsis, Phormidium,* etc), saxitoxin (produced by genera *Anabaena, Aphanizomenon, Cylindrospermopsis, Lyngbya, Planktothrix, etc),* nodularin (produced by *Nodularia spumigena),* microcystins (produced by *Microcystis aeruginosa PCC 7806, Planktothrix agardhii CYA 126, or Anabaena sp. strain 90,* and genera *Microcystis, Planktothrix, Oscillatoria, Anabaena, Anabaenopsis, Nostoc, Hapalosiphon, Snowella, Woronichinia* amongst others). cylindrospermopsin (produced by *Cylindrospermopsis raciborskii, Umezakia natans, Aphanizomenon ovalisporum, Raphidiopsis curvata, Anabaena bergii, Aphanizomenon flos-aquae, Lyngbya wolle,* etc), Jamaicamides A, B and C from *Lyngbya majuscula,* Kalkitoxin produced by *Lyngbya majuscule,* Cholera Toxin (CT-toxin) and RTX toxins produced by *Vibrio spp.* (like *V. cholerae* and *V. vulnificus)* or *Legionella pneumophila,* Shiga toxins (produced by *Shigella dysenteriae and E. coli* pathotypes (STEC), for example) and any other toxin produced by aquatic microorganisms.

Thus, in a more preferred embodiment, alone or in combination with other preferred embodiments, the recognition region is for an aquatic pathogen selected from the list consisting of Adenovirus, Salmonid Alphavirus, amphibian ranavirus, Astroviruses, reoviruses (Aquareoviridae member Atlantic halibut reovirus (AHRV)), betanodavirus, Duplodnaviria, Enteroviruses, fish herpesvirus, Hepatitis A virus, Hepatitis E virus, hepatopancreatic parvovirus (HPV), infectious hypodermal and hematopoietic necrosis virus (IHHNV), Infectious pancreatic necrosis virus (IPNV), iridoviruses, lymphoidal parvo-like virus (LPV), Monodnaviria, nimavirus (like shrimp and crayfish nimaviruses), Norovirus, orthomyxoviruses (e.g. infectious salmon anemia virus, ISAV), rhabdoviruses, Riboviria, Rotavirus, Sapoviruses, spawner-isolated mortality virus (SMV), Tilapia lake virus (TiLV), Varidnaviria, and microorganisms like: *Acanthamoeba spp., Actinobacillus pleuropneumoniae, Aerococcus viridans sp., Aeromonas hydrophila sp., Aeromonas salmonicida sp, Arthrobacter spp., Burkholderia pseudomallei, Campylobacter spp., Campylobacter jejuni, Cryptosporidium parvum, Cryptosporidium cayetanensis, Edwardsiella ictalurii sp., Edwardsiella tard sp., Entamoeba histolytica, Escherichia coli* (particularly enterohemorragic *E. coli* (EHEC), enteropathogenic (EPEC), enterotoxigenic (ETEC), and enteroinvasive (EIEC), *Flavobacterium columnare sp., Giardia spp (Giardia intestinalis), Helicobacter pylori, Legionella pneumophila* , *Mycobacterium marinum sp., Naegleria fowleri, Piscirickettsia salmonis sp., Salmonella enterica serotype Typhi, Shigella spp., Pasteurella spp., Streptococcus agalactiae sp., Streptococcus iniae sp., Toxoplasma gondii, Vibrio sp. (particularly Vibrio alginolyticus sp., Vibrio cholerae sp., Vibrio ordalii sp., Vibrio salmonicida sp., Vibrio vulnificus sp.) Yersinia enterocolitica, Yersinia ruckeri, sp.* cyanotoxins such as anatoxin-a, homoanatoxin-a, saxitoxin, nodularin, microcystins, cylindrospermopsin, Jamaicamides A, B and C, Kalkitoxin, Cholera Toxin (CT-toxin), RTX toxins and Shiga toxin.

More preferably, the recognition region is for an aquatic pathogen selected from the list consisting of *Aeromonas hydrophila* sp., *Edwardsiella tard* sp., *Mycobacterium marinum* sp., *Streptococcus agalactiae* sp., *Streptococcus iniae* sp., *Vibrio alginolyticus* sp., *Vibrio cholerae* sp., *Vibrio vulnificus* sp., *Vibrio sp., Aeromonas salmonicida sp,* species belonging to *Arthrobacter* genus, *Vibrio ordalii* sp., *Yersinia ruckeri* sp., *Infectious hematopoietic necrosis virus, infectious pancreatic necrosis virus (IPN), Edwardsiella Ictalurii* sp., *Flavobacterium columnare* sp., *Vibrio salmonicida* sp., a betanodavirus (also known as nodavirus for short), *Streptococcus agalactiae sp.,* species belonging to *Pasteurella, Piscirickettsia salmonis sp., Aerococcus viridans* sp., and Salmonid Alphavirus.

Aquatic pathogens include aquaculture pathogens. The term "aquaculture pathogen", as used herein, refers to any aquaculture pathogen, including without limitation to, any bacteria or virus, able to infect one or more aquatic organism susceptible to be cultivated, such as fish, shellfish, algae or other aquatic organisms. It also refers to toxins that may accumulate in aquatic organisms and that can be or not transferred to the food chain such as microcystins (that are found to be accumulated in mussels, fish and crustaceans, crayfish and even plants that are irrigated with contaminated water), paralytic shellfish poisons (PSP) toxins (that can accumulate in cladoceran Daphnia magna, clams, crabs and mussels), cylindrospermopsin (that accumulates in mussels) or other well-known examples for a person skilled in the the art.

Thus, in a still more preferred embodiment, alone or in combination with other preferred embodiments, the second polypeptide comprises a recognition region for an aquaculture pathogen.

Examples of aquaculture pathogens include, without limitation to, *Aeromonas salmonicida sp,* species belonging to *Arthrobacter* genus, *Vibrio ordalii* sp., *Vibrio sp., Yersinia ruckeri* sp., *Infectious hematopoietic necrosis virus, infectious pancreatic necrosis virus (IPN), Edwardsiella Ictalurii* sp., *Flavobacterium columnare* sp., *Vibrio salmonicida* sp., a betanodavirus (also known as nodavirus), *Aeromonas hydrophila sp., Streptococcus agalactiae sp.,* species belonging to *Pasteurella, Piscirickettsia salmonis sp., Aerococcus viridans* sp., or a Salmonid Alphavirus. More preferably, the second polypeptide comprises a recognition region for an aquaculture pathogen wherein the aquaculture pathogen is selected from the list consisting of *Aeromonas salmonicida sp,* species belonging to *Arthrobacter genus, Vibrio ordaliisp., Vibrio sp., Yersinia ruckeri* sp., *Infectious hematopoietic necrosis virus, infectious pancreatic necrosis virus (IPN), Edwardsiella Ictalurii* sp., *Flavobacterium columnare* sp., *Vibrio salmonicida* sp., a betanodavirus, *Aeromonas hydrophila sp., Streptococcus agalactiae sp.,* species belonging to *Pasteurella, Piscirickettsia salmonis sp., Aerococcus viridans* sp., and a Salmonid Alphavirus. Even more preferably, the aquaculture pathogen is a betanodavirus, preferably RGNNV (red-spotted grouper nervous necrosis virus) genotype, or a *Yersinia ruckeri* sp.

In a preferred embodiment, alone or in combination with other preferred embodiments, the recognition region for a pathogen comprises, or consists of, an amino acid sequence with a sequence similarity of, at least, 80, 80, 81, 82, 83, 84, 85, 86, 87, 88, 89, 90, 91, 92, 93, 94, 95, 96, 97, 98, or 99% to an amino acid sequence selected from the list consisting of SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30.

In a more preferred embodiment, the recognition region comprises, or consists of, an amino acid sequence selected from the list consisting of, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30.

**Table 1. Chimeric protein recognition region sequences**

| **Recognition region** | **Amino acid sequence** |
|---|---|
| P1 (SEQ ID NO: 4) | YYHIYNINI |
| P7 (SEQ ID NO: 5) | FFMAEALVV |
| P8 (SEQ ID NO: 6) | NHHIGWYHK |
| P9 (SEQ ID NO: 7) | NHHYSWYHK |
| P11 (SEQ ID NO: 8) | NHHYAWWHK |
| P14 (SEQ ID NO: 9) | NHHWSWYHK |
| P15 (SEQ ID NO: 10) | RIKHWSNYW |
| P17 (SEQ ID NO: 11) | SIKHWGKYW |
| P18 (SEQ ID NO: 12) | RHHSHHNGY |
| P19 (SEQ ID NO: 13) | WWYVSHHAL |
| L1 (SEQ ID NO: 14) | MIMMLTMRI |
| L2 (SEQ ID NO: 15) | YIFKMKHSM |
| L3 (SEQ ID NO: 16) | YWHFMRNNE |
| R2 (SEQ ID NO: 17) | MMNRRMILE |
| R3 (SEQ ID NO: 18) | YYFMKQWFV |
| R4 (SEQ ID NO: 19) | VADWDSAMY |
| R5 (SEQ ID NO: 20) | FMLAAGRLL |
| R7 (SEQ ID NO: 21) | DADYDSSYW |
| R9 (SEQ ID NO: 22) | YYRIYIYWD |
| P7.C1 (SEQ ID NO: 23) | VOFFMAEANLVVEE |
| P7.C2 (SEQ ID NO: 24) | FFMAEAVLVVE |
| P7.C3 (SEQ ID NO: 25) | TRFFMAEADVLVV |
| P7.C4 (SEQ ID NO: 26) | RFFMAEAVLVVN |
| P7.C5 (SEQ ID NO: 27) | FFMAEAVLVVNR |
| R2.C6 (SEQ ID NO: 28) | MMNRRMAILER |
| R2.C14 (SEQ ID NO: 29) | GMMMNRRMILE |
| R2.C19 (SEQ ID NO: 30) | IMMSRRMILE |

Furthermore, the second polypeptide of the chimeric protein of the invention can comprise a reduced-charged variant of Sso7d (rcSso7d) amino acid sequence with a recognition region. The "rcSso7d" is a binding scaffold of great stability known in the state of the art, particularly suited for biotechnological applications "outside the body" or *ex vivo.* In particular, it is a 7 kDa DNA-binding protein from the hyperthermophilic archaeon *Sulfolobus solfataricus.* Some advantages related to its use are its small size (7 kDa), lack of cysteines and glycosylation sites that favors its expression in bacteria as well as the location of the paratope on the surface of a rigid *β*-sheet, which reduces the entropic penalty upon binding. Moreover, its high stability makes it more evolvable because it tolerates a wider range of mutations maintaining its native fold, and can be easily attached to a protein module.

Thus, in another preferred embodiment, alone or in combination with other preferred embodiments, the second polypeptide comprises, or consists of, an rcSso7d amino acid sequence with a recognition region for a pathogen, or for a toxin as a pathogen, also named in the present invention as "engineered rcSso7d of the invention" This engineered rcSso7d may be obtained by yeast surface display screening of combinatorial libraries.

The rcSso7d amino acid sequence comprises an amino acid sequence with at least, 86 , 87, 88, 89, 90, 91, 92, 93, 94, 95, 96, 97, 98, or 99% sequence similarity to the amino acid sequence SEQ ID NO: 31, and/or a sequence identity of at least, 86, 87, 88, 89, 90, 91, 92, 93, 94, 95, 96, 97, 98, or 99% to the amino acid sequence SEQ ID NO: 31.

SEQ ID NO: 31 (reduced-charge Sso7d (rcSso7d) amino acid sequence

ATVKFTYQGEEKQVDISKIKKVWRVGQMISFTYDEGGGATGRGAVSEKDAPKELL
QMLEKQ

SEQ ID NO: 31 is the reduced-charge Sso7d (rcSso7d) amino acid sequence. The engineered rcSso7d may contain mutations within its sequence (for instance in the highlighted positions in bold) corresponding to amino acid residues of the recognition region.

In a more preferred embodiment, alone or in combination with other preferred embodiments, the second polypeptide comprises, or consists of, amino acid sequence SEQ ID NO: 99 
SEQ ID NO: 99
AX1VKFTYQGEEKQX2X3X4SKIX5X6X7X8RX9GQX10IX11FX12YBX13GX14GX15X16GX17
GX18VX19EX20DAPX21ELLQMLEX22Q
 wherein
X₁ is T or I,
X₂ is V or T,
X₃ is D or G,
X₄ is I or V,
X₅ is K, Q or R,
X₆ is Y, F, N, R, S, W, M, V or D,
X₇ is V or M,
X₈ is Y, F, H, A, W, I, or M,
X₉ is H, K, Y, N, F, L, D, R, M or S,
X₁₀ is I, A, Y, W, H, S, V, K, F, R or M,
X₁₁ is Y, E, G, S, A, W, H, L, M, R, K or D,
X₁₂ is N, A, W, S, G, H, T, K, R, M, Q or I,
B is D or N,
X₁₃ is E or A,
X₁₄ is G or D,
X₁₅ is A or V,
X₁₆ is I, L, Y, W, N, K, H, M A, R, or S,
X₁₇ is N, V, H, Y, G, A, R, S, L, F, M or W,
X₁₈ is I, V, K, W, Y, L, M, E or D,
X₁₉ is S or N,
X₂₀ is K, E or R,
X₂₁ is K, E or R, and
X₂₂ is K or E.

SEQ ID NO: 99 is a consensus amino acid sequence of engineered rcSso7d sequences shown in Table 2.

More preferably, the second polypeptide comprises, or consists of, amino acid sequence SEQ ID NO: 99, wherein X₁ is T, X₂ is V, X₃ is D, X₄ is I, X₅ is K, X₇ is V, B is D, X₁₃ is E, X₁₄ is G, X₁₅ is A, X₁₉ is S, X₂₀ is K, X₂₁ is K and X₂₂ is K.

In another more preferred embodiment, alone or in combination with other preferred embodiments, the second polypeptide comprises an amino acid sequence with a sequence similarity and/or a sequence identity of, at least, 80% to an amino acid sequence selected from the list consisting of SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58.

Even more preferably, the second polypeptide comprises, or consists of, an amino acid sequence with a sequence similarity and/or a sequence identity of, at least, 80, 81, 82, 83, 84, 85, 86, 87, 88, 89, 90, 91, 92, 93, 94, 95, 96, 97, 98, or 99% to an amino acid sequence selected from the list consisting of SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58.

More preferably, the second polypeptide comprises, or consists of, an amino acid sequence selected from the list consisting of SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58.

**Table 2. Engineered rcSso7d sequences. Highlighted residues in the sequences below are amino acid residues responsible for the recognition and binding of the pathogen/toxin of interest.**

| engineered rcSso7d | **Sequence** |
|---|---|
| P1 (SEQ ID NO: 32) | ATVKFTYQGEEKQVDISKIKYVYRHGQIIYFNYDEGGGAIGNGIVS EKDAPKELLQMLEKQ |
| P7 (SEQ ID NO: 33) | ATVKFTYQGEEKQVDISKIKFVFRMGQAIEFAYDEGGGALGVGV VSEKDAPKELLQMLEKQ |
| P8 (SEQ ID NO: 34) | ATVKFTYQGEEKQVDISKIKNVHRHGQIIGFWYDEGGGAYGHGK VSEKDAPKELLQMLEKQ |
| P9 (SEQ ID NO: 35) | ATVKFTYQGEEKQVDISKIKNVHRHGQYISFWYDEGGGAYGHG KVSEKDAPKELLQMLEKQ |
| P11 (SEQ ID NO: 36) | ATVKFTYQGEEKQVDISKIKNVHRHGQYIAFWYDEGGGAWGHG KVSEKDAPKELLQMLEKQ |
| P14 (SEQ ID NO: 37) | ATVKFTYQGEEKQVDISKIKNVHRHGQWISFWYDEGGGAYGHG KVSEKDAPKELLQMLEKQ |
| P15 (SEQ ID NO: 38) | ATVKFTYQGEEKQVDISKIKRVIRKGQHIWFSYDEGGGANGYGW VSEKDAPKELLQMLEKQ |
| P17 (SEQ ID NO: 39) | ATVKFTYQGEEKQVDISKIKSVIRKGQHIWFGYDEGGGAKGYGW VSEKDAPKELLQMLEKQ |
| P18 (SEQ ID NO: 40) | ATVKFTYQGEEKQVDISKIKRVHRHGQSIHFHYDEGGGANGGG YVSEKDAPKELLQMLEKQ |
| P19 (SEQ ID NO: 41) | ATVKFTYQGEEKQVDISKIKWVWRYGQVISFHYDEGGGAHGAG LVSEKDAPKELLQMLEKQ |
| L1 (SEQ ID NO: 42) | ATVKFTYQGEEKQVDISKIKMVIRMGQMILFTYDEGGGAMGRGI VSEKDAPKELLQMLEKQ |
| L2 (SEQ ID NO: 43) | ATVKFTYQGEEKQVDISKIKYVIRFGQKIMFKYDEGGGAHGSGM VSEKDAPKELLQMLEKQ |
| L3 (SEQ ID NO: 44) | ATVKFTYQGEEKQVDISKIKYVWRHGQFIMFRYDEGGGANGNG EVSEKDAPKELLQMLEKQ |
| R2 (SEQ ID NO: 45) | ATVKFTYQGEEKQVDISKIKMVMRNGQRIRFMYDEGGGAIGLGE VSEKDAPKELLQMLEKQ |
| R3 (SEQ ID NO: 46) | ATVKFTYQGEEKQVDISKIKYVYRFGQMIKFQYDEGGGAWGFG VVSEKDAPKELLQMLEKQ |
| R4 (SEQ ID NO: 47) | ATVKFTYQGEEKQVDISKIKVVARDGQWIDFSYDEGGGAAGMG YVSEKDAPKELLQMLEKQ |
| R5 (SEQ ID NO: 48) | ATVKFTYQGEEKQVDISKIKFVMRLGQAIAFGYDEGGGARGLGL VSEKDAPKELLQMLEKQ |
| R7 (SEQ ID NO: 49) | ATVKFTYQGEEKQVDISKIKDVARDGQYIDFSYDEGGGASGYG WVSEKDAPKELLQMLEKQ |
| R9 (SEQ ID NO: 50) | ATVKFTYQGEEKQVDISKIKYVYRRGQIIYFIYDEGGGAYGWGDV SEKDAPKELLQMLEKQ |
| P7.C1 (SEQ ID NO: 51) | ATVKFTYQGEEKQVDVSKIQFVFRMGQAIEFAYNEGGGALGVG VVSEKDAPEELLQMLEEQ |
| P7.C2 (SEQ ID NO: 52) | ATVKFTYQGEEKQVDISKIKFVFRMGQAIEFAYDEGGGVLGVGV VSEEDAPKELLQMLEKQ |
| P7.C3 (SEQ ID NO: 53) | ATVKFTYQGEEKQTDISKIRFVFRMGQAIEFAYDEGDGVLGVGV VSEKDAPKELLQMLEKQ |
| P7.C4 (SEQ ID NO: 54) | ATVKFTYQGEEKQVDISKIRFVFRMGQAIEFAYDEGGGVLGVGV VNEKDAPRELLQMLEKQ |
| P7.C5 (SEQ ID NO: 55) | ATVKFTYQGEEKQVDISKIKFVFRMGQAIEFAYDEGGGVLGVGV VSEKDAPKELLQMLEKQ |
| R2.C6 (SEQ ID NO: 56) | ATVKFTYQGEEKQVDISKIKMVMRNGQRIRFMYDAGGGAIGLGE VSERDAPKELLQMLEKQ |
| R2.C14 (SEQ ID NO: 57) | ATVKFTYQGEEKQVGISKIKMMMRNGQRIRFMYDEGGGAIGLGE VSEKDAPKELLQMLEKQ |
| R2.C19 (SEQ ID NO: 58) | AIVKFTYQGEEKQVDISKIKMVMRSGQRIRFMYDEGGGAIGLGE VSEKDAPKELLQMLEKQ |

Another preferred embodiment provides the chimeric protein of the invention, wherein the second polypeptide comprises, or consists of, an amino acid sequence with a sequence similarity and /or a sequence identity of, at least, 80, 80, 81, 82, 83, 84, 85, 86, 87, 88, 89, 90, 91, 92, 93, 94, 95, 96, 97, 98, or 99% to an amino acid sequence selected from the list consisting of SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58.

More preferably, the second polypeptide comprises, or consists of, an amino acid sequence selected from the list consisting of SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58.

A preferred embodiment provides the chimeric protein of the invention, wherein the first polypeptide is DBP1 and the second polypeptide comprises an amino acid sequence with a sequence similarity and/or a sequence identity of, at least, 80, 81, 82, 83, 84, 85, 86, 87, 88, 89, 90, 91, 92, 93, 94, 95, 96, 97, 98, or 99%, to an amino acid sequence selected from the list consisting of SEQ ID NO: 5, SEQ ID NO: 33, which may be also called hereinafter as DBP1-P7 chimeric protein of the invention, SEQ ID NO: 25, SEQ ID NO: 53, which may be also called hereinafter as the DBP1-P7.C3 chimeric protein of the invention, SEQ ID NO: 26, and SEQ ID NO: 54, which may be also called hereinafter as the DBP1-P7.C4 chimeric protein of the invention

Other preferred embodiment provides the chimeric protein of the invention, wherein the first polypeptide is HP1α, and the second polypeptide comprises, or consists of, an amino acid sequence with a sequence similarity and/or a sequence identity of, at least, 80, 81, 82, 83, 84, 85, 86, 87, 88, 89, 90, 91, 92, 93, 94, 95, 96, 97, 98, or 99%, to an amino acid sequence selected from the list consisting of SEQ ID NO: 5, SEQ ID NO: 33, which may be also called hereinafter as the HP1α-P7 chimeric protein of the invention, SEQ ID NO: 14, SEQ ID NO: 42, which may be also called hereinafter as the HP1α-L1 chimeric protein of the invention, SEQ ID NO: 15, SEQ ID NO: 43, which may be also called hereinafter as HP1α-L2 chimeric protein of the invention, SEQ ID NO: 17, SEQ ID NO: 45, which may be also called hereinafter as HP1α-R2 chimeric protein of the invention, SEQ ID NO: 28, SEQ ID NO: 56, which may be also called hereinafter as HP1α-R2.C6 chimeric protein of the invention, SEQ ID NO: 29, and SEQ ID NO: 57, which may be also called hereinafter as HP1α-R2.C14 chimeric protein of the invention.

Other preferred embodiment provides the chimeric protein of the invention, wherein the first polypeptide is the phosphorylated version of HP1α of the invention (HP1αPhos), and the second polypeptide comprises, or consists of, an amino acid sequence with a sequence similarity and/or a sequence identity of, at least, 80, 81, 82, 83, 84, 85, 86, 87, 88, 89, 90, 91, 92, 93, 94, 95, 96, 97, 98, or 99%, to an amino acid sequence selected from the list consisting of SEQ ID NO: 5, SEQ ID NO: 33, which may be also called hereinafter as HP1αPhos-P7 chimeric protein of the invention, SEQ ID NO: 14, SEQ ID NO: 42 which may be also called hereinafter as HP1αPhos-L1 chimeric protein of the invention, SEQ ID NO 17, SEQ ID NO: 45 which may be also called hereinafter as HP1αPhos-R2 chimeric protein of the invention, SEQ ID NO: 15, SEQ ID NO: 43 which may be also called as HP1αPhos-L2 chimeric protein of the invention, SEQ ID NO: 28, SEQ ID NO: 56, which may be also called hereinafter as HP1αPhos-R2.C6 chimeric protein of the invention, SEQ ID NO: 29, and SEQ ID NO: 57, which may be also called hereinafter as HP1αPhos-R2.C14 chimeric protein of the invention .

The term "sequence similarity", which in the present invention is computed according algorithm EMBOSS Needle using the BLOSUM62 matrix, having a Gap Open penalty of 10.0 and a Gap Extend penalty of 0.5, and the term "sequence identity", have been previously defined in the present document, and their definitions apply equally herein, and in the rest of the document.

In a more preferred embodiment, the amino acid sequence of the DBP1-P7 chimeric protein of the invention, comprises or consists of, SEQ ID NO: 59.

In another more preferred embodiment, the amino acid sequence of the DBP1-P7.C3 chimeric protein of the invention, comprises or consists of, SEQ ID NO: 60.

In another more preferred embodiment, the amino acid sequence of the DBP1-P7.C4 chimeric protein of the invention, comprises or consists of, SEQ ID NO: 61.

In another more preferred embodiment, the amino acid sequence of the HP1α-L1 chimeric protein of the invention, comprises or consists of, SEQ ID NO: 62.

In another more preferred embodiment, the amino acid sequence of the HP1α-L2 chimeric protein of the invention, comprises or consists of, SEQ ID NO: 63.

In another more preferred embodiment, the amino acid sequence of the HP1α-R2 chimeric protein of the invention, comprises or consists of, SEQ ID NO: 64.

In another more preferred embodiment, the amino acid sequence of the HP1α-R2.C6 chimeric protein of the invention, comprises or consists of, SEQ ID NO: 65.

In another more preferred embodiment, the amino acid sequence of the HP1α-R2.C14 chimeric protein of the invention, comprises or consists of, SEQ ID NO: 66.

In another more preferred embodiment, the amino acid sequence of the HP1αPhos-P7 chimeric protein of the invention, comprises or consists of, SEQ ID NO: 67.

In another more preferred embodiment, the amino acid sequence of the HP1αPhos-L1 chimeric protein of the invention, comprises or consists of, SEQ ID NO: 68.

In another more preferred embodiment, the amino acid sequence of the HP1αPhos-R2 chimeric protein of the invention, comprises or consists of, SEQ ID NO: 69.

In another more preferred embodiment, the amino acid sequence of the HP1αPhos-L2 chimeric protein of the invention, comprises or consists of, SEQ ID NO: 96.

In another more preferred embodiment, the amino acid sequence of the HP1αPhos-R2.C6 chimeric protein of the invention, comprises or consists of, SEQ ID NO: 97.

In another more preferred embodiment, the amino acid sequence of the HP1αPhos-R2.C14 chimeric protein of the invention, comprises or consists of, SEQ ID NO: 98.

**Table 3. Chimeric proteins sequences.**

| **Chimeric protein** | **Sequence** |
|---|---|
| DBP1-P7 (SEQ ID NO: 59) | MADLPQKVSNLSINNKENGGGGGKSSYVPPHLRSRGKPSFERRSPK QKDKVTGGDFFRRAGRQTGNNGGFFGFSKERNGGTSANYNRRGSS NYKSSGNRWVNGKHIPGPKNAKLQKAELFGVHDDPDYHSSGIKFDN YDNIPVDASGKDVPEPILGGRTRGGGGFFNSRNNGSRDYRKHGGNG SFGSTRPRNTGTSNWGSIGGGFRNDNEKNGYGNSNASWWGSATVK FTYQGEEKQVDISKIKFVFRMGQAIEFAYDEGGGALGVGVVSEKDAP KELLQMLEKQ |
| DBP1-P7.C3 (SEQ ID NO: 60) | MADLPQKVSNLSINNKENGGGGGKSSYVPPHLRSRGKPSFERRSPK QKDKVTGGDFFRRAGRQTGNNGGFFGFSKERNGGTSANYNRRGSS NYKSSGNRWVNGKHIPGPKNAKLQKAELFGVHDDPDYHSSGIKFDN YDNIPVDASGKDVPEPILGGRTRGGGGFFNSRNNGSRDYRKHGGNG SFGSTRPRNTGTSNWGSIGGGFRNDNEKNGYGNSNASWWGSATVK FTYQGEEKQTDISKIRFVFRMGQAIEFAYDEGDGVLGVGVVSEKDAP KELLQMLEKQ |
| DBP1-P7.C4 (SEQ ID NO: 61) | MADLPQKVSNLSINNKENGGGGGKSSYVPPHLRSRGKPSFERRSPK QKDKVTGGDFFRRAGRQTGNNGGFFGFSKERNGGTSANYNRRGSS NYKSSGNRWVNGKHIPGPKNAKLQKAELFGVHDDPDYHSSGIKFDN YDNIPVDASGKDVPEPILGGRTRGGGGFFNSRNNGSRDYRKHGGNG SFGSTRPRNTGTSNWGSIGGGFRNDNEKNGYGNSNASWWGSATVK FTYQGEEKQVDISKIRFVFRMGQAIEFAYDEGGGVLGVGVVNEKDAP RELLQMLEKQ |
| mHP1α-L1 (SEQ ID NO: 62) | MGKKTKRTADSSSSEDEEEYVVEKVLDRRMVKGQVEYLLKWKGFSE EHNTWEPEKNLDCPELISEFMKKYKKMKEGENNKPREKSEGNKRKS SFSNSADDIKSKKKREQSNDIARGFERGLEPEKIIGATDSCGDLMFLM KWKDTDEADLVLAKEANVKCPQIVIAFYEERLTWHAYPEDAENKEKE SAKSGSATVKFTYQGEEKQVDISKIKMVIRMGQMILFTYDEGGGAMG RGIVSEKDAPKELLQMLEKQ |
| mHP1α-L2 (SEQ ID NO: 63) | MGKKTKRTADSSSSEDEEEYVVEKVLDRRMVKGQVEYLLKWKGFSE EHNTWEPEKNLDCPELISEFMKKYKKMKEGENNKPREKSEGNKRKS SFSNSADDIKSKKKREQSNDIARGFERGLEPEKIIGATDSCGDLMFLM KWKDTDEADLVLAKEANVKCPQIVIAFYEERLTWHAYPEDAENKEKE SAKSGSATVKFTYQGEEKQVDISKIKYVIRFGQKIMFKYDEGGGAHGS GMVSEKDAPKELLQMLEKQ |
| mHP1α-R2 (SEQ ID NO: 64) | MGKKTKRTADSSSSEDEEEYVVEKVLDRRMVKGQVEYLLKWKGFSE EHNTWEPEKNLDCPELISEFMKKYKKMKEGENNKPREKSEGNKRKS SFSNSADDIKSKKKREQSNDIARGFERGLEPEKIIGATDSCGDLMFLM KWKDTDEADLVLAKEANVKCPQIVIAFYEERLTWHAYPEDAENKEKE SAKSGSATVKFTYQGEEKQVDISKIKMVMRNGQRIRFMYDEGGGAIG LGEVSEKDAPKELLQMLEKQ |
| mHP1α-R2.C6 (SEQ ID NO: 65) | MGKKTKRTADSSSSEDEEEYVVEKVLDRRMVKGQVEYLLKWKGFSE EHNTWEPEKNLDCPELISEFMKKYKKMKEGENNKPREKSEGNKRKS SFSNSADDIKSKKKREQSNDIARGFERGLEPEKIIGATDSCGDLMFLM |
| | KWKDTDEADLVLAKEANVKCPQIVIAFYEERLTWHAYPEDAENKEKE SAKSGSATVKFTYQGEEKQVDISKIKMVMRNGQRIRFMYDAGGGAIG LGEVSERDAPKELLQMLEKQ |
| mHP1α-R2.C14 (SEQ ID NO: 66) | MGKKTKRTADSSSSEDEEEYVVEKVLDRRMVKGQVEYLLKWKGFSE EHNTWEPEKNLDCPELISEFMKKYKKMKEGENNKPREKSEGNKRKS SFSNSADDIKSKKKREQSNDIARGFERGLEPEKIIGATDSCGDLMFLM KWKDTDEADLVLAKEANVKCPQIVIAFYEERLTWHAYPEDAENKEKE SAKSGSATVKFTYQGEEKQVGISKIKMMMRNGQRIRFMYDEGGGAIG LGEVSEKDAPKELLQMLEKQ |
| HP1αPhos-P7 (SEQ ID NO: 67) | GKKTKRTADSSSSEDEEEYVVEKVLDRRVVKGQVEYLLKWKGFSEE HNTWEPEKNLDCPELISEFMKKYKKMKEGENNKPREKSESNKRKAN FANAADDIKSKKKREQSNDIARGFERGLEPEKIIGATDSCGDLMFLMK WKDTDEADLVLAKEANVKCPQIVIAFYEERLTWHAYPEDAENKEKETA KSGSATVKFTYQGEEKQVDISKIKFVFRMGQAIEFAYDEGGGALGVG VVSEKDAPKELLQMLEKQ |
| HP1αPhos-L1 (SEQ ID NO: 68) | GKKTKRTADSSSSEDEEEYVVEKVLDRRVVKGQVEYLLKWKGFSEE HNTWEPEKNLDCPELISEFMKKYKKMKEGENNKPREKSESNKRKAN FANAADDIKSKKKREQSNDIARGFERGLEPEKIIGATDSCGDLMFLMK WKDTDEADLVLAKEANVKCPQIVIAFYEERLTWHAYPEDAENKEKETA KSGSATVKFTYQGEEKQVDISKIKMVIRMGQMILFTYDEGGGAMGRGI VSEKDAPKELLQMLEKQ |
| HP1αPhos-R2 (SEQ ID NO: 69) | GKKTKRTADSSSSEDEEEYVVEKVLDRRVVKGQVEYLLKWKGFSEE HNTWEPEKNLDCPELISEFMKKYKKMKEGENNKPREKSESNKRKAN FANAADDIKSKKKREQSNDIARGFERGLEPEKIIGATDSCGDLMFLMK WKDTDEADLVLAKEANVKCPQIVIAFYEERLTWHAYPEDAENKEKETA KSGSATVKFTYQGEEKQVDISKIKMVMRNGQRIRFMYDEGGGAIGLG EVSEKDAPKELLQMLEKQ |
| HP1αPhos-L2 (SEQ ID NO: 96) | GKKTKRTADSSSSEDEEEYVVEKVLDRRVVKGQVEYLLKWKGFSEE HNTWEPEKNLDCPELISEFMKKYKKMKEGENNKPREKSESNKRKAN FANAADDIKSKKKREQSNDIARGFERGLEPEKIIGATDSCGDLMFLMK WKDTDEADLVLAKEANVKCPQIVIAFYEERLTWHAYPEDAENKEKETA KSGSATVKFTYQGEEKQVDISKIKYVIRFGQKIMFKYDEGGGAHGSG MVSEKDAPKELLQMLEKQ |
| HP1αPhos-R2.C6 (SEQ ID NO: 97) | GKKTKRTADSSSSEDEEEYVVEKVLDRRVVKGQVEYLLKWKGFSEE HNTWEPEKNLDCPELISEFMKKYKKMKEGENNKPREKSESNKRKAN FANAADDIKSKKKREQSNDIARGFERGLEPEKIIGATDSCGDLMFLMK WKDTDEADLVLAKEANVKCPQIVIAFYEERLTWHAYPEDAENKEKETA |
| | KSGSATVKFTYQGEEKQVDISKIKMVMRNGQRIRFMYDAGGGAIGLG EVSERDAPKELLQMLEKQ |
| HP1αPhos-R2.C14 (SEQ ID NO: 98) | GKKTKRTADSSSSEDEEEYVVEKVLDRRVVKGQVEYLLKWKGFSEE HNTWEPEKNLDCPELISEFMKKYKKMKEGENNKPREKSESNKRKAN FANAADDIKSKKKREQSNDIARGFERGLEPEKIIGATDSCGDLMFLMK WKDTDEADLVLAKEANVKCPQIVIAFYEERLTWHAYPEDAENKEKETA KSGSATVKFTYQGEEKQVGISKIKMMMRNGQRIRFMYDEGGGAIGLG EVSEKDAPKELLQMLEKQ |
| DBP1-L1 (SEQ ID NO: 101) | MADLPQKVSNLSINNKENGGGGGKSSYVPPHLRSRGKPSFERRSPK QKDKVTGGDFFRRAGRQTGNNGGFFGFSKERNGGTSANYNRRGSS NYKSSGNRWVNGKHIPGPKNAKLQKAELFGVHDDPDYHSSGIKFDN YDNIPVDASGKDVPEPILGGRTRGGGGFFNSRNNGSRDYRKHGGNG SFGSTRPRNTGTSNWGSIGGGFRNDNEKNGYGNSNASWWGSATVK FTYQGEEKQVDISKIKMVIRMGQMILFTYDEGGGAMGRGIVSEKDAPK ELLQMLEKQ |
| DBP1-L2 (SEQ ID NO: 102) | MADLPQKVSNLSINNKENGGGGGKSSYVPPHLRSRGKPSFERRSPK QKDKVTGGDFFRRAGRQTGNNGGFFGFSKERNGGTSANYNRRGSS NYKSSGNRWVNGKHIPGPKNAKLQKAELFGVHDDPDYHSSGIKFDN YDNIPVDASGKDVPEPILGGRTRGGGGFFNSRNNGSRDYRKHGGNG SFGSTRPRNTGTSNWGSIGGGFRNDNEKNGYGNSNASWWGSATVK FTYQGEEKQVDISKIKYVIRFGQKIMFKYDEGGGAHGSGMVSEKDAP KELLQMLEKQ |

In addition, the chimeric protein of the invention may be labelled or coupled to an indicator. In the present invention, the term "indicator" refers to any compound or molecule capable of being, visualized and/or quantified. The term "indicator" may be interchanged with compound or molecule "indicator", "signal", "label" or "reporter".

Examples of indicators include, but are not limited to, dyes, fluorophores, substances with redox activity, plasmonic resonance or biologically active such as cytotoxic agents, proteins, small biomolecules (i.e., biotin), enzymes or nucleic acid fragments. Thus, in a preferred embodiment of the chimeric protein of the invention, alone or in combination with other preferred embodiments, the indicator is selected from a list consisting of dyes, substances with redox activity, plasmonically resonant and biologically active substances.

### Polynucleotide and gene construct encoding the amino acid sequences of the invention

Other related aspect of the present invention refers to a polynucleotide comprising a nucleotide sequence encoding the chimeric protein of the invention, hereinafter the "polynucleotide of the invention". The nucleotide sequence may be a deoxyribonucleic acid (DNA) or a ribonucleic acid.

In a preferred embodiment, the polynucleotide of the invention comprises, or consists of, a first nucleotide sequence encoding the DBP1 or HP1α the polypeptide, and a nucleotide sequence encoding the second polypeptide of the chimeric protein of the invention.

In a more preferred embodiment, alone or in combination with other preferred embodiments, the first nucleotide sequence is a sequence with a sequence identity of, at least, 80, 81, 82, 83, 84, 85, 86, 87, 88, 89, 90, 91, 92, 93, 94, 95, 96, 97, 98, or 99% to SEQ ID NO: 70

SEQ ID NO: 70:
ATGGCGGATCTGCCGCAGAAAGTGAGCAACCTGAGCATTAACAACAAAGAAAA
CGGCGGCGGCGGCAAAAGCAGCTATGTGCCGCCGCATCTGCGCAGCCG
CGGCAAACCGAGCTTTGAACGCCGCAGCCCGAAACAGAAAGATAAAGTGACC
GGCGGCGATTTTTTTCGCCGCGCGGGCCGCCAGACCGGCAACAACGGCGGCT
TTTTTGGCTTTAGCAAAGAACGCAACGGCGGCACCAGCGCGAACTATAACCGC
CGCGGCAGCAGCAACTATAAAAGCAGCGGCAACCGCTGGGTGAACGGCAAAC
ATATTCCGGGCCCGAAAAACGCGAAACTGCAGAAAGCGGAACTGTTTGGCGTG
CATGATGATCCGGATTATCATAGCAGCGGCATTAAATTTGATAACTATGATAAC
ATTCCGGTGGATGCGAGCGGCAAAGATGTGCCGGAACCGATTCTGGGCGGCC
GCACCCGCGGCGGCGGCGGCTTTTTTAACAGCCGCAACAACGGCAGCCGCGA
TTATCGCAAACATGGCGGCAACGGCAGCTTTGGCAGCACCCGCCCGCGCAAC
ACCGGCACCAGCAACTGGGGCAGCATTGGCGGCGGCTTTCGCAACGATAACG
AAAAAAACGGCTATGGCAACAGCAACGCGAGCTGGTGG

SEQ ID NO: 70 corresponds to a DBP1 nucleotide encoding sequence. In a still more preferred embodiment, the first nucleotide sequence comprises, or consists of, sequence SEQ ID NO: 70.

In another more preferred embodiment, the first nucleotide sequence is a sequence with a sequence identity of, at least, 80, 81, 82, 83, 84, 85, 86, 87, 88, 89, 90, 91, 92, 93, 94, 95, 96, 97, 98, or 99% to SEQ ID NO:71

SEQ ID NO:71
ATGGGCAAAAAAACCAAACGCACCGCGGATAGCAGCAGCGAAGATGAAG
AAGAATATGTGGTGGAAAAAGTGCTGGATCGCCGCATGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAGGCAAC
AAACGCAAAAGCAGCTTTAGCAACAGCGCGGATGATATTAAAAGCAAAAAAAAA
CGCGAACAGAGCAACGATATTGCGCGCGGCTTTGAACGCGGCCTGGAACCGG
AAAAAATTATTGGCGCGACCGATAGCTGCGGCGATCTGATGTTTCTGATGAAAT
GGAAAGATACCGATGAAGCGGATCTGGTGCTGGCGAAAGAAGCGAACGTGAA
ATGCCCGCAGATTGTGATTGCGTTTTATGAAGAACGCCTGACCTGGCATGCGT
ATCCGGAAGATGCGGAAAACAAAGAAAAAGAAAGCGCGAAAAGC

SEQ ID NO:71 corresponds to mHP1α nucleotide encoding sequence. In a still more preferred embodiment, the first nucleotide sequence comprises, or consists of, sequence SEQ ID NO:71.

In another more preferred embodiment, the first nucleotide sequence is a sequence with a sequence identity of, at least, 80, 81, 82, 83, 84, 85, 86, 87, 88, 89, 90, 91, 92, 93, 94, 95, 96, 97, 98, or 99% to SEQ ID NO: 72

SEQ ID NO:72
ATGGGCAAAAAAACCAAACGCACCGCGGATAGCAGCAGCAGCGAAGATGAAG
AAGAATATGTGGTGGAAAAAGTGCTGGATCGCCGCGTGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAAGCAAC
AAACGCAAAGCGAACTTTGCGAACGCGGCGGATGATATTAAAAGCAAAAAAAA
ACGCGAACAGAGCAACGATATTGCGCGCGGCTTTGAACGCGGCCTGGAACCG
GAAAAAATTATTGGCGCGACCGATAGCTGCGGCGATCTGATGTTTCTGATGAAA
TGGAAAGATACCGATGAAGCGGATCTGGTGCTGGCGAAAGAAGCGAACGTGA
AATGCCCGCAGATTGTGATTGCGTTTTATGAAGAACGCCTGACCTGGCATGCG
TATCCGGAAGATGCGGAAAACAAAGAAAAAGAAACCGCGAAAAGC

SEQ ID NO: 72 corresponds to HP1α phosphorylated version (HP1αPhos) nucleotide encoding sequence. In a still more preferred embodiment, the first nucleotide sequence comprises, or consists of, sequence SEQ ID NO:72.

In an even more preferred embodiment, the polynucleotide of the invention comprises a first nucleotide sequence selected from the list consisting of SEQ ID NO: 70, SEQ ID NO: 71 and SEQ ID NO: 72, and a second nucleotide sequence selected from the list consisting of: SEQ ID NO: 73, SEQ ID NO: 74, SEQ ID NO: 75, SEQ ID NO: 76, SEQ ID NO: 76, SEQ ID NO: 77, SEQ ID NO: 78, SEQ ID NO: 79, SEQ ID NO: 80, and SEQ ID NO: 81.

**Table 4. Binder element encoding sequences.**

| **Binder** | **DNA Sequence (encoding sequences)** |
|---|---|
| P7 (SEQ ID NO: 73) | GCAACCGTGAAATTCACATACCAAGGCGAAGAAAAACAGGTGGATA TTAGCAAAATCAAGTTCGTGTTCCGTATGGGCCAGGCTATTGAATTT GCTTATGATGAAGGTGGTGGTGCCCTGGGTGTTGGTGTTGTGAGCG AAAAAGATGCACCGAAAGAACTGCTGCAGATGCTGGAAAAGCAA |
| P7 opt (SEQ ID NO: 74) | GCGACCGTGAAATTTACCTATCAGGGCGAAGAAAAACAGGTGGATA TTAGCAAAATTAAATTTGTGTTTCGCATGGGCCAGGCGATTGAATTT GCGTATGATGAAGGCGGCGCGCTGGGCGTGGGCGTGGTGAG CGAAAAAGATGCGCCGAAAGAACTGCTGCAGATGCTGGAAAAACAG |
| P7.C3 (SEQ ID NO: 75) | GCAACCGTGAAATTCACATACCAAGGCGAAGAAAAACAGACGGATA TTAGCAAAATCAGGTTCGTGTTCCGTATGGGCCAGGCTATTGAATTT GCTTATGATGAAGGTGATGGTGTCCTGGGTGTGGGTGTTGTGAGCG AAAAAGATGCACCGAAAGAACTGCTGCAGATGCTGGAAAAGCAA |
| P7.C4 (SEQ ID NO: 76) | GCAACCGTGAAATTCACATACCAAGGCGAAGAAAAACAGGTGGACA TTAGCAAAATCAGGTTCGTGTTCCGTATGGGCCAGGCTATTGAATTT GCTTATGATGAAGGTGGTGGTGTCCTGGGTGTTGGTGTTGTGAACG AAAAAGATGCACCGAGAGAACTGCTGCAGATGCTGGAAAAGCAA |
| L1 (SEQ ID NO: 77) | GCAACCGTGAAATTCACATACCAAGGCGAAGAAAAACAGGTGGATA TTAGCAAAATCAAGATGGTGATCCGTATGGGCCAGATGATTCTGTTT ACTTATGATGAAGGTGGTGGTGCCATGGGTCGGGGTATTGTGAGCG AAAAAGATGCACCGAAAGAACTGCTGCAGATGCTGGAAAAGCAA |
| L2 (SEQ ID NO: 78) | GCAACCGTGAAATTCACATACCAAGGCGAAGAAAAACAGGTGGATA TTAGCAAAATCAAGTACGTGATCCGTTTCGGCCAGAAAATTATGTTT AAATATGATGAAGGTGGTGGTGCCCATGGTAGCGGTATGGTGAGCG AAAAAGATGCACCGAAAGAACTGCTGCAGATGCTGGAAAAGCAA |
| R2 (SEQ ID NO: 79) | GCAACCGTGAAATTCACATACCAAGGCGAAGAAAAACAGGTGGATA TTAGCAAAATCAAGATGGTGATGCGTAACGGCCAGCGTATTCGTTTT ATGTATGATGAAGGTGGTGGTGCCATTGGTCTGGGTGAAGTGAGCG AAAAAGATGCACCGAAAGAACTGCTGCAGATGCTGGAAAAGCAA |
| R2.C6 (SEQ ID NO: 80) | GCAACCGTGAAATTCACATACCAAGGCGAAGAAAAACAGGTGGATA TTAGCAAAATCAAGATGGTAATGCGTAACGGCCAGCGTATTCGTTTT ATGTATGANGNNNGNGGTGGTGCCATTGGTNNGGGGNNAANNNGA GCNAAANAANATGCACCGAAANAACTGCTGCANATGCTGGAAAAGC AA |
| R2.C14 (SEQ ID NO: 81) | GCAACCGTGAAATTCACATACCAAGGCGAAGAAAAACAGGTGGGTA TTAGCAAAATCAAGATGATGATGCGTAACGGCCAGCGTATTCGTTTT ATGTATGATGAAGGTGGTGGTGCCATTGGTCTGGGTGAAGTGAGCG AAAAAGATGCACCGAAAGAACTGCTGCAGATGCTGGAAAAGCAA |

More preferably, the polynucleotide of the invention comprises, or consists of, a nucleotide sequence with a sequence identity of at least 80, 81, 82, 83, 84, 85, 86, 87, 88, 89, 90, 91, 92, 93, 94, 95, 96, 97, 98, or 99%, to a sequence selected from the list consisting of SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92 and SEQ ID NO: 93.
SEQ ID NO: 82 (DBP1-P7 polynucleotide encoding sequence): 
ATGGCGGATCTGCCGCAGAAAGTGAGCAACCTGAGCATTAACAACAAAGAAAA
CGGCGGCGGCGGCAAAAGCAGCTATGTGCCGCCGCATCTGCGCAGCCG
CGGCAAACCGAGCTTTGAACGCCGCAGCCCGAAACAGAAAGATAAAGTGACC
GGCGGCGATTTTTTTCGCCGCGCGGGCCGCCAGACCGGCAACAACGGCGGCT
TTTTTGGCTTTAGCAAAGAACGCAACGGCGGCACCAGCGCGAACTATAACCGC
CGCGGCAGCAGCAACTATAAAAGCAGCGGCAACCGCTGGGTGAACGGCAAAC
ATATTCCGGGCCCGAAAAACGCGAAACTGCAGAAAGCGGAACTGTTTGGCGTG
CATGATGATCCGGATTATCATAGCAGCGGCATTAAATTTGATAACTATGATAAC
ATTCCGGTGGATGCGAGCGGCAAAGATGTGCCGGAACCGATTCTGGGCGGCC
GCACCCGCGGCGGCGGCGGCTTTTTTAACAGCCGCAACAACGGCAGCCGCGA
TTATCGCAAACATGGCGGCAACGGCAGCTTTGGCAGCACCCGCCCGCGCAAC
ACCGGCACCAGCAACTGGGGCAGCATTGGCGGCGGCTTTCGCAACGATAACG
AAAAAAACGGCTATGGCAACAGCAACGCGAGCTGGTGGGGATCCGCAACCGT
GAAATTCACATACCAAGGCGAAGAAAAACAGGTGGATATTAGCAAAATCAAGTT
CGTGTTCCGTATGGGCCAGGCTATTGAATTTGCTTATGATGAAGGTGGTGGTG
CCCTGGGTGTTGGTGTTGTGAGCGAAAAAGATGCACCGAAAGAACTGCTGCAG
ATGCTGGAAAAGCAA
SEQ ID NO: 83 (DBP1-P7.C3 polynucleotide encoding sequence): 
ATGGCGGATCTGCCGCAGAAAGTGAGCAACCTGAGCATTAACAACAAAGAAAA
CGGCGGCGGCGGCGGCAAAAGCAGCTATGTGCCGCCGCATCTGCGCAGCCG
CGGCAAACCGAGCTTTGAACGCCGCAGCCCGAAACAGAAAGATAAAGTGACC
GGCGGCGATTTTTTTCGCCGCGCGGGCCGCCAGACCGGCAACAACGGCGGCT
TTTTTGGCTTTAGCAAAGAACGCAACGGCGGCACCAGCGCGAACTATAACCGC
CGCGGCAGCAGCAACTATAAAAGCAGCGGCAACCGCTGGGTGAACGGCAAAC
ATATTCCGGGCCCGAAAAACGCGAAACTGCAGAAAGCGGAACTGTTTGGCGTG
CATGATGATCCGGATTATCATAGCAGCGGCATTAAATTTGATAACTATGATAAC
ATTCCGGTGGATGCGAGCGGCAAAGATGTGCCGGAACCGATTCTGGGCGGCC
GCACCCGCGGCGGCGGCGGCTTTTTTAACAGCCGCAACAACGGCAGCCGCGA
TTATCGCAAACATGGCGGCAACGGCAGCTTTGGCAGCACCCGCCCGCGCAAC
ACCGGCACCAGCAACTGGGGCAGCATTGGCGGCGGCTTTCGCAACGATAACG
AAAAAAACGGCTATGGCAACAGCAACGCGAGCTGGTGGGGATCCGCAACCGT
GAAATTCACATACCAAGGCGAAGAAAAACAGACGGATATTAGCAAAATCAGGTT
CGTGTTCCGTATGGGCCAGGCTATTGAATTTGCTTATGATGAAGGTGATGGTGT
CCTGGGTGTGGGTGTTGTGAGCGAAAAAGATGCACCGAAAGAACTGCTGCAG
ATGCTGGAAAAGCAA
SEQ ID NO: 84 (DBP1-P7.C4 polynucleotide encoding sequence): 
ATGGCGGATCTGCCGCAGAAAGTGAGCAACCTGAGCATTAACAACAAAGAAAA
CGGCGGCGGCGGCGGCAAAAGCAGCTATGTGCCGCCGCATCTGCGCAGCCG
CGGCAAACCGAGCTTTGAACGCCGCAGCCCGAAACAGAAAGATAAAGTGACC
GGCGGCGATTTTTTTCGCCGCGCGGGCCGCCAGACCGGCAACAACGGCGGCT
TTTTTGGCTTTAGCAAAGAACGCAACGGCGGCACCAGCGCGAACTATAACCGC
CGCGGCAGCAGCAACTATAAAAGCAGCGGCAACCGCTGGGTGAACGGCAAAC
ATATTCCGGGCCCGAAAAACGCGAAACTGCAGAAAGCGGAACTGTTTGGCGTG
CATGATGATCCGGATTATCATAGCAGCGGCATTAAATTTGATAACTATGATAAC
ATTCCGGTGGATGCGAGCGGCAAAGATGTGCCGGAACCGATTCTGGGCGGCC
GCACCCGCGGCGGCGGCGGCTTTTTTAACAGCCGCAACAACGGCAGCCGCGA
TTATCGCAAACATGGCGGCAACGGCAGCTTTGGCAGCACCCGCCCGCGCAAC
ACCGGCACCAGCAACTGGGGCAGCATTGGCGGCGGCTTTCGCAACGATAACG
AAAAAAACGGCTATGGCAACAGCAACGCGAGCTGGTGGGGATCCGCAACCGT
GAAATTCACATACCAAGGCGAAGAAAAACAGGTGGACATTAGCAAAATCAGGTT
CGTGTTCCGTATGGGCCAGGCTATTGAATTTGCTTATGATGAAGGTGGTGGTG
TCCTGGGTGTTGGTGTTGTGAACGAAAAAGATGCACCGAGAGAACTGCTGCAG
ATGCTGGAAAAGCAA
SEQ ID NO: 85 (mHP1α-L1 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCATGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAGGCAAC
AAACGCAAAAGCAGCTTTAGCAACAGCGCGGATGATATTAAAAGCAAAAAAAAA
CGCGAACAGAGCAACGATATTGCGCGCGGCTTTGAACGCGGCCTGGAACCGG
AAAAAATTATTGGCGCGACCGATAGCTGCGGCGATCTGATGTTTCTGATGAAAT
GGAAAGATACCGATGAAGCGGATCTGGTGCTGGCGAAAGAAGCGAACGTGAA
 
ATGCCCGCAGATTGTGATTGCGTTTTATGAAGAACGCCTGACCTGGCATGCGT
ATCCGGAAGATGCGGAAAACAAAGAAAAAGAAAGCGCGAAAAGCGGATCCGC
AACCGTGAAATTCACATACCAAGGCGAAGAAAAACAGGTGGATATTAGCAAAAT
CAAGATGGTGATCCGTATGGGCCAGATGATTCTGTTTACTTATGATGAAGGTGG
TGGTGCCATGGGTCGGGGTATTGTGAGCGAAAAAGATGCACCGAAAGAACTG
CTGCAGATGCTGGAAAAGCAA
SEQ ID NO: 86 (mHP1 α-L2 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCATGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAGGCAAC
AAACGCAAAAGCAGCTTTAGCAACAGCGCGGATGATATTAAAAGCAAAAAAAAA
CGCGAACAGAGCAACGATATTGCGCGCGGCTTTGAACGCGGCCTGGAACCGG
AAAAAATTATTGGCGCGACCGATAGCTGCGGCGATCTGATGTTTCTGATGAAAT
GGAAAGATACCGATGAAGCGGATCTGGTGCTGGCGAAAGAAGCGAACGTGAA
ATGCCCGCAGATTGTGATTGCGTTTTATGAAGAACGCCTGACCTGGCATGCGT
ATCCGGAAGATGCGGAAAACAAAGAAAAAGAAAGCGCGAAAAGCGGATCCGC
AACCGTGAAATTCACATACCAAGGCGAAGAAAAACAGGTGGATATTAGCAAAAT
CAAGTACGTGATCCGTTTCGGCCAGAAAATTATGTTTAAATATGATGAAGGTGG
TGGTGCCCATGGTAGCGGTATGGTGAGCGAAAAAGATGCACCGAAAGAACTG
CTGCAGATGCTGGAAAAGCAA
SEQ ID NO: 87 (mHP1α-R2 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCATGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAGGCAAC
AAACGCAAAAGCAGCTTTAGCAACAGCGCGGATGATATTAAAAGCAAAAAAAAA
CGCGAACAGAGCAACGATATTGCGCGCGGCTTTGAACGCGGCCTGGAACCGG
AAAAAATTATTGGCGCGACCGATAGCTGCGGCGATCTGATGTTTCTGATGAAAT
GGAAAGATACCGATGAAGCGGATCTGGTGCTGGCGAAAGAAGCGAACGTGAA
ATGCCCGCAGATTGTGATTGCGTTTTATGAAGAACGCCTGACCTGGCATGCGT
ATCCGGAAGATGCGGAAAACAAAGAAAAAGAAAGCGCGAAAAGCGGATCCGC
AACCGTGAAATTCACATACCAAGGCGAAGAAAAACAGGTGGATATTAGCAAAAT
CAAGATGGTGATGCGTAACGGCCAGCGTATTCGTTTTATGTATGATGAAGGTG
GTGGTGCCATTGGTCTGGGTGAAGTGAGCGAAAAAGATGCACCGAAAGAACTG
CTGCAGATGCTGGAAAAGCAA
SEQ ID NO: 88 (mHP1 α-R2.C6 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCATGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
 
AAAAAATGAAAGAAGGCGAAAACAACAAACCGCGCGAAAAAAGCGAAGGCAAC
AAACGCAAAAGCAGCTTTAGCAACAGCGCGGATGATATTAAAAGCAAAAAAAAA
CGCGAACAGAGCAACGATATTGCGCGCGGCTTTGAACGCGGCCTGGAACCGG
AAAAAATTATTGGCGCGACCGATAGCTGCGGCGATCTGATGTTTCTGATGAAAT
GGAAAGATACCGATGAAGCGGATCTGGTGCTGGCGAAAGAAGCGAACGTGAA
ATGCCCGCAGATTGTGATTGCGTTTTATGAAGAACGCCTGACCTGGCATGCGT
ATCCGGAAGATGCGGAAAACAAAGAAAAAGAAAGCGCGAAAAGCGGATCCGC
AACCGTGAAATTCACATACCAAGGCGAAGAAAAACAGGTGGATATTAGCAAAAT
CAAGATGGTAATGCGTAACGGCCAGCGTATTCGTTTTATGTATGATGCAGGTG
GTGGTGCCATTGGTCTGGGTGAAGTGAGCGAAAGAGATGCACCGAAAGAACT
GCTGCAGATGCTGGAAAAGCAA
SEQ ID NO: 89 (mHP1α-R2.C14 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCATGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAGGCAAC
AAACGCAAAAGCAGCTTTAGCAACAGCGCGGATGATATTAAAAGCAAAAAAAAA
CGCGAACAGAGCAACGATATTGCGCGCGGCTTTGAACGCGGCCTGGAACCGG
AAAAAATTATTGGCGCGACCGATAGCTGCGGCGATCTGATGTTTCTGATGAAAT
GGAAAGATACCGATGAAGCGGATCTGGTGCTGGCGAAAGAAGCGAACGTGAA
ATGCCCGCAGATTGTGATTGCGTTTTATGAAGAACGCCTGACCTGGCATGCGT
ATCCGGAAGATGCGGAAAACAAAGAAAAAGAAAGCGCGAAAAGCGGATCCGC
AACCGTGAAATTCACATACCAAGGCGAAGAAAAACAGGTGGGTATTAGCAAAAT
CAAGATGATGATGCGTAACGGCCAGCGTATTCGTTTTATGTATGATGAAGGTGG
TGGTGCCATTGGTCTGGGTGAAGTGAGCGAAAAAGATGCACCGAAAGAACTGC
TGCAGATGCTGGAAAAGCAA
SEQ ID NO: 90 (mHP1 α-C14 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCGTGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAAGCAAC
AAACGCAAAGCGAACTTTGCGAACGCGGCGGATGATATTAAAAGCAAAAAAAA
ACGCGAACAGAGCAACGATATTGCGCGCGGCTTTGAACGCGGCCTGGAACCG
GAAAAAATTATTGGCGCGACCGATAGCTGCGGCGATCTGATGTTTCTGATGAAA
TGGAAAGATACCGATGAAGCGGATCTGGTGCTGGCGAAAGAAGCGAACGTGA
AATGCCCGCAGATTGTGATTGCGTTTTATGAAGAACGCCTGACCTGGCATGCG
TATCCGGAAGATGCGGAAAACAAAGAAAAAGAAACCGCGAAAAGCGGCAGCG
CGACCGTGAAATTTACCTATCAGGGCGAAGAAAAACAGGTGGATATTAGCAAA
ATTAAATTTGTGTTTCGCATGGGCCAGGCGATTGAATTTGCGTATGATGAAGGC
GGCGGCGCGCTGGGCGTGGGCGTGGTGAGCGAAAAAGATGCGCCGAAAGAA
CTGCTGCAGATGCTGGAAAAACAG
SEQ ID NO: 91 (HP1αPhos-P7 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCGTGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAAGCAAC
AAACGCAAAGCGAACTTTGCGAACGCGGCGGATGATATTAAAAGCAAAAAAAA
ACGCGAACAGAGCAACGATATTGCGCGCGGCTTTGAACGCGGCCTGGAACCG
GAAAAAATTATTGGCGCGACCGATAGCTGCGGCGATCTGATGTTTCTGATGAAA
TGGAAAGATACCGATGAAGCGGATCTGGTGCTGGCGAAAGAAGCGAACGTGA
AATGCCCGCAGATTGTGATTGCGTTTTATGAAGAACGCCTGACCTGGCATGCG
TATCCGGAAGATGCGGAAAACAAAGAAAAAGAAACCGCGAAAAGCGGCAGCG
CGACCGTGAAATTTACCTATCAGGGCGAAGAAAAACAGGTGGATATTAGCAAA
ATTAAATTTGTGTTTCGCATGGGCCAGGCGATTGAATTTGCGTATGATGAAGGC
GGCGGCGCGCTGGGCGTGGGCGTGGTGAGCGAAAAAGATGCGCCGAAAGAA
CTGCTGCAGATGCTGGAAAAACAG
SEQ ID NO: 92 (HP1αPhos-L1 polynucleotide encoding sequence): 
ATGGGCAAAAAAACCAAACGCACCGCGGATAGCAGCAGCAGCGAAGATGAAG
AAGAATATGTGGTGGAAAAAGTGCTGGATCGCCGCGTGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAAGCAAC
AAACGCAAAGCGAACTTTGCGAACGCGGCGGATGATATTAAAAGCAAAAAAAA
ACGCGAACAGAGCAACGATATTGCGCGCGGCTTTGAACGCGGCCTGGAACCG
GAAAAAATTATTGGCGCGACCGATAGCTGCGGCGATCTGATGTTTCTGATGAAA
TGGAAAGATACCGATGAAGCGGATCTGGTGCTGGCGAAAGAAGCGAACGTGA
AATGCCCGCAGATTGTGATTGCGTTTTATGAAGAACGCCTGACCTGGCATGCG
TATCCGGAAGATGCGGAAAACAAAGAAAAAGAAACCGCGAAAAGCGGCAGCG
CAACCGTGAAATTCACATACCAAGGCGAAGAAAAACAGGTGGATATTAGCAAAA
TCAAGATGGTGATCCGTATGGGCCAGATGATTCTGTTTACTTATGATGAAGGTG
GTGGTGCCATGGGTCGGGGTATTGTGAGCGAAAAAGATGCACCGAAAGAACT
GCTGCAGATGCTGGAAAAGCAA
SEQ ID NO: 93 (HP1αPhos-R2 polynucleotide encoding sequence):

ATGGGCAAAAAAACCAAACGCACCGCGGATAGCAGCAGCAGCGAAGATGAAG
AAGAATATGTGGTGGAAAAAGTGCTGGATCGCCGCGTGGTGAAAGGCCAGGT
GGAATATCTGCTGAAATGGAAAGGCTTTAGCGAAGAACATAACACCTGGGAAC
CGGAAAAAAACCTGGATTGCCCGGAACTGATTAGCGAATTTATGAAAAAATATA
AAAAAATGAAAGAAGGCGAAAACAACAAACCGCGCGAAAAAAGCGAAAGCAAC
AAACGCAAAGCGAACTTTGCGAACGCGGCGGATGATATTAAAAGCAAAAAAAA
ACGCGAACAGAGCAACGATATTGCGCGCGGCTTTGAACGCGGCCTGGAACCG
GAAAAAATTATTGGCGCGACCGATAGCTGCGGCGATCTGATGTTTCTGATGAAA
TGGAAAGATACCGATGAAGCGGATCTGGTGCTGGCGAAAGAAGCGAACGTGA
AATGCCCGCAGATTGTGATTGCGTTTTATGAAGAACGCCTGACCTGGCATGCG
TATCCGGAAGATGCGGAAAACAAAGAAAAAGAAACCGCGAAAAGCGGCAGCG
CAACCGTGAAATTCACATACCAAGGCGAAGAAAAACAGGTGGATATTAGCAAAA
TCAAGATGGTGATGCGTAACGGCCAGCGTATTCGTTTTATGTATGATGAAGGTG
GTGGTGCCATTGGTCTGGGTGAAGTGAGCGAAAAAGATGCACCGAAAGAACTG
CTGCAGATGCTGGAAAAGCAA

Likewise, the polynucleotide of the invention may be comprised in a gene construction. Thus, other aspect of the invention relates to a gene construct, hereinafter, the "gene construct of the invention", which comprises the polynucleotide of the invention.

To generate gene constructs, molecular tools known to those skilled in the art can be used, including molecular cloning and use of polymerase chain reaction (PCR), restriction enzymes and ligases. This gene construct of the invention may comprise the polynucleotide of the invention, operatively linked to a regulatory sequence for the expression of the nucleic acid of the invention, thereby constituting an expression cassette.

"Operatively linked" refers to a juxtaposition in which the components of the gene construction have a relationship that allows them to function in the intended manner. A control sequence "operatively linked" to the nucleic acid, is linked to the nucleic acid in such a manner as to achieve the expression of the polynucleotide of the invention.

"Control sequence" refers to nucleic acid sequences that affect the expression of the sequences to which they are linked. Such control sequences include, for example, but are not limited to, promoters, initiation signals, termination signals, enhancers or silencers. The term "control sequences" is intended to include those components whose presence is necessary for the expression and may also include additional components whose presence is advantageous.

The control sequences depend on the origin of the cell in which the nucleic acid of the invention is to be expressed. In a preferred embodiment, the expression control sequences attached to the polynucleotide of the invention are functional in prokaryotic (bacterial) cells. In another preferred embodiment, the expression control sequences attached to the polynucleotide of the invention are functional in eukaryotic cells and/or organisms. These alternative organisms could include other bacteria such as *Bacillus subtilis* or *Corynebacterium glutamicum;* yeasts such as *Komagataella phaffii* (syn. *Pichia pastoris), Schizosaccharomyces pombe* or *Sacharomyces cerevisiae,* as well as filamentous fungi from the genera *Aspergillus (e.g A. niger, A. oryzae), Trichoderma* (*T. reesei*) and *Neurospora.* Furthermore, the baculovirus-based technology could be another alternative since it has become an established commercial manufacturing platform for the production of viral vaccines and gene therapy vectors.

### Host cell of the invention

The polynucleotide or the gene construct of the invention may be introduced into a cell, referred to as a host cell, including prokaryotic or eukaryotic cells, for example, but not limited to, as naked nucleic acid or by means of a vector.

The term "vector", as used herein, refers to a construct capable of delivering, and optionally expressing, the polynucleotide of the invention into a host cell. Examples of vectors include, but are not limited to, viral vectors, RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. The vectors can be stable and can be self-replicating. The vector can be a cloning vector, suitable for propagation and for obtaining polynucleotides, gene constructs or expression vectors incorporated to several heterologous organisms. Suitable vectors include prokaryotic expression vectors (*e.g.,* pET28, pSEVA, pET and their derivatives), mpl8, mpl9, pBR322, pMB9, ColEl, pCRI, RP4, phages and shuttle vectors (e.g., pSA3 and pAT28), and eukaryotic expression vectors based on viral vectors (e.g., adenoviruses, adeno- associated viruses as well as retroviruses and lentiviruses), as well as non-viral vectors such as pSilencer 4.1-CMV (Ambion^{®}, Life Technologies Corp., Carslbad, CA, US), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-I, pML2d and pTDTI.

Thus, other aspect of the invention relates to a host cell, hereinafter the "host cell of the invention" which comprises the polynucleotide, gene construct or the chimeric protein of the invention. The present invention also relates to a cell population comprising the host cell of the invention.

The introduction of said polynucleotide or gene construct may be carried out by methods known in the state of the art.

### Composition of the invention

The chimeric protein, of the invention can be present in a composition, hereinafter the "composition of the invention". Thus, another aspect of the present invention relates to a composition, which comprises the chimeric protein. The composition of the invention may be formulated as a liquid formulation or a solid formulation, such as a hydrogel formulation. Particularly, composition of the present invention can be formulated in a variety of particular forms known in the state of the art, such as, but not limited to, in aqueous or non-aqueous solutions, emulsions or suspensions, and solid-phase structures like hydrogels.

Examples of non-aqueous solutions are, without being limited to, protein liquid droplets, propylene glycol, polyethylene glycol, vegetable oils, such as olive oil, or organic esters, such as such as ethyl oleate.

Examples of aqueous solutions are, without being limited to, water, alcoholic solutions in water, or saline media. Aqueous solutions may be buffered or not and may have additional active or inactive components. Additional components include salts to modulate ionic strength, preservatives including, but not limited to, antimicrobial agents, antioxidants, antimicrobial agents, antioxidants, chelators, or the like, or nutrients, including glucose, dextrose, vitamins, and minerals. To prevent the formation of liquid droplets in the stock solution and for stability it may contain imidazole, filtered tap water, Phosphate Buffered Saline, L-Arginine and L-Glutamic Acid.

Alternatively, the compositions may be prepared in solid form.

Compositions may be combined with various vehicles or inert excipients, including, without being limited to binders, such as microcrystalline cellulose, gum tragacanth, or gelatin; excipients, such as starch or lactose; dispersing agents, such as alginic acid or starch; and alginic acid or corn starch; lubricants, such as magnesium stearate, glidants, such as coloured silicon dioxide, silicon such as colloidal silicon dioxide; sweetening agents, such as sucrose or saccharose; or flavouring agents, such as saccharin; or flavouring agents, such as peppermint or methyl salicylate.

In a preferred embodiment, alone or in combination with other of preferred embodiments, the composition of the invention is formulated as a liquid formulation. In a more preferred embodiment, the composition of the invention is lyophilized.

As used herein, the term "lyophilized" means that the composition of the invention, in particular, the composition of the invention formulated as a liquid formulation, has undergone a freeze-drying process. The term "freeze-drying" refers to the rapid freezing and dehydration of a product under high vacuum conditions.

Inventors have tested the chimeric protein of the invention at different concentrations. In a preferred embodiment, alone or in combination with other preferred embodiments, the composition of the invention comprises the chimeric protein of the invention at a concentration of 0.5 µM-5 µM. Preferably the concentration is close to 1 µM to avoid phase separation by self-assembly before interacting with the pathogen. In a more preferred embodiment, the composition of the invention comprises the chimeric protein of the invention, at a concentration of 0.5 to 3 µM (including the end values of the range). Even more preferably, at a concentration selected from the list consisting of 0.5, 0.6, 0.7, 0.8, 0.9, 1, 1.1, 1.2, 1.3, 1.4 and 1.5 µM.

Some terms used to define the composition of the invention have been described in previous aspects of the invention, and apply equally, as well as its preferred embodiments, to the present aspect of the invention.

### Hydrogel of the invention

Inventors have developed a hydrogel formulation based on the chimeric protein of the invention when it is phosphorylated, or the composition of the invention that comprises said phosphorylated chimeric protein, which can be used in close system aquaculture, but also in open system aquaculture. Furthermore, inventors have also characterized hydrogel properties (microarchitecture, surface features, flow and deformation behaviour, chemical resistance) showing physical properties of interest, as it is shown in Example 2.7. Physical properties of phosphorylated HP1α hydrogels.

Thus, an aspect of the invention relates to a hydrogel comprising the chimeric protein of the invention wherein the first polypeptide is a phosphorylated HP1α or the composition of the invention when it comprises said chimeric protein (i.e. the chimeric protein of the invention wherein the first polypeptide is a phosphorylated HP1α), , hereinafter, the "hydrogel of the invention".

Some of the terms used to define the hydrogel of the invention have been described in previous aspects of the invention, and apply equally, as well as its preferred embodiments, including the corresponding chimeric protein preferred embodiments, to the present aspect of the invention.

The term "hydrogel" as used herein, refers to a three-dimensional network system, which is highly dynamic, by reversibly assembling and disassembling in response to some environmental condition, such as phosphorylation of a protein undergoing phase separation, in the present invention, the chimeric protein first polypeptide. Furthermore, the hydrogel of the invention can have a solid, semi-solid or semi-liquid texture.

Inventors have performed the phosphorylation of HP1α (HP1αPhos), obtaining a hydrogel. Furthermore, the HP1αPhos based hydrogel showed appropriate properties, as previously mentioned.

In a preferred embodiment, alone or in combination with other preferred embodiments, the hydrogel of the invention comprises the chimeric protein of the invention, wherein the first polypeptide is a phosphorylated HP1α, and wherein HP1α comprises, or consists of, an amino acid sequence with a sequence similarity and/or identity of, at least, 80, 81, 82, 83, 84, 85, 86, 87, 88, 89, 90, 91, 92, 93, 94, 95, 96, 97, 98, or 99% to SEQ ID NO: 3, wherein sequence similarity is computed according algorithm EMBOSS Needle using the BLOSUM62 matrix, having a Gap Open penalty of 10.0, a Gap Extend penalty of 0.5, or a composition comprising said chimeric protein.

More preferably, the hydrogel of the invention comprises the chimeric protein of the invention, wherein the first polypeptide is a phosphorylated HP1α, and wherein HP1α comprises, or consists of, amino acid sequence SEQ ID NO: 3.

In a preferred embodiment of the hydrogel of the invention, alone or in combination with other preferred embodiments, the HP1α is phosphorylated in serine residues at positions 10, 11, 12 and 13 of SEQ ID NO: 3

In a preferred embodiment of the hydrogel of the invention, alone or in combination with other preferred embodiments, the second polypeptide of the chimeric protein of the invention comprises an amino acid sequence with a sequence similarity and/or identity of, at least, 80, 81, 82, 83, 84, 85, 86, 87, 88, 89, 90, 91, 92, 93, 94, 95, 96, 97, 98, or 99% to an amino acid sequence selected from the list consisting of SEQ ID NO: 5, SEQ ID NO: 33, SEQ ID NO: 14, SEQ ID NO: 42 SEQ ID NO 17, and SEQ ID NO: 45, wherein sequence similarity is computed according algorithm EMBOSS Needle using the BLOSUM62 matrix, having a Gap Open penalty of 10.0, a Gap Extend penalty of 0.5, or a composition comprising said chimeric protein.

In another preferred embodiment of the hydrogel of the invention, alone or in combination with other preferred embodiments, the second polypeptide of the chimeric protein of the invention comprises, or consists of, amino acid sequence SEQ ID NO: 99. 
SEQ ID NO: 99
AX1VKFTYQGEEKQX2X3X4SKIX5X6X7X8RX9GQX10IX11FX12YBX13GX14GX15X16GX17
GX18VX19EX20DAPX21ELLQMLEX22Q
 wherein
X₁ is T or I,
X₂ is V or T,
X₃ is D or G,
X₄ is I or V,
X₅ is K, Q or R,
X₆ is Y, F, N, R, S, W, M, V or D,
X₇ is V or M,
X₈ is Y, F, H, A, W, I, or M,
X₉ is H, K, Y, N, F, L, D, R, M or S,
X₁₀ is I, A, Y, W, H, S, V, K, F, R or M,
X₁₁ is Y, E, G, S, A, W, H, L, M, R, K or D,
X₁₂ is N, A, W, S, G, H, T, K, R, M, Q or I,
B is D or N,
X₁₃ is E or A,
X₁₄ is G or D,
X₁₅ is A or V,
X₁₆ is I, L, Y, W, N, K, H, M A, R, or S,
X₁₇ is N, V, H, Y, G, A, R, S, L, F, M or W,
X₁₈ is I, V, K, W, Y, L, M, E or D,
X₁₉ is S or N,
X₂₀ is K, E or R,
X₂₁ is K, E or R, and
X₂₂ is K or E.

More preferably, the second polypeptide comprises, or consists of, amino acid sequence SEQ ID NO: 99, wherein X₁ is T, X₂ is V, X₃ is D, X₄ is I, X₅ is K, X₇ is V, B is D, X₁₃ is E, X₁₄ is G, X₁₅ is A, X₁₉ is S, X₂₀ is K, X₂₁ is K and X₂₂ is K.

Even more preferably, the second polypeptide comprises, or consists of, an amino acid sequence selected from the list consisting of SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58.

In a preferred embodiment of the hydrogel of the invention, alone or in combination with other preferred embodiments ), the chimeric protein concentration is between 50 to 800 µM. More preferably, the chimeric protein concentration is 80 to 500 µM. Even more preferably, the chimeric protein concentration is between 100 to 150 µM (including the end values of the range).

In the present invention, the hydrogel of the invention may be lyophilized to use it in characterization techniques under typical lyophilizer conditions. Thus, in a preferred embodiment, alone or in combination with other preferred embodiments, the hydrogel of the invention is lyophilized.

As used herein, the term "lyophilized" means that the hydrogel has undergone a freeze-drying process. The term "freeze-drying" refers to the rapid freezing and dehydration of a product under high vacuum conditions.

A hydrogel may be further characterized by its porous architecture, such as pore diameter. In a preferred embodiment, the lyophilized hydrogel of the invention comprises pores with a diameter between 10 and 100 µm (including the end values of the range). More preferably, the lyophilized hydrogel of the invention comprises pores with a diameter between 14 and 80 µm.

In another preferred embodiment, alone or in combination with other preferred embodiments, the thickness of the hydrogel of the invention ranges between 5 to 1000 µm. More preferably, the thickness of the hydrogel is between 10 to 500 µm. Even more preferably, the thickness of the hydrogel is between 50 to 250 µm.

In other aspect, the invention relates to a method for the obtention of the hydrogel of the invention, hereinafter referred as the "method for the hydrogel obtention of the invention" which comprises the following steps:
a) phosphorylation of the chimeric protein of the invention, and
b) evaporation step, which comprises the incubation at a temperature of 10ºC to 40ºC, preferably at a temperature of 15ºC to 30ºC, of the chimeric protein of the invention, or the composition comprising said chimeric protein of the invention.

Techniques and methods for protein phosphorylation are well known in the state of the art, such as, but without limitation to, the use of kinase enzymes after a protein purification step or the co-transformation before production stage, in *E. coli.* In the present invention, a co-transformation approach was used, which consists in introducing two plasmids into the expression host organism, *E. coli.* One of those plasmids contains a sequence of the suited kinase, particularly a casein kinase II. It was also used a pRSTDuet-1 plasmid with the two subunits of that kinase with co-expression (simultaneously) with the protein of interest by performing the phosphorylation process into the bacteria. However, other techniques and methods for protein phosphorylation can be used.

### Filter system

Inventors have also developed a filter system based on the hydrogel of the invention (Example 2.8. Phosphorylated HP1α can form thin hydrogel films on commercial scaffolds), which has application as a pathogen trapping system, and additionally in aquaculture applications such as aquaculture pathogen detection/control in both, closed and open system aquaculture.

Other aspect of the invention relates to a filter system, which comprises the hydrogel of the invention and a scaffold material, wherein the hydrogel of the invention is arranged on the surface of the scaffold, hereinafter the "filter system of the invention".

The term "scaffold material" (also referred in the present document simply as "scaffold") can be defined as any polymeric or metallic material where the hydrogel of the invention can be functionalised on its surface.

Examples of scaffolds known in the state of the art include, without limitation to Low-density polyethylene (LDPE), Polypropylene (PP), Polyvinyl chloride (PVC), Polystyrene (PS), Nylon, nylon 6, nylon 6,6., Teflon (Polytetrafluoroethylene), Thermoplastic polyurethanes (TPU) and high-density polyethylene (HDPE) derived material.

Thus, in a preferred embodiment of the invention the scaffold is selected from the list consisting of Low-density polyethylene (LDPE), Polypropylene (PP), Polyvinyl chloride (PVC), Polystyrene (PS), Nylon, nylon 6, nylon 6,6., Teflon (Polytetrafluoroethylene), Thermoplastic polyurethanes (TPU) and high-density polyethylene (HDPE) derived material.

More preferably, the scaffold is a high-density polyethylene (HDPE) derived material.

A well-known commercial substrate used as part of aquaculture filtering systems is the kaldnes scaffold, which is made on virgin high-density polyethylene (v-HDPE). Inventors have coated the surface of a kaldnes based scaffold, thus developing a filter formulation which have application as a pathogen trapping system. Thus, in a still more preferred embodiment, the scaffold is a kaldnes scaffold.

Other aspect of the invention relates to a method for the obtention of the filter system of the invention, which comprises:
- the obtention of the hydrogel of the invention, which has been previously explained and defined in a previous aspect of the invention, the "method for the hydrogel obtention of the invention", whose steps and preferred embodiments apply equally herein; and
- coating the surface of a scaffold material, preferably, wherein the coating comprises incubating the material scaffold with the hydrogel of the invention and performing an evaporation step.

Preferably, the evaporation step comprises an incubation at a temperature between 20 to 40 ºC (including the end values of the range) during at least 24 hours, or incubating the material scaffold with the hydrogel of the invention at room temperature during, at least, 72 hours.

Preferred embodiments of the scaffold material of the filter system of the invention apply equally to the method for the obtention of said filter.

The terms used to define the present aspect of the invention have been previously described, and apply equally, as well as their preferred embodiments, to the present aspect.

### Kit of the invention

The chimeric protein, composition, hydrogel, or filter of the invention may be comprised by a kit. Thus, other aspect of the present invention relates to a kit, hereinafter the "kit of the invention", comprising the chimeric protein, composition, hydrogel or the filter of the invention.

Moreover, the kit of the invention may comprise other components useful in the implementation of the present invention, such as, buffers, delivery vehicles, carriers, positive and/or negative control components, etc. In addition of the above-mentioned components, the kit may also include instructions for carrying out the object of the invention. These instructions may be present in said kit in a variety of forms, one or more of which may be present in the kit. One form in which these instructions may be present is as printed information on a suitable medium or substrate, e.g., a sheet or sheets of paper on which the information is printed, on the kit packaging, on a package insert, and so on. Another medium would be a computer readable medium, e.g. CD, USB, etc., on which the information has been recorded. Another medium that may be present is a website address that can be used via the Internet to access the information at a remote site. Any convenient medium may be present in the kits.

The terms used to define the kit of the invention have already been described in previous aspects of the present invention and apply equally, as well as its preferred embodiments, to the kit of the invention.

### Uses of the invention

Other aspect of the invention relates to the use of the chimeric protein, polynucleotide, gene construct, host cell, composition, hydrogel, filter, or the kit of the invention, for pathogen control, in a system, preferably an aquatic system or in a sample hereinafter the "pathogen control use of the invention", preferably wherein the pathogen is an aquatic pathogen, more preferably an aquaculture pathogen.

The term "pathogen control" as used herein refers to controlling, reducing and/or eliminating a pathogen, or for preventing and/or reducing/inhibiting the growth, colonization and/or spreading of the pathogen in a system. In the present invention, pathogen control is based on the pathogen trapping/retention in the system by the chimeric protein of the invention, and related formulations previously described along the description.

As used herein, the term "controlling", "reducing" or "eliminating" refers to any measurable decrease in the amount of pathogen with respect to the initial amount of pathogen, in particularly, it may mean a decrease by 10%, 20%, 30%, 40%, 50%, 60%, 70%, 80%, 90%, 95%, 98% and/or 99% of the original pathogen count.

Other aspect of the invention relates to the use of the chimeric protein, polynucleotide, gene construct, host cell, composition, hydrogel, filter, or the kit of the invention for pathogen retention, in a system, preferably an aquatic system,or in a sample, hereinafter the pathogen retention use of the invention.

Furthermore, the chimeric protein, polynucleotide, gene construct, host cell, composition, hydrogel, filter, or the kit of the invention can be used for *in vitro* pathogen detection in a sample. Thus, other aspect of the invention relates to the use of the chimeric protein, polynucleotide, gene construct, host cell, composition, hydrogel, filter, or the kit of the invention, for *in vitro* pathogen detection in a sample, hereinafter the "pathogen detection use of the invention", preferably wherein the pathogen is an aquatic pathogen, more preferably an aquaculture pathogen.

The term "system", as used herein, can be defined as any medium or surface wherein presence of pathogens is possible and on which the chimeric protein, polynucleotide, gene construct, host cell, composition, hydrogel, filter, or the kit of the invention can be used for pathogen control/retention.

Regarding this pathogen control use of the invention and pathogen retention use of the invention, although not limited to, preferably encompass the use in aquatic systems. The term "aquatic system", encompasses, without limitation to, lakes, rivers, oceans, seas, bays, estuaries, coastal lagoons, fountains, ponds (e.g., fish ponds), canals, aquariums, aquaculture systems, water holding or conveying systems, water reservoirs, open drinking water systems, brackish water environments and waste water. More preferably, the aquatic system is an aquaculture system.

In another preferred embodiment, alone or in combination with other preferred embodiments, the aquatic system is a closed aquatic system or an open aquatic system.

As used herein, "closed aquatic system", may be referred as to a system that doesn't exchange any matter with its surroundings, wherein the matter is water.

As used herein, "open aquatic system" refers to natural water bodies such as, but without limitation to, oceans, sea, bays, estuaries, coastal lagoons, lakes, or rivers.

As indicated above, the pathogen control and pathogen retention uses of the invention may also encompass the use in a sample.

In the present invention, the term "sample" refers to a part or small quantity of a thing which is considered representative of the whole and which is taken or separated from it for the purpose of study, analysis or experimentation.

Examples of samples include, without limitation to, water, an environmental sample, an isolated biological sample (such as sputum, saliva, whole blood, serum, plasma, urine, feces, ejaculate, a buccal or buccal-pharyngeal swab, pleural fluid, peritoneal fluid, pericardic fluid, cerebrospinal fluid, intra-articular fluid, bronchial aspirates, or bronchioalveolar lavages, preferably saliva, whole blood or urine), or a food sample. Thus, in a preferred embodiment, alone or in combination with other preferred embodiments, the sample is selected from the group consisting of water, an environmental sample, an isolated biological sample, a food sample, a feed sample, and an environmental sample.

The water sample may be either from fresh water/aquatic system or marine aquatic/water system.

The term "isolated biological sample", refers to any sample isolated from a subject and includes, but is not limited to, biological fluids from an individual, obtained by any method known to a person skilled in the art for that purpose.

In a particular embodiment of any of the uses of the invention, alone or in combination with other preferred embodiments, the pathogen is a toxin. Preferably, the toxin is produced by an aquatic organism, including aquatic pathogens.

The term "pathogen", as previously mentioned in the first aspect of the invention, may be defined as a microorganism or infectious agent, which causes or can cause a disease or infection, including viruses, bacteria, protozoans, prions, viroids or any other infectious agent. In the present invention, the term "pathogen" may also encompass toxins. The term "toxin" may be defined as a naturally occurring molecule that is injurious to some living organism.

In a preferred embodiment of the uses of the invention, alone or in combination with other preferred embodiments, the pathogen is a bacterium (which may or may not be antibiotic resistant) or a virus.

In another preferred embodiment of the uses of the invention, alone or in combination with other preferred embodiments, the pathogen is an aquatic pathogen, more preferably an aquaculture pathogen. The terms "aquatic pathogen" and "aquaculture pathogen" have already been described in a previous aspect of the present invention and apply equally, as well as their preferred embodiments, to the uses of the invention.

In a more preferred embodiment of the uses of the invention, the aquaculture pathogen is *Yersinia ruckeri* sp. or betanodavirus.

In another preferred embodiment of the uses of the invention, alone or in combination with other preferred embodiments, the pathogen concentration ranges between 0,022 nM to 500 nM.

In a more preferred embodiment of the uses of the invention, alone or in combination with other preferred embodiments, when the use is referred to the hydrogel or the filter system of the invention, the pathogen concentration ranges between 5 to 250 nM (including the end values of the range). Even more preferably, the pathogen concentration is selected from the list consisting of 5, 10, 15, 20, 25, 30, 35, 40, 45, 50, 55, 60, 65, 70, 75, 80, 85, 90, 95, 100, 105, 110, 115, 120, 125, 130, 140, 145, 150, 155, 160, 165, 170, 175, 180, 185, 190, 195, 200, 205, 210, 215, 220, 225, 230, 235, 240, 245 and 250 nM.

In another more preferred embodiment of the uses of the invention, alone or in combination with other preferred embodiments, the pathogen concentration ranges between 0.11 to 200 nM (including the end values of the range).

A preferred embodiment provides any of the uses of the invention, alone or in combination with other preferred embodiments, at a temperature between 4 to 40ºC (including the end values of the range). More preferably, the temperature is ranging between 15 and 25ºC (including the end values of the range).

A preferred embodiment provides any of the uses of the invention, alone or in combination with other preferred embodiments, at a pH of 7 to 8.2. More preferably, the pH is 7 to 8.2.

The terms used to define the uses of the invention have been already described in previous aspects of the present invention and apply equally, as well as its preferred embodiments herein.

In a still more preferred embodiment, alone or in combination with other preferred embodiments, when the chimeric protein of the invention is DBP1-P7, DBP1-P7.C3, DBP1-P7.C4 or HP1αPhos-P7 chimeric protein of the invention, the aquaculture pathogen is a betanodavirus, preferably a RGNNV (red-spotted grouper nervous necrosis virus) genotype.

In other still more preferred embodiment, alone or in combination with other preferred embodiments, when the chimeric protein of the invention is HP1α-L1, HP1α-L2, HP1α-R2, HP1α-R2.C6, HP1α-R2.C14, HP1αPhos-L1, HP1αPhos-R2, chimeric protein of the invention, the aquaculture pathogen is *Yersinia ruckeri* sp.

Additionally, inventors have demonstrated that the chimeric proteins are able to undergo phase separation in fresh and marine water, two basic fish farm conditions. In the case of DBP1-containing chimeras, better phase separation was displayed in marine water conditions.

Thus, a preferred embodiment, alone or in combination with other preferred embodiments, provides any of the uses of the invention in marine water, when the chimeric protein of the invention comprises a DBP1 polypeptide.

A preferred embodiment, alone or in combination with other preferred embodiments, provides any of the uses of the invention in marine water or fresh water, although preferably in fresh water when the chimeric protein of the invention comprises a HP1α polypeptide.

Besides, the uses of the invention can be performed in closed system aquaculture. Closed system aquaculture (CSA) is any aquaculture system with a controlled interface between the farm organisms and the natural environment. When used in CSA, the chimeric protein of the invention takes advantage of the liquid-liquid phase separation capabilities, forming liquid droplets that act as a flocculant solution added directly to the water to specifically bind the pathogen by the binder element and block the targeted pathogen. Thus, pathogens are inactivated by being trapped into liquid droplets or by being coated by the chimera. These pathogen-chimera complexes could be flocculated and be eliminated from the water by, for instance, sedimentation or filtration.

Thus, a preferred embodiment provides the uses of the invention in CSA.

In the case of the other formulation, the hydrogel or the filter of the invention (or any other product comprising it), can be also used in OSA, taking advantage of the hydrogel properties of said aspects of the invention. Open system aquaculture (OSA) generally refers to fish farming in natural water bodies such as oceans, bays, estuaries, coastal lagoons, lakes, or rivers. When used in OSA, the hydrogel of the invention or the filter of the invention retains the targeted pathogen directly from water as water flows over it.

Thus, another preferred embodiment provides the use of the hydrogel or the filter of the invention in OSA, for pathogen control, pathogen retention or pathogen detection.

### Methods of the invention

In other aspect, the invention refers to a method for pathogen control in a system, preferably in an aquatic system, or in a sample, which comprises the addition of the chimeric protein (pathogen trapping or coating system), polynucleotide, gene construct, host cell, composition, hydrogel, filter, or the kit of the invention to the system, preferably an aquatic system, or sample, , preferably wherein the pathogen is an aquatic pathogen, more preferably an aquaculture pathogen.

Preferably, the system is an aquatic system, more preferably, a closed aquatic system or an open aquatic system.

As used herein, "closed aquatic system", may be referred as to a system that doesn't exchange any matter with its surroundings, wherein the matter is water.

As used herein, "open aquatic system" refers to natural water bodies such as, but without limitation to, oceans, sea, bays, estuaries, coastal lagoons, lakes, or rivers.

In other aspect, the invention refers to a method for *in vitro* pathogen detection, in a sample, preferably wherein the pathogen is an aquatic pathogen, more preferably an aquaculture pathogen, hereinafter the "detection method of the invention", which comprises a) the addition of the chimeric protein (pathogen trapping or coating system), polynucleotide, gene construct, host cell, composition, hydrogel, filter, or the kit of the invention to the sample.

The terms "pathogen control", "pathogen detection", "aquatic pathogen" and "aquaculture pathogen" have been defined/mentioned in the uses of the invention, and apply equally, as well as their preferred embodiments to the methods of the invention.

In the present invention, the term "sample" refers to a part or small quantity of a thing which is considered representative of the whole and which is taken or separated from it for the purpose of study, analysis or experimentation.

Examples of samples include, without limitation to, water, an environmental sample, an isolated biological sample (such as sputum, saliva, whole blood, serum, plasma, urine, feces, ejaculate, a buccal or buccal-pharyngeal swab, pleural fluid, peritoneal fluid, pericardic fluid, cerebrospinal fluid, intra-articular fluid, bronchial aspirates, or bronchioalveolar lavages, preferably saliva, whole blood or urine), or a food sample. Thus, in a preferred embodiment, alone or in combination with other preferred embodiments, the sample is selected from the group consisting of water, an environmental sample, an isolated biological sample, a food sample, a feed sample, and an environmental sample.

The water sample may be either from fresh water system or marine water system.

The term "isolated biological sample", refers to any sample isolated from a subject and includes, but is not limited to, biological fluids from an individual, obtained by any method known to a person skilled in the art for that purpose.

The detection method of the invention, based on the trapping/retention of the pathogen by the chimeric protein, can further comprise an amplification step of the nucleotide sequence of the trapped/retained pathogen.

Thus, in some embodiments, the detection method of the invention further comprises an amplification step after the step a).

The amplification step can be performed by amplification methods known in the state of the art. Examples of amplification methods include without limitation to, polymerase chain reactions (PCR), including qPCR, recombinase polymerase amplification (RPA), or loop-mediated isothermal amplification (LAMP).

Thus, in a more preferred embodiment of the detection method of the invention, the amplification step is carried out by a method selected from the list consisting of PCR, RPA and LAMP.

Furthermore, the detection method of the invention may comprise an additional step after the step a), of culturing the trapped/retained pathogen in said step a) in an appropriate culture medium, depending of the pathogen detected.

Thus, in some embodiments, the detection method of the invention further comprises the culturing of the pathogen after the step a).

Culture relies on the ability of pathogens to grow and multiply on laboratory media. These culture may give either qualitative or quantitative information on the number and type of viable microorganisms present in the sample. A series of steps may be required before a definitive identification can be confirmed, which may include pre-enrichment, selective enrichment, plating on selective media, and then biochemical or serological confirmatory tests.

In a preferred embodiment of any of the methods of the invention, alone or in combination with other preferred embodiments, the pathogen is a toxin. Preferably, the toxin is produced by an aquatic organism, including aquatic pathogens.

A preferred embodiment, alone or in combination with other preferred embodiments, provides any of the methods of the invention in marine water, when the chimeric protein of the invention comprises a DBP1 polypeptide.

A preferred embodiment, alone or in combination with other preferred embodiments, provides any of the methods of the invention in marine water or fresh water, although preferably in fresh water when the chimeric protein of the invention comprises a HP1α polypeptide.

A preferred embodiment provides any of the methods of the invention, alone or in combination with other preferred embodiments, wherein the method is carried out at a temperature between 4 to 40ºC (including the end values of the range). More preferably, the temperature is of 15 to 25ºC (including the end values of the range).

A preferred embodiment provides any of the methods of the invention, alone or in combination with other preferred embodiments, wherein the method is carried out at a pH of 7 to 8.2. More preferably, the pH is 7 to 8.2.

In another preferred embodiment of the methods of the invention, alone or in combination with other preferred embodiments, the pathogen concentration ranges between 0,022 nM to 500 nM.

In a more preferred embodiment of the methods of the invention, alone or in combination with other preferred embodiments, when the method is referred to the hydrogel or the filter system of the invention, the pathogen concentration ranges between 5 to 250 nM (including the end values of the range). Even more preferably, the pathogen concentration is selected from the list consisting of 5, 10, 15, 20, 25, 30, 35, 40, 45, 50, 55, 60, 65, 70, 75, 80, 85, 90, 95, 100, 105, 110, 115, 120, 125, 130, 140, 145, 150, 155, 160, 165, 170, 175, 180, 185, 190, 195, 200, 205, 210, 215, 220, 225, 230, 235, 240, 245 and 250 nM.

In another more preferred embodiment of the methods of the invention, alone or in combination with other preferred embodiments, the pathogen concentration ranges between 0.11 to 200 nM (including the end values of the range).

The terms used to define the methods of the invention have been already described in previous aspects of the present invention and apply equally, as well as its preferred embodiments herein.

### DESCRIPTION OF THE DRAWINGS

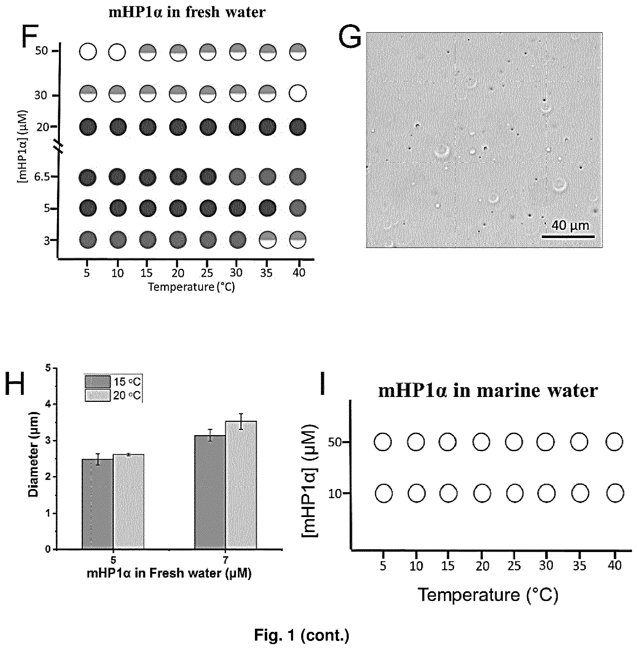

**Fig. 1****.** A) Schematic representation of pathogen trapping system. B-I Phase separation properties of candidate LCRs. B) and E) LLPS diagrams of DBP1 in fish farm conditions: marine and fresh water respectively. C). Representative micrograph of observed liquid droplets of 50 *µ*M DBP1 at 25°C in marine water. D). Average liquid droplets size at different concentrations and temperatures. F) and I). LLPS diagrams of mHP1α in fish farm conditions: fresh water and marine water respectively. G). Representative image of mHP1α phase separation obtained at 6.5 µM at RT in fresh water. H) Average liquid droplets diameter at different concentration and temperatures. Symbols: open cycles, no LLPS; half circles, poor LLPS; light gray circles, low intensity LLPS; dark gray circles, high intensity LLPS.

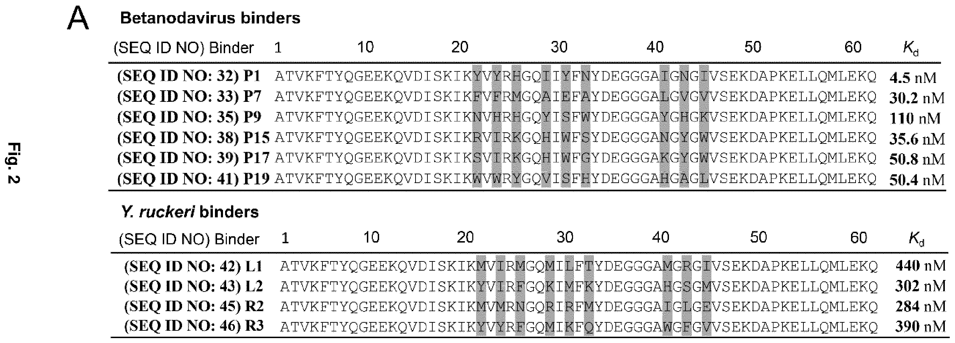

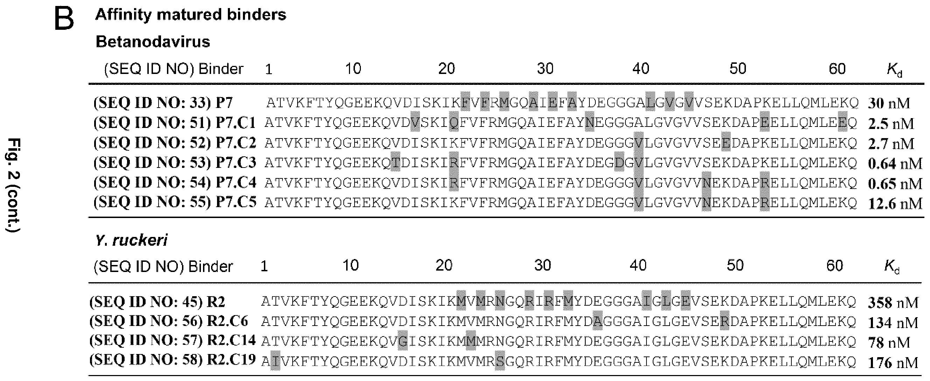

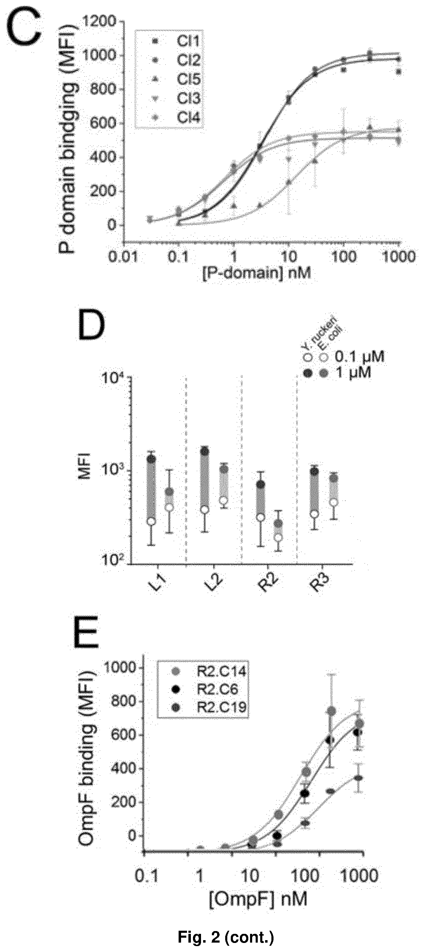

Fig. 2. Analysis of selected binders able to bind targeted antigens of the selected pathogens. A) sequences, sequence ID number and K_{d} values of the initial selected best clones for each pathogen obtained from the screening of the two libraries. The variability of residues at the nine randomly mutated positions is highlighted B) sequences, sequence ID number and K_{d} values of the matured clones for each pathogen obtained after affinity maturation process. The parental clone appears in the first line of each targeted pathogen. The variability of residues towards the parental one is highlighted. C) Individual titrations performed by flow cytometry of the matured clones against *betanodavirus.* D) Specificity assay performed by flow cytometry of the best *Y. ruckeri* binders against the OmpFs from *E. coli* and *Y. ruckeri* at two different concentrations. MFI, mean fluorescence intensity. E) Individual titrations of the matured clones against *Y. ruckeri* OmpF obtained by flow cytometry.

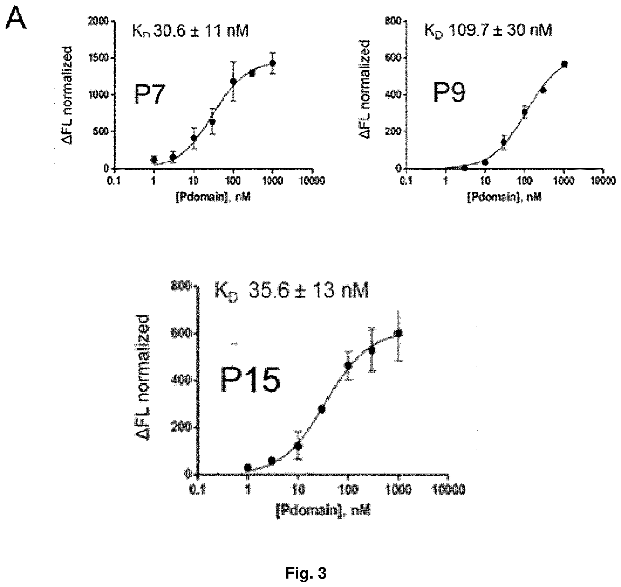

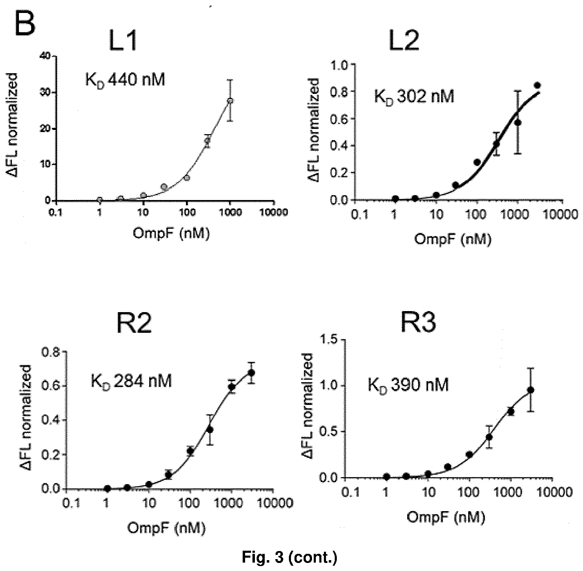

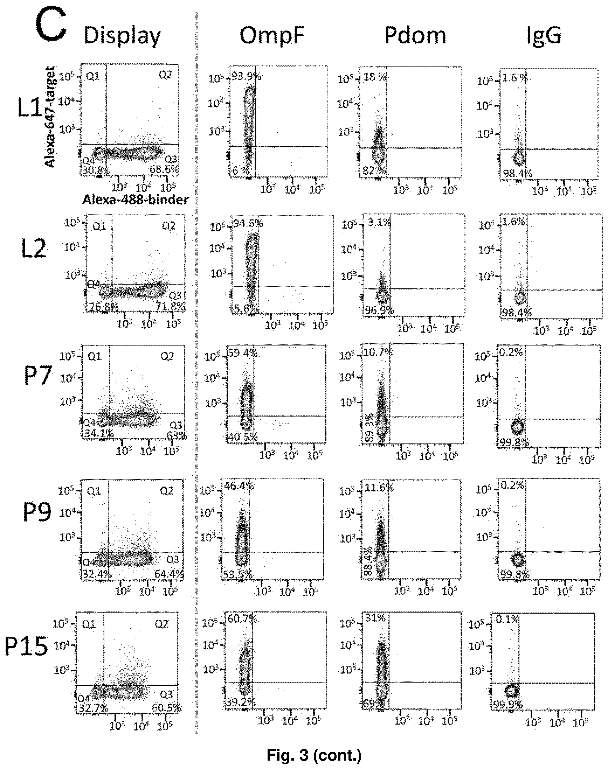

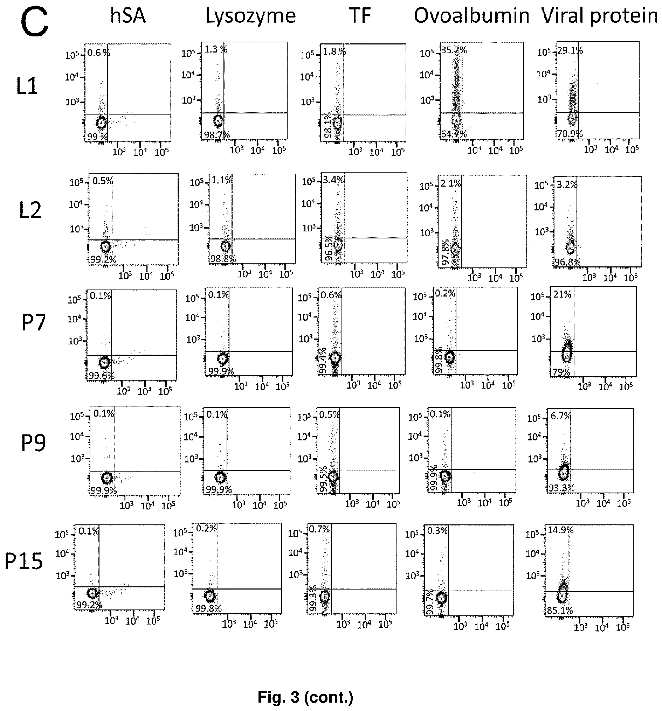

Fig. 3. Individual clone titrations of the best initial binders for each targeted antigens obtained by flow cytometry. A) binders to betanodavirus antigen, B) binders to Y. *ruckeri* antigen. C) cross-reactivity tests against non-related targets performed by flow cytometry. First column, display of the selected binder corroborated by high fluorescence intensity at 488 nm (X axis) while positive interaction is observed by high fluorescence intensity at 647 nm (Y axis). OmpF and P domain (Pdom) are the antigens for *Y. ruckeri* and betanodavirus pathogens respectively while L1 and L2 are the binders for *Y. ruckeri* and P7, P9 and P15 are the ones for betanodavirus. hSA (human seroalbumin), IgG (immunoglobulin G), TF (transferrin).

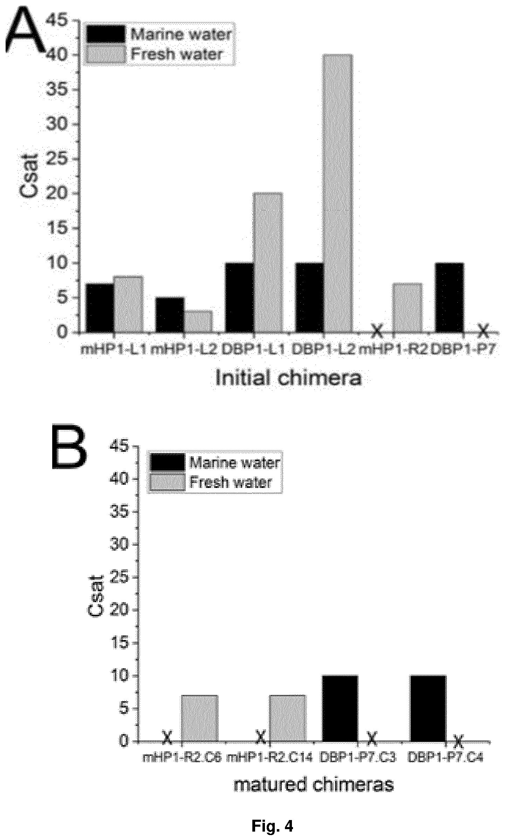

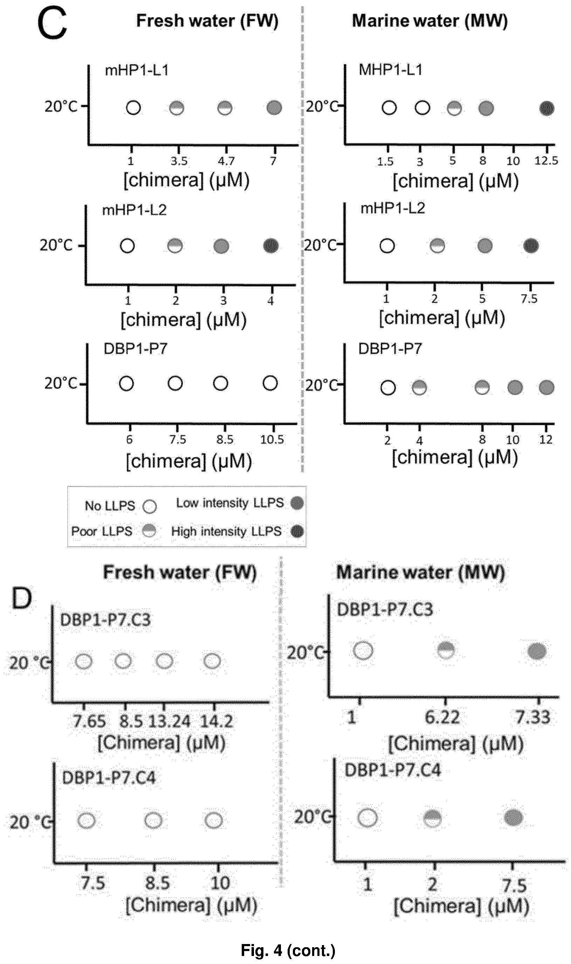

**Fig. 4****.** Phase separation properties of engineered chimeras in fish farm conditions.
   A) Minimum concentration able to undergo LLPS (Csat) of initial chimeras (contains non-matured binders) in fish farm conditions. B) Minimum concentration able to LLPS (Csat) of matured chimeras (contains affinity-matured binders) in fish farm conditions.
   C) LLPS diagrams of chimeras formed by the combination of the corresponding LCR (DBP1 or mHP1 α) and the initial binders selected for each pathogen (P7 for betanodavirus, L1 and L2 for *Y. ruckeri*). D) LLPS diagrams of betanodavirus matured chimeras.
**Fig. 5****.** Diagrams of the control chimeras (containing a binder able to recognize a non-pathogen related target, mouse serum albumin) in fish farm conditions at different temperatures and concentrations.

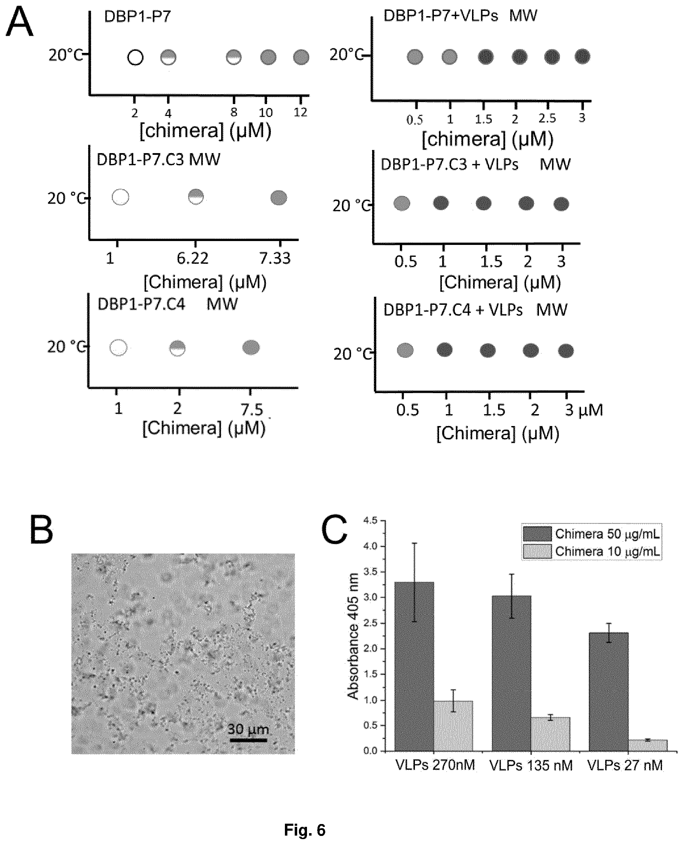

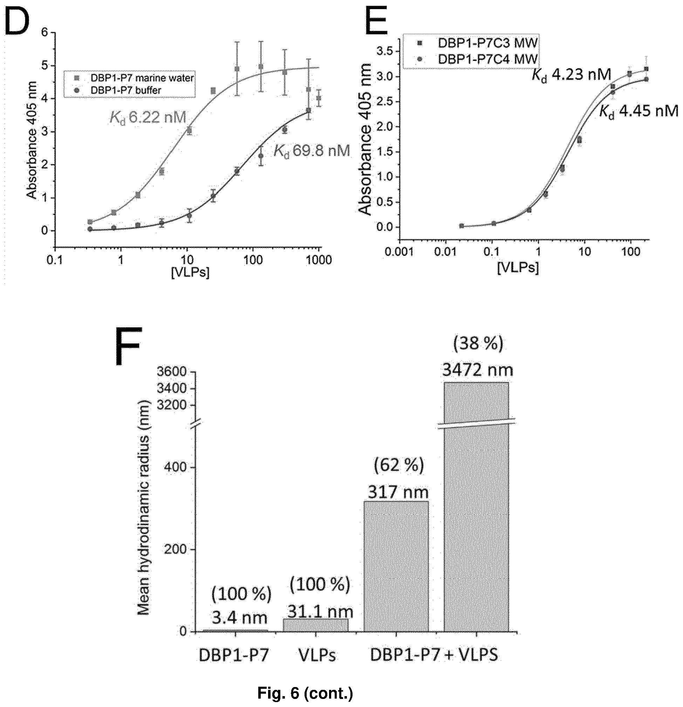

**Fig. 6****.** Interaction studies of chimeras formed by DBP1 LCR and different binders with virus-like particles (VLPs) from the selected betanodavirus. A) Phase separation diagram of DBP1-P7, DBP1-P7.C3 and DBP1-P7.C4 in marine water in absence and presence of VLPs. Symbols: open cycles, no LLPS; half circles, poor LLPS; light gray circles, low intensity LLPS; dark gray circles, high intensity LLPS. B) Representative image of DBP1-P7 phase separation in presence of VLPs obtained at 1.5 *µ*M at 20 ºC in marine water. C) Interaction of chimera-VLPs studied by ELISA at increasing concentrations of antigen. D) Titration experiments of DBP1-P7 chimera using increasing concentrations of VLPs (from 0.34 nM to 1,000 nM) to obtain K_{d} values performed in marine water and buffer PBS. E) Titration experiments of DBP1-P7.C3 and DBP1-P7.C4 chimera at increasing concentrations of VLPs (0.022 nM to 500 nM) performed in marine water. F) Hydrodynamic radius and percentage of mass calculated by dynamic light scattering of chimera DBP1-P7, betanodavirus VLPs and the complex involving the interaction of both.

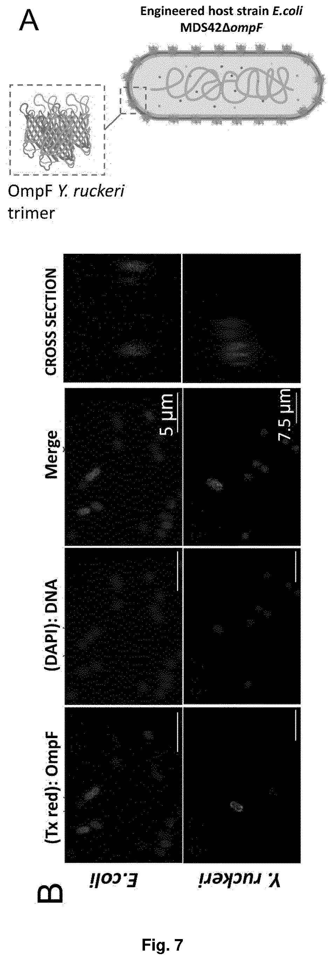

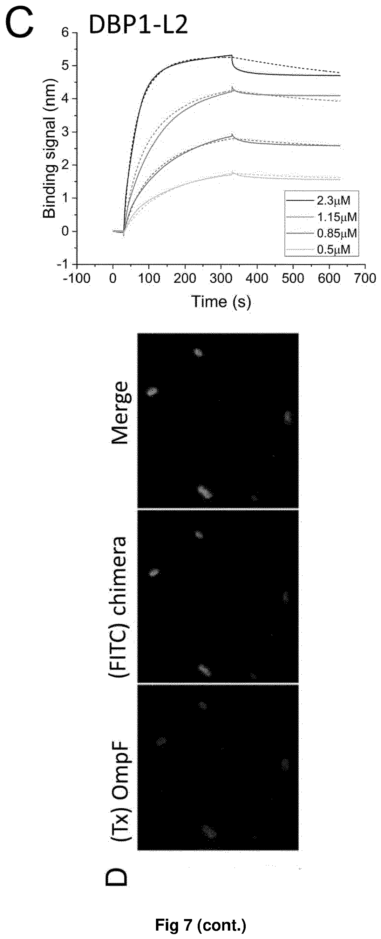

**Fig. 7****.** A) Schematic representation of the engineered *E.coli* strain expressing OmpF from *Y. ruckeri* integrated on the outer cell membrane. B) Immunofluorescence analysis of both OmpFs (*E.coli* and *Y. ruckeri*) expressed on the membrane of the engineered *E.coli* host strain. Three optical sections are showed (left) using different fluorophores and an optical cross-section (right). C) BLI measured data (solid lines) and curves from a global fit 1:1 binding model (dashed lines). Inset indicates the amount of rOmpF added. D) Fluorescence microscopy images of E. *coli* cells expressing OmpF *Y. ruckeri* embedded on the membrane revealed with anti-Histag and secondary Alexa Fluor-568 (Left) or incubated with biotinylated DBP1-L1 chimera and revealed with Streptavidin-Alexa Fluor-488 (Middle). Merge image of both fluorescence labelled cells (Right).

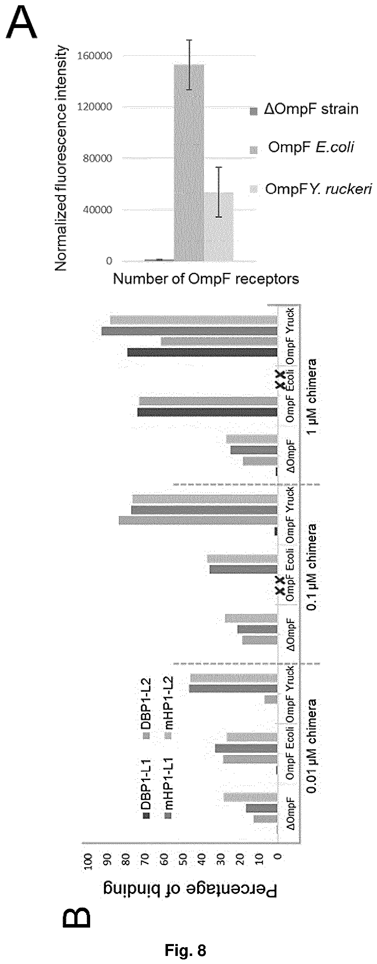

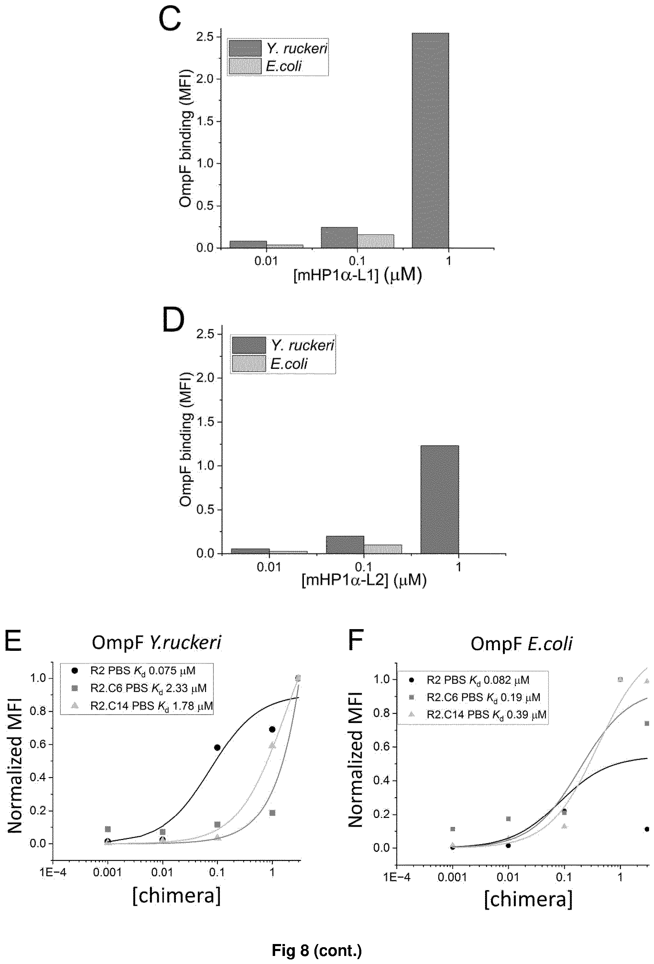

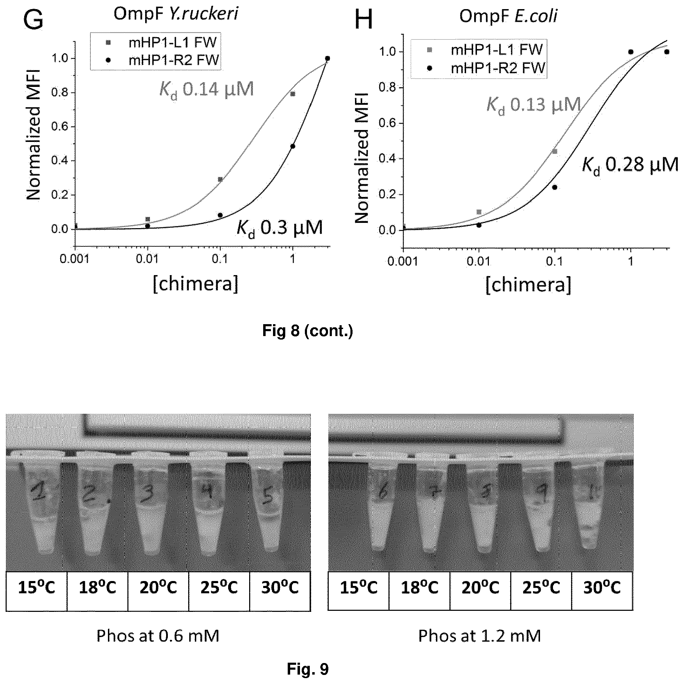

**Fig. 8****.** A) Quantification of the number of OmpF receptors expressed by the three strains: ΔompF, *Y.ruckeri* OmpF and wtOmpF. B) Histogram gathering the double positives (chimera-OmpF) for three different *E.coli* strains: ΔompF, wtOmpF and *Y. ruckeri* OmpF followed by flow cytometry. Four chimeras were analysed and three different concentrations: 0.01µM 0.1µM and 1µM. The cross icon represents not measured data. C-D) Chimera-target interaction by flow cytometry of chimeras mHP1α-L1 and mHP1α-L2 respectively for the two OmpF expressing strains (wtOmpF, OmpF from *Y. ruckeri*) at different concentrations. E) Titration experiments by flow cytometry of chimeras mHP1-R2, mHP1-R2.C6, mHP1-R2.C14 against OmpF *Y. ruckeri* expressing strain. The plot was build using 5 different concentrations of chimera in PBS and normalized for the number of OmpF receptors displayed by bacteria. F) Titration experiments in PBS of chimeras mHP1-R2, mHP1-R2.C6, mHP1-R2.C14 against OmpF *E.coli* expressing strain. G-H) Titration experiments of chimera obtaining affinity values in fresh water of mHP1-L1 and mHP1-R2 against OmpF *Y. ruckeri* expressing strain and OmpF *E.coli* expressing strain respectively.
**Fig. 9****.** HP1αPhos-canonical binder hydrogels at different temperatures and two concentrations.
**Fig. 10****.** SEM (Scanning Electron Microscope) images of HP1αPhos-canonical binder-like hydrogels at 500 µM. a, b, c, show the typical net-like structure of hydrogels.
**Fig. 11****.** ESEM (Environmental scanning electron microscope) micrograph of HP1αPhos-canonical binder-like hydrogels at 500 µM, showing the typical aspect of a wet hydrogel.
**Fig. 12****.** 3D surface metrology images of HP1αPhos-canonical binder-like hydrogels at 500 µM. A) Dry hydrogel. B) Swollen hydrogel after adding a drop of Milli-Q water. C. Swollen hydrogel 15 minutes after. D) Swollen hydrogel 30 minutes after. Note the profilometry shift.
**Fig. 13****.** Results on the rheological analysis of HP1αPhos-canonical binder-like hydrogels **A)** Time sweep **B)** Temperature sweep. **C)** Frequency sweep **D)** Viscosity.
**Fig. 14****.** The strain sweep behavior of HP1αPhos-canonical binder-like hydrogels at different concentrations shows a "solid-like" behavior for the bulk hydrogels formed.
**Fig. 15**. A) Different commercial (uncoated) kaldnes scaffolds made of v-HDPE. **B)** Experimental setups for surface kaldnes coating. **C)** Kaldnes surface coating with HP1αPhos chimera hydrogel **D.** Comparison between kaldnes coated (left) and uncoated (right) **E)** SEM micrograph of v-HDPE uncoated **F)** SEM micrograph of a HP1α film hydrogel coating the v-HDPE surface.
**Fig. 16****.** Boxplots of ELISA absorbance at 405 nm vs each VLPs concentrations before (white boxes) and after (gray boxes) kaldnes incubation.
**Fig. 17****.** Percentage of particle retention after incubation of the kaldnes in different concentrations of VLPs. Percentages were estimated from absorbance values at 405 nm.
**Fig. 18****.** Percentage of particle retention vs. relationship between chimera concentration (first number) and VLPs concentration (second number).

### Examples

### 1. Material and methods

### 1.1. Low-complexity regions (LCR) Bioinformatics screening

In order to search for low complexity regions capable to onset phase separation at the require fish farms conditions (15-25 °C) and pH 6.5-8 an extensively literature search was performed. To that end, LLPSDB database (http://bio-comp.org.cn/llpsdb/home.html) based on empirical phase separation results as well as other sources such as PubMed were searched. This search was refined using several algorithms such as SPOT-Disorder2, and PSPredictor. Furthermore, in order to get a simpler system and facilitate production in *Escherichia coli* (*E.coli*), the absence of disulfide bond formation in all candidates was checked using DISULFIND and DiANNA software. This process leads up to 6 candidate LCRs which were reduced to just 2 after testing their phase separation behavior in fish farm conditions: mHP1α and DBP1NtCt (DBP1, for short from now on).

### 1.2. Production of antigens

The sequence of the betanodavirus antigens (complete capsid protein and P-domain from red-spotted grouper nervous necrosis virus, RGNNV 283_2009 isolate) contained into pETite expression vectors were supplied by Dr. Anna Toffan (IZSVe) and transformed into *E. coli* BL21* (DE3) pLysS strain. Briefly, cells were grown at 37 ºC and 280 rpm in 1 L of LB medium with antibiotics until the OD₅₉₅ reached ∼ 0.6-0.8 and then induced with 1 mM isopropyl-*β*-D-thiogalactopyranoside (IPTG) and grown for 5 additional hours at 30 ºC and 150 rpm and at 37 ºC and 250 rpm for the P-domain and for the complete capsid protein, respectively. Then, cultures were harvested by centrifugation at 4 ºC, 6,260 *g* for 10 min. In the case of the P-domain, the cell pellet was resuspended in an equilibration buffer composed of 50 mM Na₂HPO₄ pH 7.4, 500 mM NaCl, 50 mM imidazole while the complete capsid protein pellet was resuspended in 10 mM Tris-HCl pH 7.6, 500 mM NaCl, 60 mM L-arginine, 5% glycerol and 0.05% Tween-20. To both equilibration buffers 5% glycerol, 1% Triton X-100, 0.5% Tween 20, 1 mM PMSF, 1:1000 protease inhibitor cocktail set III (Calbiochem) 1 mg/mL lysozyme, 5 µg/mL DNAse I, 5 µg/mL RNAse A were added and incubated for 1h at 4 ºC with gentle agitation and later, the sonicated lysates were centrifuged at 36,000 g for 1 h. In the case of P-domain supernatant was purified by Ni²⁺-affinity chromatography (FPLC system ÄKTA Pure, Cytiva) eluting the protein in 50 mM NaPO₄, 500 mM NaCl, 500 mM imidazole, 5% glycerol pH 7.4. The purified protein was dialyzed to PBS pH 7.4 containing 5% glycerol, concentrated using Amicon Ultra centrifugal devices (Millipore) and stored at -80 ºC. In the case of the complete capsid protein, a heparin affinity resin was used, eluting the protein in 10mM Tris-HCl pH 7.6, 1.7 M NaCl, 60 mM L-arginine, 5% glycerol and 0.05% tween 20. The purified protein was ultracentrifuged at 145,000 *g* at 4 ºC for 3h, then the pellet was resuspended in equilibration buffer and kept for 48 h at 4 ºC with gentle shaking to favor the spontaneous self-assembly into virus like particles (VLPs). The correct association into VLPs was tested directly by transmission electron microscopy while the high purity of antigens was checked by PAGE-SDS and Immunoblot using a specific antibody against betanodavirus RGNNV 283_2009 isolate 1:3000 (provided by Dr. Anna Toffan, IZSVe).

Regarding the *Y. ruckeri* antigens, the DNA sequence of the outer membrane protein (OmpF) was synthetized by ATG: biosynthetics GmbH while the OmpF sequence from *E.coli* (for negative selection) was obtained from pRK2 ompF(wild-type OmpF) vector, a kind gift from Dr. Rafael Giraldo (CNB-CSIC). Both sequences were cloned into the pET28 vector and transformed into the E. *coli* BL21* (DE3) strain that co-expresses chaperones (Takara Bio), obtaining fusion proteins containing a 6xHis tag and a TEV Niα protease cleavage site. Briefly, cells were grown in 1 L of LB medium with antibiotics at 37 ºC and 280 rpm until an OD₅₉₅ of ∼ 0.6-0.8 and then induced with 1 mM IPTG and grown o/n at 25 ºC and 250 rpm. Then, cultures were harvested at 4 ºC, 6,260 g for 10 min. Cell pellets were resuspended in a 50 mM NaPO4 pH 7.4 buffer containing 8M Urea, 500 mM NaCl, 50 mM imidazole 1% Triton X-100 and 0.5% Tween-20 and subjected to sonication pulses. Lysates were centrifuged at 36,000 g for 1 h. Supernatants were purified by Ni²⁺-affinity chromatography (FPLC system ÄKTA Purifier, GE Healthcare) eluting the protein in 50 mM NaPO4 pH 7.4 containing 6M urea, 500 mM NaCl and 500 mM imidazole. The purified protein was dialyzed in PBS pH 7.4 containing 5% glycerol, concentrated using Amicon Ultra centrifugal devices (Millipore) and stored at -80 ºC. The purity of antigens was checked by PAGE-SDS and Immunoblot using an anti-Histag antibody (Novagen) at 1:2000 dilution.

### Production of OmpF wild type and from Y. ruckeri embedded in the membrane of E. coli

The reduced genome E. *coli* strain MDS42 Δ*omp*F as well as the strain MDS42 containing the synthetic cassette pRK2-ompF(wt) previously used for wtOmpF membrane expression (Vendrell-Fernández, et al., Conversion of the OmpF Porin into a Device to Gather Amyloids on the E. coli Outer Membrane. ACS Synth. Biol. 11, 655-667 (2022)) were kindly donated by Prof. Rafael Giraldo (CNB-CSIC). For the production of *Y. ruckeri* porin F embedded in E. *coli* membrane a similar strategy was followed: a synthetic cassette pRK2-ompF (*Y. ruckeri*) was built by ATG:biosynthetics GmbH and cloned into MDS42 Δ*omp*F strain. Expression of the OmpF variants was carried out from overnight inocula of the strains in LB medium, supplemented with 100 µg·mL⁻¹ ampicillin, at 30 °C. At OD₅₉₅ ∼ 0.4-0.5, 0.1 mM IPTG was added to and grown for up to 4 h. Cells were harvested, and the total protein content was analyzed by immunoblot against an anti-Histag antibody,1:2000 (Novagen) as well as by immunofluorescence. Briefly, the culture supernatant was then carefully removed, and the pellet rinsed twice with 200 µL of PBS, and finally fixed, for 30 min at room temperature (RT), with 100 µL of 2% paraformaldehyde (PFA) in PBS. After a blocking step, 1:200 anti-Histag antibody (Invitrogen) and 0.5 mg/mL DAPI were added to the suspension. Finally, 10 µL drops of cell suspension were spread on glass slides, mounted with Vectashield^{®} and observed by a Leica SP-5 inverted confocal microscope.

### 1.3. Binder screening

Naïve yeast display libraries expressing binders were provided by Dr. Alessandro Angelini (Ca´Foscari University of Venice) as a service subcontract from their facilities. Yeast display experiments were conducted as described elsewhere (Traxlmayr MW, Kiefer JD, Srinivas RR, Lobner E, Tisdale AW, Mehta NK, Yang NJ, Tidor B, Wittrup KD. Strong Enrichment of Aromatic Residues in Binding Sites from a Charge-neutralized Hyperthermostable Sso7d Scaffold Library. J Biol Chem. 2016 Oct 21 ;291(43):22496-22508. Doi: 10.1074/jbc.M116.741314). Briefly, cultures were grown to stationary phase in SD-CAA medium overnight followed by dilution in SD-CAA to an OD₅₉₅ of 1 at 30 °C. When culture reached an OD₅₉₆ of 3 cells were centrifuged and yeast surface expression was induced in SG-CAA overnight at 20 °C. For selections, artificial marine or fresh water (supplied by the Istituto Zooprofilattico Sperimentale delle Venezie, IZSVe) + 0.1% BSA was used for the betanodavirus and *Y. ruckeri* antigens, respectively. Antigens were biotinylated using EZ-link Sulfo-NHS-LC-Biotin (Thermo) following the instructions from the manufacturer.

Bead selections were performed exposing S. *cerevisiae* libraries to non-loaded streptavidin-coated magnetic beads followed by a screening against beads immobilized antigens (P-domain and OmpF). Two beads selection campaigns were performed and in each one two negative and one positive selection were conducted. The selected yeast cells isolated after magnetic bead-based processes were analyzed on an Attune^{™} NxT Flow Cytometer (Invitrogen) and further screened using fluorescence-activated cell sorting (FACS) on a FACSAria III. For these experiments, washed cells were incubated with biotinylated antigen and mouse anti-c-myc (clone 9E10, Covance) followed by incubation with Neutravidin-Dylight 650 and goat anti-mouse Alexa Fluor-488. Several rounds of cell sorting were performed to enrich the population with better binding capacities, characterizing individual clones by yeast surface titrations using flow cytometry. Cross-reactivity tests of the best selected binders against 1 µM of non-related targets (IgG, human seroalbumin, lysozyme, transferrin, ovoalbumin and a viral protein) were performed using OmpF and P domain antigens as positive controls. Best single binders for each antigen were subjected to an affinity maturation process creating new variability of binders based on error-prone PCR (epPCR). During this process, the recombinant OmpF from *E. coli was* used for the negative selection to increase selectivity for the *Y. ruckeri* antigen. Several rounds of cell sorting were conducted to enrich for target binders. Single clone binding capacities of affinity-maturated binders were studied by flow cytometry as previously described.

### 1.4. Cloning, production and purification of LCRs and chimeras

DNA sequences of the low complexity regions of DEAD-Box helicase 1 (DBP1) from *Saccharomyces cerevisiae* following the Faltova and col. (Faltova, L., et al. Multifunctional protein materials and microreactors using low complexity domains as molecular adhesives. ACS Nano 12, 9991-9999 (2018)) strategy (fusing the first N-terminal 155 residues together with the last 67 C-terminal residues; SEQ ID NO: 1) and Heterochromatin Protein 1α from mouse (191 residues; SEQ ID NO: 2), isoform α (mHP1α) were synthetized by ATG biosynthetics and cloned into the pET28 vector. For the chimera constructs, the corresponding binder sequence was amplified by PCR, attached to the C-terminus of the LCR and inserted into the above-mentioned vector. Recombinant proteins were overexpressed in *E. coli* BL21 Star (DE3) strain as fusion proteins containing a 6xHis tag and a TEV Niα protease cleavage site. For DBP1 and chimeras containing this LCR, cells were grown in 1 L of LB medium at 37 ºC and 280 rpm until OD₅₉₅ of ∼ 0.6-0.8, being then induced with 1 mM IPTG and grown for 4 hours at 37 ºC. Cells pellet was resuspended in 50mM NaPO₄ pH 7.4 containing 500 mM NaCl, 50 mM Imidazole, 6 M GndCl, 1% Triton X-100 and 0.5% Tween-20 and incubate for 30 minutes at 4ºC with gentle shaking and sonicated. The lysates were centrifuged at 36,000g for 50 min and supernatants were purified by Ni²⁺affinity chromatography (FPLC system ÄKTA Pure, GE ÄKTA Pure, Cytiva), eluting DBP1 and chimera in 50mM NaPO₄ pH 7.4, 150mM NaCl, 500mM Imidazole, 0.5M GndCl. Since some additives were added to the DBP1 chimeras to avoid degradation, the elution buffer in this case contains 50 mM L-arginine and 50 mM L-glutamic acid.

For mHP1α and chimeras containing this LCR, cells were grown in 1 L of LB medium at 37 ºC and 280 rpm until OD₅₉₅ of ∼ 0.6-0.8, and then induced with 0.5 mM IPTG and grown o/n at 16 ºC. Cells pellet was resuspended in PBS pH 8, 10 mM Imidazole, 1% Triton X-100, 1 :1000 protease inhibitor cocktail Set III (Calbiochem) and incubate for 30 minutes at 4ºC with gentle shaking and sonicated. The lysates were centrifuged at 36,000g for 50 min and supernatants were purified by Ni²⁺-affinity chromatography. mHP1α and chimeras containing this LCR were eluted in PBS pH 8 buffer containing 250mM Imidazole and dialyzed to PBS pH 8 using a PD10 desalting columns (Cytiva). Finally, LCRs and chimeras were stored at -80 ºC until used.

### 1.5. Phase separation analysis by optical and fluorescence microscopy

Phase separation of chimeras (both alone and in the presence of the pathogens) was studied by bright field and fluorescence microscopy. Images were acquired using a Leizt DM IRB microscope with 40x/0.80 objective equipped with a temperature controller Peltier (Linkam) and a K5 camera (Leica). Images were analyzed with Leica LAS AF Lite software. Chimeras control (containing a non-specific binder) were produced to check the phase separation behavior of the LCR-binder construct. Chimeras were pre-incubated at several concentrations and buffer conditions: conservation buffer, marine or fresh water (supplied by SmartwaterPlanet SL.) at 10-20 °C in a thermoblock (Eppendorf) for 10 min.

To test the phase separation of the chimeras alone, samples were pre-incubated at different concentrations, temperatures and buffer conditions (conservation buffer, marine or fresh water supplied by SmartwaterPlanet SL) using a thermoblock without stirring (Eppendorf) for 10 min at 20 ºC. To observe interaction and phase separation behavior upon pathogen binding, chimeras DBP1-P7 (SEQ ID NO: 59), DBP1-P7.C3 (SEQ ID NO: 60) and DBP1-P7.C4 (SEQ ID NO: 61) were incubated 10 min at 20 ºC with equimolar concentration of virus like particles (VLPs) of betanodavirus. Then, samples were observed under the optical microscope using glass bottom dishes (Ibidi).

To observe the chimera-pathogen interaction and phase separation behavior upon pathogen binding, chimeras mHP1α-L1 (SEQ ID NO: 62), mHP1α-L2 (SEQ ID NO: 63), DBP1-L1 (SEQ ID NO: 101) and DBP1-L2 (SEQ ID NO: 102) labelled with streptavidin-488 in the case of mHP1α or Alexa Fluor-488 for DBP1 chimeras, were incubated for 10 min at 20 ºC with DAPI-labelled *E.coli* cells expressing *Y.ruckeri* OmpF embedded on the membrane. Then, samples were observed under microscope using glass bottom dishes (Ibidi). Phase separation of the chimeras alone was monitored in bright filed microscopy. Binding and behavior upon OmpF binding was studied by fluorescence microscopy.

### 1.6. Enzyme-Linked ImmunoSorbent Assay (ELISA) to evaluate chimera-VLPs binding

DBP1-P7, DBP1-P7.C3 and DBP1-P7.C4 chimeras were loaded into the wells (10-50 µg/mL) in a volume of 50 µL on a 96-well ELISA plate (Falcon) and left to coat overnight at 4 °C. The wells were washed four times with washing buffer (PBST 1% BSA) and blocked by 5% BSA PBST in the volume of 200 µL/well for 1.30h at RT. After four washes, different concentrations of VLPs (0.022 - 1000 nM) were added to the wells for 3h at RT. Wells were washed four times, and then primary antibody (anti-VLPs, diluted 1:100,000) was added in the volume of 50 µL/well and incubated o/n at 4°C. Wells were washed four times, and then the secondary antibody (goat anti-rabbit with horseradish peroxidase, diluted 1:2000) was added in the volume of 50 µL/well and incubated for 1 h at RT. Wells were again washed four-five times and ABTS substrate was added (100 µL/well) and the absorbance (405 nm) was measured by a plate reader (Multiscan Ascent).

### 1.7. Dinamic light scattering (DLS)

DLS was used to determine the hydrodynamic radius of the VLPs and chimera individually and then together to test their interaction as an increase in the hydrodynamic radius. DLS analysis was performed using a sample volume of 50 µl at 100-500 µg/mL in a 3x3 mm quartz cuvette (Hellma Analytics, Müllheim, DE) using a DynaPro MS/X (Wyatt Inc). Laser intensity was adjusted between 30 and 80% depending on the sample and 40 acquisitions per sample were recorded.

### 1.8. Biolayer interferometry (BLI)

Chimera-antigen interaction was analyzed on a BLIZT instrument (ForteBio) using PBS pH 7.4. For *Y. ruckeri* antigens, biotinylated chimeras were immobilized onto streptavidin coated BLI tips (ForteBio) at 100 µg/mL. Later, association to OmpF was analyzed at various concentrations (from 0.5-2.3 µM) followed by measuring dissociation in buffer. Baselines (chimera-loaded tips without addition of antigen) were subtracted from the data followed by global fitting to a 1:1 binding model. K_{d} values were obtained from steady state binding analysis.

### 1.9. Flow cytometry to evaluate binding chimera-OmpF embedded in the membrane

Three *E.coli* strains were used for these experiments: *E.coli* strain MDS42 Δ*omp*F (negative control), *E.coli* strain MDS42 containing plasmid pRK2-ompF *(wt)* for membrane expression of wtOmpF and *E.coli* strain MDS42 containing plasmid pRK2-*omp*F (*Y. ruckeri*) for expression of OmpF embedded in the membrane. For all variants, cells were grown in LB medium supplemented with 100 µg·mL⁻¹ ampicillin at 30 °C until the culture reached an OD₅₉₅ of ~ 0.4-0.5, then 0.1 mM IPTG was added to the cultures and grown for 3 h at the same temperature. Later, culture OD₅₉₅was adjusted at 0.2 and cells were fixed in 1% PFA solution in PT100 buffer (PBS pH 7.4 and 0.5% Triton X-100) for 30 minutes at room temperature and stored for up to a week at 4ºC in the same buffer. In order to detect the His tag of the membrane-expressed OmpF, bacteria were treated with 0.01 µg/mL lysozyme in GTE solution (50 mM EDTA, 20 mM Tris-HCl and 50 mM glucose) for 7 minutes at room temperature and then washed in PBS pH 7.4 to stop the reaction. For labelling samples, they were blocked with a PBS 1x pH 7.4, 0.05% Tween-20 and 1% BSA solution at RT for 30 minutes and then incubated overnight at 4ºC with mouse-αHis antibody (Novagen) at 1.5 µg/mL. Next day cells were incubated with biotinylated antigen at different concentrations (from 0.001 µM to 1 µM or up to 3 µM for titrations) for 20 minutes at RT, followed by an incubation of 30 min with both streptavidin labelled with Alexa fluor-488 and anti-mouse Alexa Fluor-568 or Alexa Fluor-647. Samples were analyzed by Cytoflex-S Beckman coulter. Negative controls were used to control auto-fluorescence of cells, and positive controls were employed to monitor and control the crossing of fluorescence between labelled proteins. CytExpert software (Beckamn-Couter) was used to analyze the data. Qualitative binding of the chimera was determined by the number of double positives. To obtain the quantitative data, the normalized mean fluorescence intensity based on the number of OmpF receptors of each *E.coli* strain was calculated.

### 1.10. Cytotoxicity assays

The cell viability assay was performed in 96-well tissue plates containing 2x10⁴ human neuroblastoma SH-SY5Y cells per well in DMEM/F12 1:1 GlutaMAX medium supplemented with 10% FBS and 1% penicillin/streptomycin maintained at 37 ºC for 24 hours prior to the addition of chimera samples. Cell cultures were incubated with different concentration of the chimeras for 24h or 48h. Then, cell plates were tempered to room temperature for 30 minutes and viability was determined adding 100 µL of CellTiter-Glo^{®} reagent (Promega) to the same volume of medium-containing cells. Content was mixed for 2 minutes on an orbital shaker to induce cell lysis and then maintained at RT for 10 minutes to stabilize the signal. Peak emission of luminescence at 560 nm was recorded using a FLUOstar OPTIMA microplate reader. The average of readings of three replicate wells was taken as one-point value while the control cultures values (without chimera) were considered as 100% viability.

### 1.11. Cloning, production and purification of HP1αPhos chimeras

### Prediction of phosphorylation sites.

The mHP1α phosphorylation sites were predicted using the ELM (Eukaryotic Liner Motif) functional site prediction program according with Hiragami-Hamada *et al.* (Hiragami-Hamada, K. et al. N-Terminal Phosphorylation of Promotes Its Chromatin Binding. Molecular and Cellular Biology 31, 1186-1200 (2011)).

### Plasmid construction

The sequence and mutations of phosphorylated human (HP1αPhos) were taken from Larson (Larson, A. G. et al. Phase Separation in Heterochromatin (Liquid droplet formation by suggests a role for phase separation in heterochromatin). Nature Publishing Group 547, 236-240 (2017)). Sequence of casein kinase II was taken from Uniprot (UniProtKB) with codes for Casein kinase II subunit alpha P68400.1 CSK21_HUMAN and Casein kinase subunit beta A0A0G2JM12 A0A0G2JM12_HUMAN.

Gene synthesis were made for GenScript. The mHP1α was subcloned in pET16b vector between Ncol and Xhol to eliminate the 10xHis label and the Factor Xa site from the original pET16b sequence and obtain our 6xHis label and the TEV site. For subsequent subcloning assays the BamHl site from the original vector sequence was eliminated and to that end position 5,391 was mutated from T to C. Casein kinase II (CKII) subunits (subunit α and subunit β) were subcloned in a pRSFDuet-1 vector (Merck) on each open reading frame (ORF). Thus, subunit α was subcloned between BamHl and Hindlll on the first multiple cloning site (MCS1) and the subunit β was subcloned from Bglll to Kpnl on the MCS2.

The sequence used as negative control is the following one:
HP1αPhos-canonical binder chimera

MGKKTKRTADSSSSEDEEEYVVEKVLDRRVVKGQVEYLLKWKGFSEEHNTWEPE
KNLDCPELISEFMKKYKKMKEGENNKPREKSESNKRKANFANAADDIKSKKKREQ
SNDIARGFERGLEPEKIIGATDSCGDLMFLMKWKDTDEADLVLAKEANVKCPQIVIA
FYEERLTWHAYPEDAENKEKETAKSGSATVKFTYQGEEKQVDISKIKWVIRWGQH
IAFKYDEGGGAAGYGWVSEKDAPKELLQMLEKQ (SEQ ID NO: 103)

Code: bold italics for serines *in vivo* phosphorylated, bold and dotted underlined for alanines substituting serines with respect to the wild type protein to prevent their phosphorylation (Larson, A. G. et al. Phase Separation in Heterochromatin (Liquid droplet formation by suggests a role for phase separation in heterochromatin). Nature Publishing Group 547, 236-240 (2017)), and bold for the canonical binder with reduced charge (rc) rcSso7d-M11.1 taken from Traxlmayr, M. W. *et al.* (Traxlmayr MW, Kiefer JD, Srinivas RR, Lobner E, Tisdale AW, Mehta NK, Yang NJ, Tidor B, Wittrup KD. Strong Enrichment of Aromatic Residues in Binding Sites from a Charge-neutralized Hyperthermostable Sso7d Scaffold Library. J Biol Chem. 2016. 291:22496-22508. Doi: 10.1074/jbc.M116.741314)) since a yeast surface titration process of the mouse serum albumin (MSA) binders revealed affinities in the nanomolar range. The affinity of M11.1 was of 226 nM.

The sequence of the Phosphorylated-HP1α-P7-binder chimera is the following one:
**Phosphorylated-HP1α-P7-binder chimera** (HP1αPhos-P7)

GKKTKRTADSSSSEDEEEYVVEKVLDRRVVKGQVEYLLKWGFSEEHNTWEPEK
NLDCPELISEFMKKYKKMKEGENNKPREKSESNKRKANFANAADDIKSKKKREQS
NDIARGFERGLEPEKIIGATDSCGDLMFLMKWKDTDEADLVLAKEANVKCPQIVIAF
YEERLTWHAYPEDAENKEKETAKSGSATVKFTYQGEEKQVDISKIKFVFRMGQAI
EFAYDEGGGALGVGVVSEKDAPKELLQMLEKQ (SEQ ID NO: 67)

Key: bold Italics for serines *in vivo* phosphorylated, bold and dotted underlined for alanines that replace serines to prevent their phosphorylation, and double underlining for the semi-matured P7 binder against the betanodavirus.

### Recombinant protein production.

For *in vivo* CKII phosphorylation, a simultaneously transformed (co-transformation) of pET16b-HP1α and pRSFDuet-CKII_α&β_subunits vectors were made using ampicillin resistance for pET16b and kanamycin resistance for pRSFDuet.
CKII Alpha subunit (SEQ ID NO: 94)
CKII Beta subunit (Seq ID NO: 95)

MSSSEEVSWISWFCGLRGNEFFCEVDEDYIQDKFNLTGLNEQVPHYRQALDMILD
LEPDEELEDNPNQSDLIEQAAEMLYGLIHARYILTNRGIAQMLEKYQQGDFGYCPR
VYCENQPMLPIGLSDIPGEAMVKLYCPKCMDVYTPKSSRHHHTDGAYFGTGFPHM
LFMVHPEYRPKRPANQFVPRLYGFKIHPMAYQLQLQAASNFKSPVKTIR

Recombinant His-tagged-HP1αPhos was expressed in *E. coli* BL21 derivate Rosetta (DE3) and purified by tetradentate chelating agarose resin charged with divalent cobalt (Co²⁺) through gravity-flow column according to the instructions of the manufacturer (Thermofisher).

### In vitro phosphorylation

For the *in vitro* kinase reaction, 5 µg of the recombinant protein was treated with 100 U of recombinant CK2 (New England BioLabs) in CK2 buffer (20 mM Tris-HCl [pH 7.5], 50 mM KCl, 10 mM MgCl2, 200 mM ATP) for 3 h at 30°C.

### 1.12. Hydrogel formation

Temperature assays were performed in an Applied Biosystems Veriti Pro Thermal Cycler with VeriFlex temperature control technology to run up to 5 different temperatures in same protocol step.

### 1.13. Optical characterization

The macroscopic characterization of hydrogels was performed with the naked eye and using an inverted optical microscopy on a LEITZ DMIRB Inverted Leica Modulation Contrast Microscope. The roughness of the samples was characterized on 3D Surface Metrology Microscope DCM8 equipped with a High Definition confocal microscopy with interferometry.

### 1.14. SEM characterization

Ultra-high vacuum SEM characterization was performed on a Field Emission Scanning Electron Microscope (FESEM) Teneo of Thermo Fisher Scientific (FEI). Environmental SEM characterization (ESEM) was performed with a FEI ESEM Quanta 200 in Environment Scanning Electron Microscope (ESEM) mode, indicated for wet samples to characterize the dry-swelling process.

### 1.15. Rheological characterization

A rheometer with an oscillatory technique in a parallel plate geometry for dynamic mechanical measurements was used. The sample conditions used were 1 mL of each sample on liquid state to form a hydrogel of 1 mm of thickness. Temperature, time, strain and frequency sweeps were performed.

### 2. Results

### 2.1. Liquid-Liquid phase separation (LLPS) of selected LCRs in fish farm conditions

For the design of the chimeras we first set up the minimum required framework parameters on which the chimeras should work. To that end, in addition to consider the parameters to which the selected fish are farmed, we also considered the fish farms conditions at which the infection outbreaks usually occur for the two selected pathogen/fish couples. Accordingly, we set up the working temperature range at 15-25 °C and the pH at 6.5-8. The first element of the chimera is the low complexity region (LCR), which should be able to phase separate in these fish farm conditions and with the widest possible temperature range.

Candidate LCRs were produced and their LLPS properties tested in different buffers (with or without the addition of salt) mimicking the natural fish farm environments. This analysis rendered two main candidates: DEAD-box protein 1 from *Saccharomices cerevisiae* (DBP1; SEQ ID NO: 1) and Heterochromatin Protein 1 from *Mus musculus* (mHP1α; SEQ ID NO: 2). Later, those pre-selected candidate LCRs were further tested in fresh water and marine water to characterize their phase transitions capabilities. DBP1 was able to undergo LLPS in marine water from 10 µM, which represents the critical protein concentration threshold for phase separation (Cₛₐₜ). The temperature range for LLPS was observed between 5-25 °C at 10 µM becaming wider with increasing chimera concentrations (5-40°C) **(**Fig. 1B-C) and fusion events between droplets became more frequent. Thus, the diameter of the droplets raises 2-fold from 10 µM to 50 µM, achieving droplets from 2 µm to ones of ∼ 4 µm **(****Fig. 1D****).** On the other hand, no phase separation was observed when DBP1 was dialyzed in fresh water at any concentration (50-1330 µM) or temperatures tested **(****Fig. 1E****).** Therefore, the presence of salt seems to be critical for DBP1 phase separation.

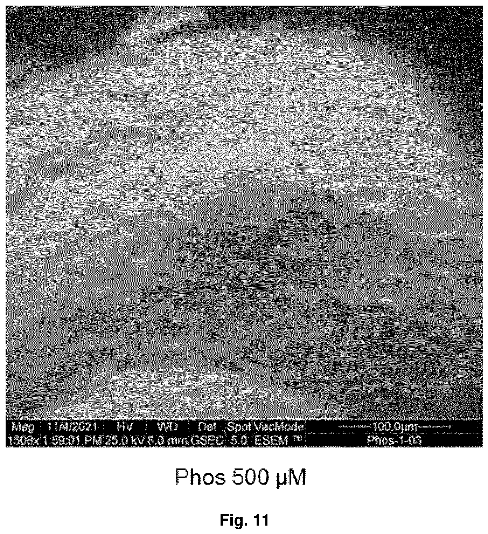

Conversely, mHP1α was able to LLPS in fresh water at lower concentrations showing a critical protein concentration for phase separation (Cₛₐₜ) of 3 µM, with phase separation observed between 5 °C to 35°C from 5 µM up to 20 µM **(**Fig. 1F-G). The size of the droplets varied from 2.5 µm to 3.5 µm with a 1.4-fold protein increment **(****Fig. 1H****).** On the other hand, mHP1α solubilized in marine water was not able to phase separate at any concentration tested **(****Fig. 11****).** Contrary to the situation for DBP1 this LCR required very low salt concentration to show LLPS although its Cₛₐₜ is much lower. In conclusion, in their corresponding working conditions both candidate LCRs showed good features such as low critical concentration and a wide temperature range at which they undergo phase separation.

### 2.2. Selection of binders against betanodavirus RGNNV and Y. ruckeri antigens

The second element of the chimera is the pathogen recognition moiety. To develop it we screened the yeast-displayed libraries rcSso7d-11 and rcSso7d-18 containing 1.4 x 10⁹ binders each, which were mixed to increase diversity. The corresponding antigens for selected pathogens, P-domain region of betanodavirus RGNNV and the *Y. ruckeri* porin OmpF, were produced in *E. coli.* The screenings were performed in artificial marine water and fresh water, respectively. Binder screening began with two rounds of negative selection against unloaded streptavidin beads to decrease the frequency of nonspecific binders. Later, rounds of enrichment for binding to specific antigen-loaded magnetic beads were performed. For each antigen, two beads selection campaigns were performed and in each one, two negative and one positive selection were conducted. Then, the magnetic bead-enriched libraries were sorted by FACS several times in order to increase the number of clones with high avidity. Yeast surface titrations of individual clones of both antigens revealed that the best affinities were in the low nanomolar range (30-100 nM) for the betanodavirus and in the medium nanomolar range (300-500 nM) for *Y. ruckeri* (Fig. **2A** and Fig. 3A-B). In order to test the specificity of the best initial binders for each target, cross-reactivity assays against a variety of non-targeted proteins were also performed. Results showed low nonspecific binding for betanodavirus binders, although some binding to OmpF was found for clone P15 **(****Fig. 3C****).** In the case of selected binders for *Y. ruckeri* OmpF, only clone L1 showed slight cross-reactivity against ovalbumin and a glycoprotein from a pathogenic virus.

Next, three betanodavirus binders from different sequence families (P7, P9 and P15) were subjected to affinity maturation process by error-prone PCR and subsequent FACS sorts. The best maturated-binders revealed affinities in the very low nanomolar range (0.64-2.7 nM) increasing 10-fold to 46-fold the parental affinity **(****Fig. 2B-2C****).** Regarding *Y. ruckeri* binders, some no specificity against the embedded OmpF from *E. coli* (which shares 69 % sequence similarity with *Y. ruckeri* porin) was found in the first-round selected binders (L1 and L2) **(****Fig. 2D****).** Therefore, we further analyzed the specificity of the best individual clones towards both recombinant OmpFs, finding that R2 clone showed the lower non-specificity **(****Fig. 2D****)** and thus it was subjected to the affinity maturation process. During the affinity maturation process, recombinant OmpF from *E. coli was* used for the negative selection to increase selectivity for the *Y. ruckeri* antigen. The best maturated-binders revealed affinities in the nanomolar range (78-176 nM) increasing 2-fold to 4.5-fold the parental affinity **(****Fig. 2B-2E****).**

### 2.3. LLPS of the engineered chimeras in the fish farm conditions

Once we selected the two chimera components, the approach was building chimeras containing the selected LCRs and several initial binders (non-matured) for each pathogen to check that the fusion proteins maintain their phase separation capabilities. Since, affinities were considerably better for the betanodavirus antigen than for *Y. ruckeri,* we produced one chimera for betanodavirus (DBP1-P7) and five for the bacterium (mHP1α-L1, mHP1α-L2, mHP1 α-R2, DBP1-L1, DBP1-L2) using the selected LCRs. After affinity maturation process we constructed new chimeras with the best-performance matured binders: DBP1-P7.C3, DBP1-P7.C4 for betanodavirus and mHP1α-R2.C6 and mHP1α-R2.C14 for the bacterium antigen. The corresponding chimeric protein sequences are included in Table 3, shown above.

In order to study the LLPS properties of these chimeras under natural fish farm conditions and to get the concentration value above which the system starts to phase separate (Cₛₐₜ), different concentrations were assayed. The Cₛₐₜ values obtained **(**Fig. 4A-B) indicate that the chimeras displayed different phase transition properties than their corresponding LCRs alone. Indeed, chimeras containing L1 and L2 binders were able to phase separate in both marine and fresh water at lower concentration, suggesting that the specific binder sequence attached to the LCR modulates LLPS. DBP1-containing chimeras were considerably better for marine water conditions because Cₛₐₜ values are lower, and therefore it was discarded for it use to block the freshwater pathogen *Y. ruckeri.*

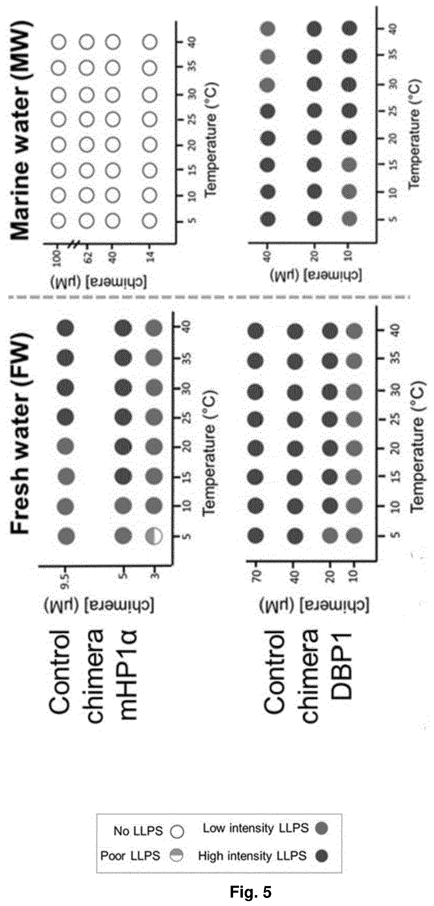

Based on our previous results with the control chimeras **(****Fig. 5****)** we chose to perform the phase diagrams at 20 °C **(**Fig. 4C-D), since we did not observe many differences on their LLPS behavior in the 15-25 °C temperature range. These results showed the versatility of mHP1α chimeras containing L1 and L2 binders, which were able to phase separate in both water environments at low concentration and no substantial differences. In the case of the chimeras able to recognize betanodavirus pathogen, the phase separation was observed only in marine water.

### 2.4. The selected betanodavirus chimera binds virus-like particles (VLPs)

Once we demonstrated that the selected chimeras underwent LLPS in fish farm conditions, we analyzed its LLPS behavior in presence of the antigen. Chimeras at increasing concentrations (0.5 to 4 µM) were incubated with VLPs in equimolar concentrations at 20 °C in marine water. When VLPs were present we observed a drastic reduction of more than 10-fold (from 10 µM to 0.5 µM) in the Cₛₐₜ of the chimera **(****Fig. 6A****).** Thus, massive LLPS was triggered at 1.5 µM, which strongly suggests the existence of a local concentration effect prompted by the density of antigens on the surface of the pathogen. Formed liquid droplets that behaved a little different from the ones formed in absence of the pathogen. Instead of coalescing into progressively bigger droplets, they formed aggregated structures reminiscent of bunches of grapes **(****Fig. 6B****).**

In order to assess the binding affinity and dynamics of the chimeras to VLPs, we performed a series of ELISAs. First, we concentrated on finding the best conditions to standardize the assay by testing different concentrations of immobilized protein obtaining, higher interaction at increasing concentrations of the target **(****Fig. 6C****).** Later, titrations experiments were performed both in PBS and in marine water obtaining K_{d} values of 69.8 nM and 6.22 nM, respectively for chimera DBP1-P7 **(****Fig. 6D****).** These results indicate that DBP1-P7 chimera performed even better under fish farm condition, lowering 10 times its K_{d}. This affinity, in the low nM range is within the range of high-affinity commercial antibodies. These results indicate that the conditions used for the binders screening (marine or fresh water) have been successful and confirm the binding of DBP1-P7 chimera to VLPs. Later, we performed the same binding experiments in marine water with the matured chimeras DBP1-P7.C3 and DBP1-P7.C4 **(****Fig. 6E****),** obtaining affinities in the low nanomolar range (4.23-4.45 nM) achieving a little increase with regard to the parental chimera, being very similar the values found in both matured chimeras. These results are in concordance with the obtained by yeast surface display although the improvement by this latter method was much greater.

Whereas betanodaviruses have been reported to enter fish cells by clathrin-mediated endocytosis and that the maximum particle size described to follow this pathway is 200 nm (Rejman, J., et al., Biochem. J. 377, 159-169 (2004)), we next measured the hydrodynamic radius of the chimera by dynamic light scattering (DLS) in the absence and presence of VLPs. First, we studied each of the components separately. Thus, samples containing DBP1-P7, at a concentration below the Cₛₐₜ, showed a single population with a hydrodynamic radius (Rₕ) of 3.4 nm **(****Fig. 6F****),** which corresponds well on the average found for monomeric proteins. For the VLPs, a unique population of Rₕ 31.1 nm was observed, similar to the one described for other non-enveloped VLPs (Nikolai, N. et al. Comparative study of non-enveloped icosahedral viruses size. PLoS One 10, 1-11 (2015)). The incubation of the chimera and the VLPs together rendered two different populations, one represented the 62% of the total mass with a Rₕ of 317 nm while the remaining 38% had a Rₕ of 3,472 nm. Thus, these two populations had a radius 10-fold and 100-fold larger than that of the VLP_{S}. This result strongly suggests that the effective interaction between chimera and antigen triggers the formation of liquid droplets that give rise to larger structures. Therefore, we anticipate that the size of the complex liquid droplets that are being formed (317-3472 nm) should inactivate the virus infection mechanism.

In view of all these results, we conclude that all the chimeras tested DBP1-P7, DBP1-P7.C3 and DBP1-P7.C4 are appropriate candidates for the molecular trap/coating system since it is able to perform LLPS in marine water conditions at relatively low concentration, binds to betanodavirus VLPs with very high affinity and it phase separates at concentrations as low as 1 µM upon binding, which strongly suggests the existence of a local concentration effect.

### 2.5. The selected OmpF-binding chimera binds the Y. ruckeri's OmpF antigen expressed in E. coli

In order to facilitate the experimental handling of the *Y. ruckeri* antigen in a regular laboratory set up (i.e., BSL-1), we first genetically engineered a non-infective model analog of this pathogen consisting on an *E.coli* strain (MDS42) that is depleted in dispensable membrane proteins (i.e., about one third of the genome) including its endogenous OmpF and that expresses *Y. ruckeri* OmpF embedded on the membrane under an inducible promoter **(****Fig. 7A****).** We corroborated the model by analyzing the membrane expression of both OmpFwt and OmpF from *Y. ruckeri* by confocal microscopy **(****Fig. 7B****).** Since the affinities of initial OmpF binders obtained by yeast surface display were not very optimal and considering that the work with membrane proteins tends to be a complicated matter, we decided to do a series of preliminary assays to evaluate the interaction of chimera-antigen before going on to perform exhaustive studies of binding dynamics.

First, we used Biolayer interferometry (BLI) to test the binding between the immobilized chimeras and the soluble recombinant OmpF from *Y.ruckeri* at increasing concentrations of the target. The calculated affinities were 119 nM and 26 nM for chimeras DBP1-L1 and DBP1-L2 respectively **(****Fig. 7C****).** These kinetic data calculated by BLI experiments were better than those obtained for the binders alone (without the LCR of the chimera) by yeast surface titrations, but the affinities' degree were coherent between both assays. Furthermore, we also performed immunocytochemistry assays. Here, the bacteria were visualized using DAPI dye while the OmpF-embedded on the membrane and chimeras were labelled with Alexa Fluor-568 and Alexa Fluor-488, respectively. The binding was confirmed by the co-localization observed by merging images taken from these two fluorescence channels **(****Fig. 7D****).**

Once we confirmed the binding of the chimeras, we further analyzed the affinity and specificity of the interaction by flow cytometry. To that end, first we check the number of OmpF receptors expressed on the surface of the different strains tested (Fig. 8A), since the fluorescence obtained at λ=568 nm is proportional to the number of OmpF receptors, thus confirming that indeed ΔOmpF strain was defective on those.

Later, we analyzed the binding of initial four chimeras to three *E.coli* strains: one expressing OmpF from *Y. ruckeri* embedded in the membrane (selected target), one expressing wild-type OmpF (wtOmpF, set as control of specificity) and another one with the deleted OmpF (ΔOmpF, as negative control without the target) to check for unwanterd interactions. In order to set up this protocol, three concentrations of the chimeras: 0.01 µM, 0.1 µM and 1 µM and PBS buffer were used, obtaining in this way semi-quantitative data by comparing the percentage of double positive cells, which are the ones that display fluorescence for both Alexa Fluor-568 (labelling OmpF) and Alexa Fluor-488 (point the chimera), indicating the percentage of binding between both components **(****Fig. 8B****).** These data revealed, a low attraction (less than 25%) to the ΔOmpF strain, particularly evident at medium and higher concentrations of the chimera (0.1 µM and 1 µM) comparing with the binding to the specific target *(Y. ruckeri* OmpF) for which the percentage of double positives were > 75%. Regarding the specificity, we found that although there was binding to wtOmpF in all the chimeras, the percentage was lower than that of the specific target at least in the mHP1α-formed chimeras. Since the minimum concentration for phase separation (Cₛₐₜ) in fresh water of the previously analyzed chimeras was much higher in those formed by DBP1 LCR and their binding capacities were not better than those reported for chimeras containing mHP1α, the former were discarded as chimeras to block *Y. ruckeri* pathogen.

Regarding specificity, we found that the strain expressing wtOmpF had a higher level of receptors than the transgenic OmpF *Y. ruckeri* strain, thus to analyze the affinity and specificity of the best fresh water-chimeras, mHP1α-L1 and mHP1α-L2) we normalized the data based on the number of receptors. These results showed than although both chimeras displayed almost none binding to ΔOmpF strain and low binding to wtOmpF, mHP1α-L1 displayed higher affinity and specificity than mHP1α-L2 to the specific target **(**Fig. 8C-D). Later, we performed individual titrations in PBS of the recently developed chimeras (mHP1α-R2, mHP1α-R2.C6 and mHP1α-R2.C14). To that end, a total of 5 concentrations (from 0.001 to 3 µM) were analyzed and normalized, obtaining affinity values between 0.075 µM and 2.33 µM for OmpF *Y. ruckeri* and between 0.082 µM and 0.39 µM for wtOmpF **(**Fig. 8E-F). Thus, the chimera containing the R2 binder showed the best affinities. In this case maturation process performed by yeast surface display did not improved the affinity

To test the binding capacity in real fish farm conditions, we performed individual titrations of the best performance chimeras (mHP1α-L1 and mHP1α-R2) in fresh water towards both OmpF expressing strains **(**Fig. 8G-H). Results showed affinities in the low micromolar range (0.14 and 0.3 µM respectively) for OmpF *Y. ruckeri* similar to the affinities found for wtOmpF (0.13 µM and 0.28 µM respectively). Thus, both chimeras possess similar affinity towards both OmpFs. The kinetic values obtained by the chimeras were in the same range as those obtained by individual binders by yeast surface display, since membrane-integrated OmpF is a trimer exposing exclusively some motifs of the protein whereas recombinant OmpF use during binder screening could exposed other regions or adopts different conformations at it is not embedded.

The infection of *Y. ruckeri* to fish goes *via* phagocytosis. The bacteria interact *via* integrins with the host cells, and then trigger the phagocytic process. We hypothesize that the binding of the chimeras to the OmpF receptors exposed on the bacterium would coat its surface and could act as a protective shield around *Y. ruckeri* blocking or hindering the infection. Furthermore, since many of the virulence factors of Y. *ruckeri* produce proteins localized in the outer membrane, those would likely be also blocked by this chimera.

### 2.6. Cytotoxicity of chimeras in human neuroblastoma cells

In order to get a first assessment on whether the selected chimeras could have any toxicity on humans we performed exposure time-course experiments using the neuroblastoma cell line SH-SY5Y. Several concentrations of each chimera were tested using two control chimeras (containing a binder unrelated to the target) and DBP1-P7 chimera.

In these assays, a molecule is considered toxic whether it lowers cell viability by more than a 25% **(Table 5).** According to this criterium, we only found toxicity at higher concentrations of the tested chimeras after 48 h exposure. However, the putative local concentration effect observed for DBP1-P7 chimera, showed that phase separation occurs at lower concentrations (1.5 µM), for which no there is no toxicity and the high affinity reported for the binders, could further indicate that working conditions of the chimera will not be toxic.

**Table 5. Toxicity tests performed in cell line SH-SY5Y exposed to the chimeras for 24h and 48h. Cell viability refers to the quantification of the number of living cells, expressed as a percentage with respect to the control not exposed to chimera by the CellTiterGlo assay.**

| Control chimera mhp1α | | | Control chimera DBP1 | | | DBP1-P7 | | |
|---|---|---|---|---|---|---|---|---|
| [chimera] | Cell viability 24h | Cell viability 48h | [chimera] | Cell viability 24h | Cell viability 48h | [chimera] | Cell viability 24h | Cell viability 48h |
| 15 µM | ↓ 19% | ↓ 26 %* | 8 µM | ↓ 20 % | ↓ 41 %* | 4.6 µM | ↓23.9 % | ↓ 30%* |
| 5 µM | ↓ 16.5 % | ↓ 24 % | 4.6 µM | ↓ 5.5 % | ↓ 24 % | 2.6 µM | ↓ 6.1 % | No toxicity |
| 1 µM | ↓ 3.4% | ↓ 1.3% | 2.6 µM | ↓ 13.5% | ↓ 1.75% | 1.5 µM | ↓ 5.4% | No toxicity |

### 2.7. Physical properties of phosphorylated HP1α hydrogels

In order to develop a formulation of the chimera that could be used not only in open system aquaculture (OSA) but also in close system aquaculture (CSA), a hydrogel chimera (PathogelTrap Filter) was produced. Hydrogels are highly dynamic systems that can be reversibly assembled and disassembled in response to some environmental condition, such as phosphorylation of a protein undergoing phase separation. Thus, we used phosphorylated (HP1αPhos; in particular, phosphorylated amino acid sequence SEQ ID NO: 3).

The hydrogels were produced following an evaporation method from 180 µM to 1.2 mM and from 15 to 30 ºC. Regarding the concentration, the higher amount of chimera, the larger was the amount of hydrogel obtained and it was more solid. The appearance of hydrogels was more whitish above 300 µM **(****Fig. 9****)** while at lower concentrations they appeared transparent stopped forming bulk hydrogels and instead, a new type of hydrogel was deposited on top of the walls of the microtube as thin film, almost unnoticed to the naked eye. This later observation is in contrast with a previous report where hydrogels only started to form at concentrations higher than 800 µM and with a much higher degree of protein purity (Larson, A. G. et al. Phase Separation in Heterochromatin. Nature Publishing Group 547, 236-240 (2017).

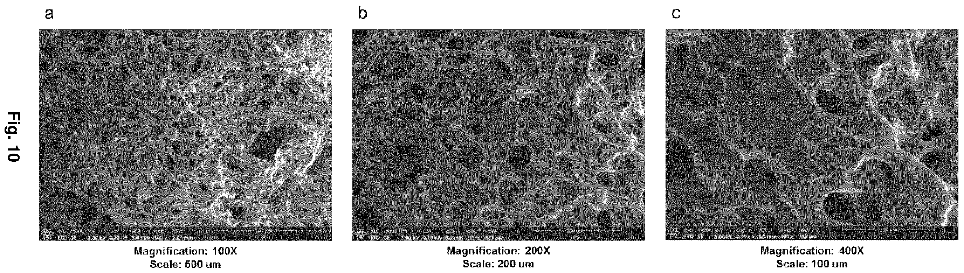

Upon the lyophilization of the sample, a SEM characterization on high vacuum, **(****Fig. 10****)** shows a range of pore sizes between 14 and 80 µm. It must be noted that, although for some authors hold that the pore size of hydrogels usually changes after a freeze-drying process and therefore TEM and SEM techniques would not reliably represent the real structure of the hydrogel when it is swollen, these are still commonly accepted techniques for a first characterization of those structures (De France, K. J., et al., T. Structured Macroporous Hydrogels: Progress, Challenges, and Opportunities. Adv. Healthc. Mater. 7, 1-17 (2018); Hermansson, A. M. & Buchheim, W. Characterization of Protein Gels by Scanning and Transmission Electron Microscopy. J. Colloid Interface Sci. 81, 519-530 (1981)). Interestingly, we found quite homogeneity of the porous structures in each of the samples, a feature that seems to be independent of the initial chimera concentration and temperature used for hydrogel formation. This highly contrasts with the solid appearance of the hydrogels were the concentration of chimera was highest.

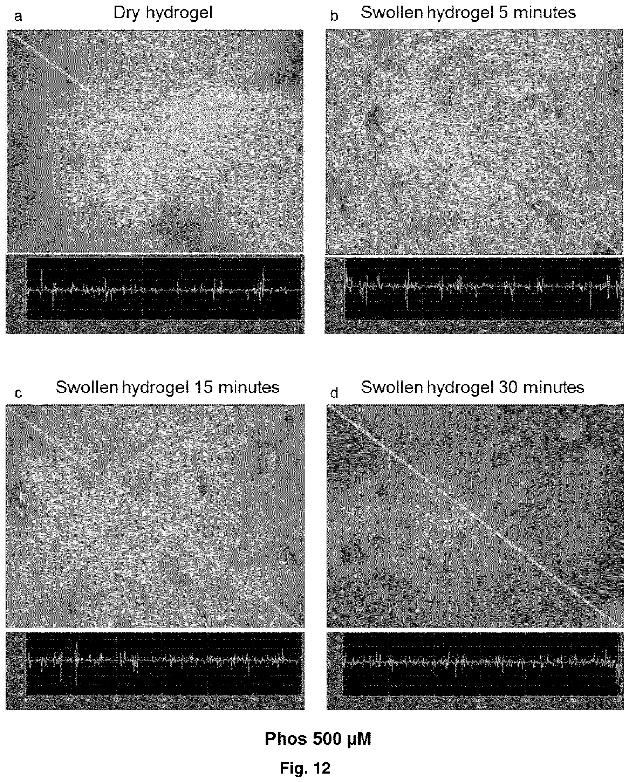

To further characterize the properties of the hydrogel we performed environmental scanning electron microscopy (ESEM, **Fig. 11****)** and surface metrology **(****Fig. 12****)** to test the swelling capabilities of these gels once they were freeze-dried.

In surface metrology different swelling times were taken to determine the surface roughness of the hydrogel during the swelling process. The results of both techniques showed the full swelling capacity of these hydrogels, showing that they can be stored dry without losing their water absorption and retention capacity.

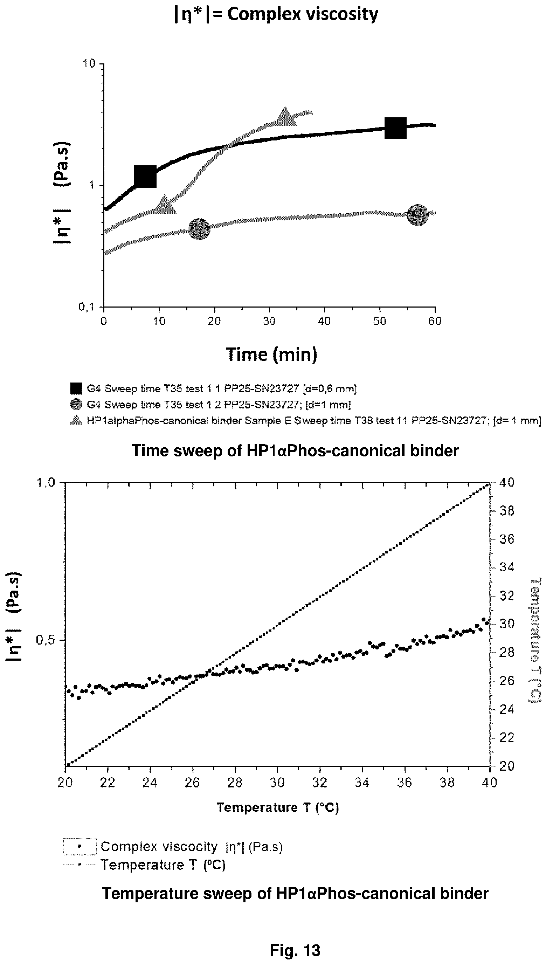

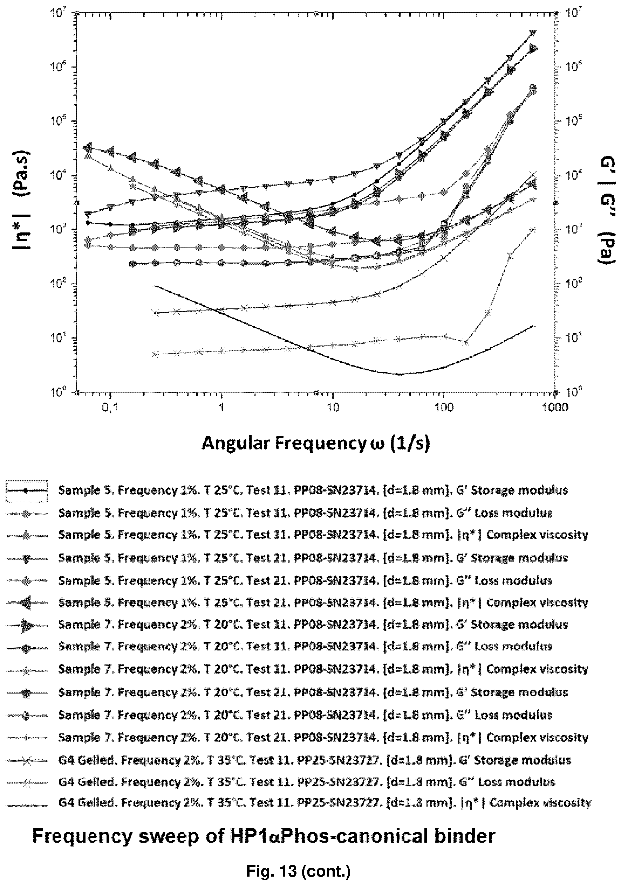

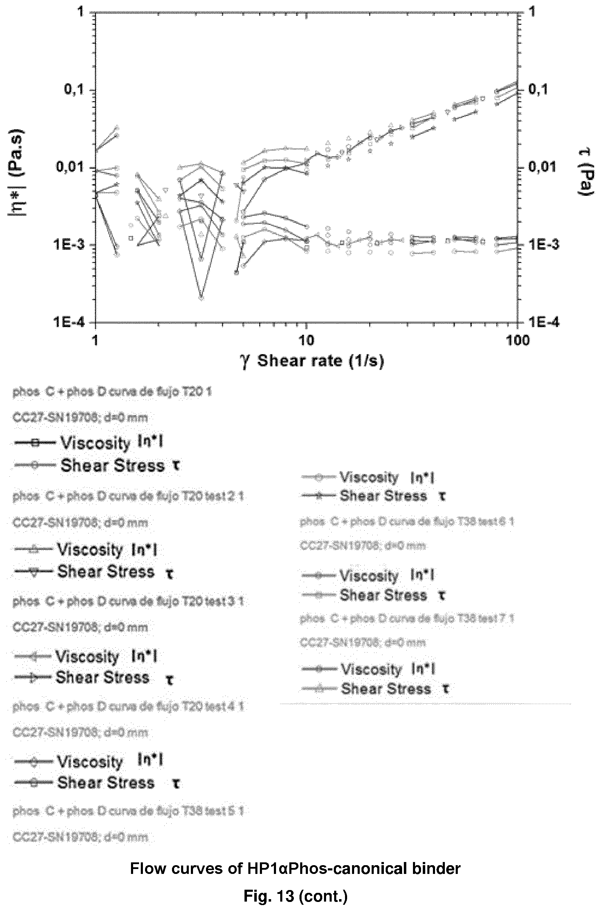

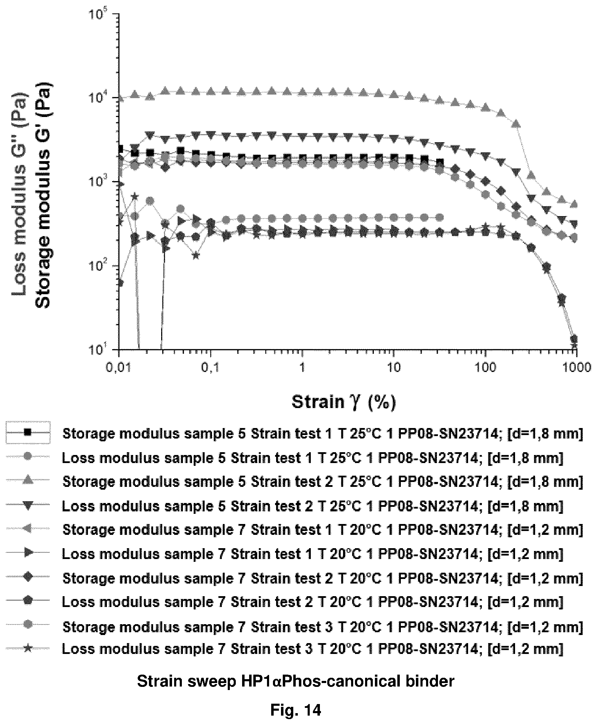

Finally, in order to determine ideal gelation temperature, a temperature sweep measurement was performed (Fig. 13A). These results showed a gelation event only in the more concentrated samples (over 800 µM). At lower concentrations the high temperatures are not working well. According to the time sweep to determine the ideal gelation time, one rise in the signal shows a gelation event close to 15 minutes for more concentrated samples of Phos-chimera (900 µM) at 38ºC. Gel formation is slower at lower concentrations of Phos-chimera, even at high temperatures (35ºC and 38ºC) (Fig.13B). Similar behavior occurs in all concentrations at lower temperatures, 25ºC. Regarding the linear stress **(****Fig. 14****),** the maximum deformation of the material occurs at about the 100 % when the material is starting to breakage. Hydrogels obtained at high temperatures (35°C and 38°C) were more brittle. G' (squares) over G" (triangles) is typical solid performance. This behavior was common for all hydrogels.

### 2.8. Phosphorylated HP1α can form thin hydrogel films on commercial scaffolds

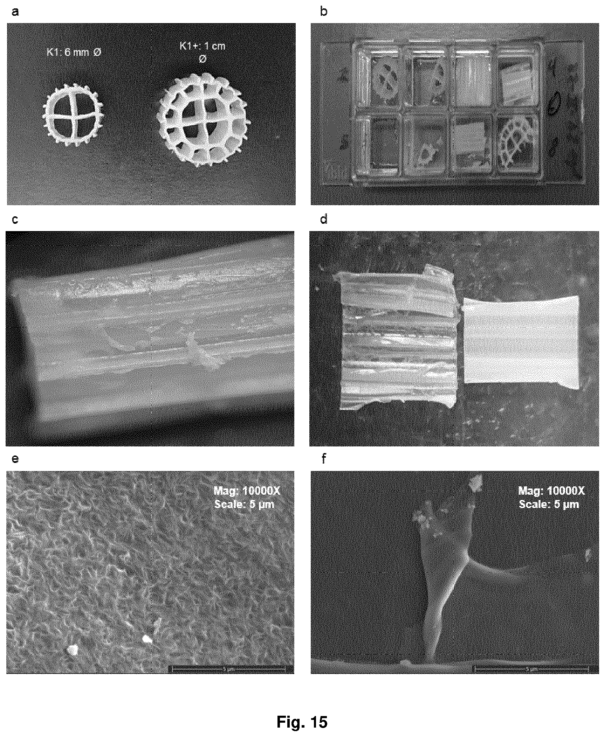

In order to use this hydrogel in both CSA y OSA with maximal surface coating we next de decided to functionalize kaldnes scaffolds, a popular commercial substrate commonly used for filtering systems in aquaculture and that is made on virgin high-density polyethylene (v-HDPE) **(****Fig. 15A****).** Two methods were used to coat the surface of the kaldnes devices. The first one consisted in dipping pieces of kaldnes into a solution of the chimera of known concentration and incubating them at room temperature in a humid chamber for 24 hours to prevent evaporation of the chimera solvent. The second one is an evaporation method, where the kaldnes is immersed in the chimera solution and then left in the open air for 24 hours so that a constant and slow evaporation of the solvent takes place concentrating the chimera, which gets fixed as a film on the surface of the v-HDPE substrate **(****Fig. 15B****).**

In the dipping method we could not achieve a proper coating of the material while the evaporation strategy got good coatings on the v-HDPE **(****Fig. 15** **C, D)** The characterization of these coatings by optical and SEM showed thin films being formed on top of kaldnes with the later method **(****Fig. 15** **E, F).**

### 2.9. Thin hydrogel films of HP1αPhos-P7 chimeras can effectively trap pathogens

*In vitro* HP1αPhos-P7 hydrogel-VLPs interaction assays were performed using an antibody-mediated viral particle detection method. ELISA-like experiments consisted of immersing kaldnes scaffolds, previously functionalized with HP1αPhos-P7 hydrogel, in 500 µL of known concentrations of VLPs from betanodavirus RGNNV in seawater. After 45 minutes, each kaldnes were removed from each solution and 100 µL of the solution were immobilized on the wells of ELISA plates. Also, 100 µL of VLPs solution not exposed to the functionalized kaldnes were immobilized as the untreated sample. In both cases 250 nM, 125 nM, 20 nM, 10 nM and 5 nM concentrations of VLPs were used. The assays were repeated three times for each concentration. The idea was to determine the percentage retention of VLPs based on the difference between the absorbance (in ELISA arbitrary units) before and after exposure of the solutions to the functionalized kaldnes.

Once the incubations were carried out a statistical treatment of the data was performed.

A one-way Anova is performed to test our null hypothesis.

H0: There is not entrapment of VLPs by HP1α-P7 hydrogel functionalized kaldnes. (All group means are equal).

HA: There is entrapment of VLPs by specific functionalized scaffold. (At least one group mean is different from the rest).

We then analyzed two main values (Table 6), the F-statistic and the p-value. The F-statistic is the ratio of the mean square's treatment to the mean squares error. In other words, the F-statistic is the variation between sample means / variation within samples. In this way, the larger the F-statistic, the greater the evidence that there is a difference between the group means. On other hand, another value to evaluate is the p-value. If it is lower than α = 0.05, we reject the null hypothesis of the ANOVA and therefore one can conclude that there is a statistically significant difference between the means of the samples. Besides, the F critical value is a specific value you compare your F-statistic to. In general, if your calculated F value in a test is larger than your F critical value, you can reject the null hypothesis.

**Table 6. Anova single factor data. To emphasize the larger F-statistic and the P-value less than α = 0.05. SS: Sum of Squares. df: degree of freedom. MS: Means Squares. F crit: F critical value.**

| | | | | | | |
|---|---|---|---|---|---|---|
| Anova: | | | | | | |
| Single factor | | | | | | |

| SUMMARY | | | | | | |
|---|---|---|---|---|---|---|
| *Groups* | *Count* | *Sum* | *Average* | *Variance* | | |
| Column 1 | 6 | 1,194333 | 0,199055 | 0,000381 | | |
| Column 2 | 6 | 0,936333 | 0,156055 | 0.001763 | | |

| ANOVA | | | | | | |
|---|---|---|---|---|---|---|
| *Source of Variation* | SS | Df | MS | F | P-value | F crit |
| Between Groups | 0.005547 | 1 | 0,005547 | 5,170278 | 0,046269 | 4,964603 |
| Within Groups | 0,010728 | 10 | 0,001072 | | | |
| Total | 0,016275 | 11 | | | | |

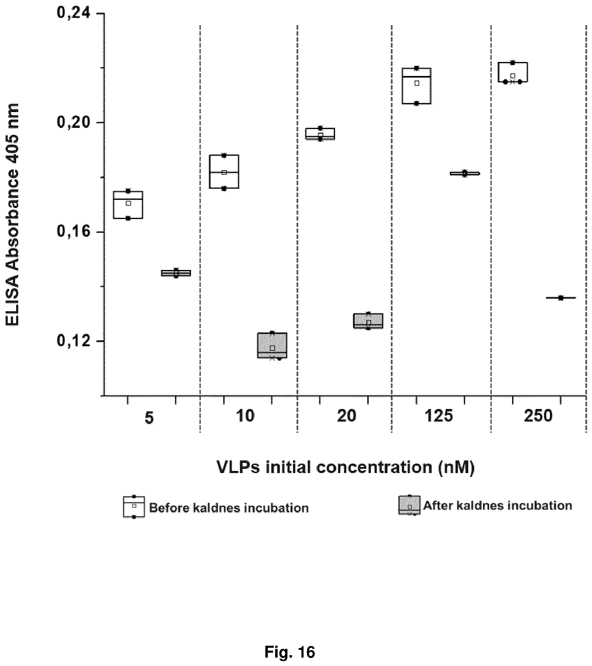

The boxplots **(****Fig. 16****)** of both conditions, before and after kaldnes incubation into the solution, give us a more illustrative information. It is evident that hydrogel coatings are capable of trapping VLPs regardless of their initial concentration.

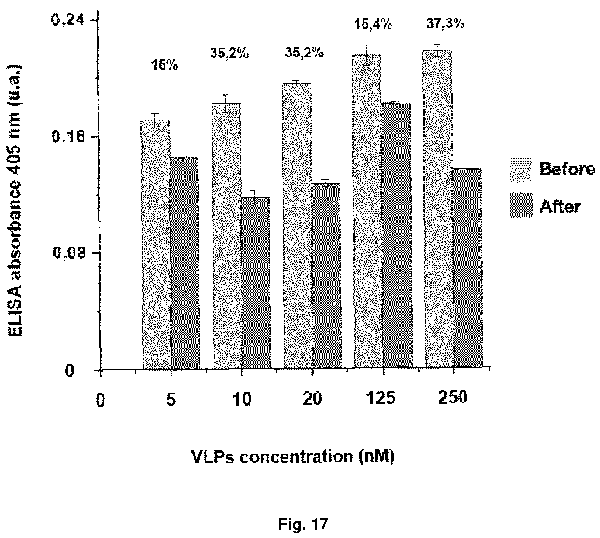

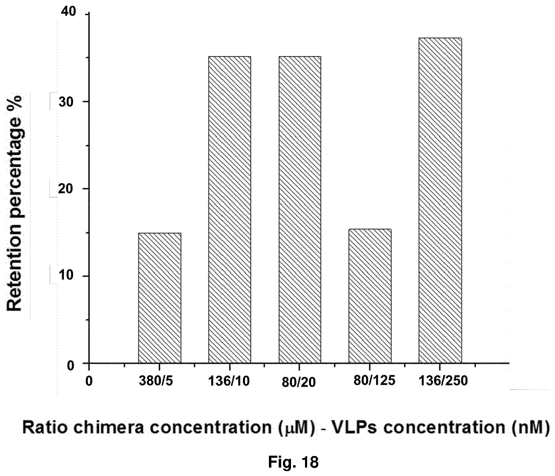

Percentage of retention and the ratio between hydrogel concentration and VLPs concentration are shown in **Fig. 17** and **Fig. 18****,** respectively. In general, we can conclude that the best chimera concentration for hydrogel formation lays between 100 and 150 µM. This is the one that gives the best trapping response at both high and low VLPs concentrations.

### 3. Conclusions

Taken together, the results show that for the close system aquaculture (CSA) several chimeras per target antigen were obtained (DBP1-P7.C3 and DBP1-P7.C4 for betanodavirus and mHP1-L1 and mHP1-R2 for *Y. ruckeri*), which are able to phase separate at low concentrations and with high affinity (in the case of the virus) or reasonable affinity (for the bacterium) towards targeted pathogens in real fish farm conditions.

Based on the results from the tests performed on model pathogens, both chimeras are able to hamper the infectious mechanisms of the pathogens upon binding.

For the open system aquaculture (OSA, but also for the CSA) specifically it has been shown that the phosphorylation of the mHP1α chimera allows the formation of hydrogels for which protein concentration and temperature are important parameters although they do not seem to alter the porous architecture of the gel. Using an evaporation protocol, these hydrogels can be produced as think films on top of commercial kaldnes scaffolds, which trap the corresponding specific pathogens as water flows over these devices.

The results show that the two chimera formulations developed (in solution for CSA and hydrogel for both OSA and CSA) are able to effectively trap the specific pathogens for which they were designed in real fish farm water (both marine and fresh) and should be able to prevent fish infections in fish farm. This opens the door to this disruptive technology for the effective control of pathogens in aquaculture.

## Claims

1. A chimeric protein comprising:
- a first polypeptide which comprises a low-complexity region (LCR), preferably wherein said first polypeptide is DBP1 or HP1α; and
- a second polypeptide which comprises a recognition region for a pathogen.

2. The chimeric protein according to claim 1, wherein the second polypeptide comprises a recognition region for an aquatic pathogen, preferably an aquaculture pathogen.

3. The chimeric protein according to claim 2, wherein the aquaculture pathogen is a *betanodavirus* or a *Yersinia ruckeri* sp.

4. The chimeric protein according to any one of claims 1 to 3, wherein the second polypeptide comprises an amino acid sequence with a sequence similarity of, at least, 80% to an amino acid sequence selected from the list consisting of SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 30, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO:53, SEQ ID NO: 54, SEQ ID NO: 55, SEQ ID NO: 56, SEQ ID NO: 57 and SEQ ID NO: 58, wherein sequence similarity is computed according algorithm EMBOSS Needle using the BLOSUM62 matrix, having a Gap Open penalty of 10.0 and a Gap Extend penalty of 0.5.

5. The chimeric protein according to any one of claims 1 to 4, wherein the first polypeptide is DBP1 and the second polypeptide comprises an amino acid sequence with a sequence similarity of, at least, 80% to an amino acid sequence selected from the list consisting of SEQ ID NO: 14, SEQ ID NO: 42, SEQ ID NO: 15, SEQ ID NO: 43, SEQ ID NO: 17, SEQ ID NO: 45, SEQ ID NO: 28, SEQ ID NO: 56, SEQ ID NO: 29, and SEQ ID NO: 57, wherein sequence similarity is computed according algorithm EMBOSS Needle using the BLOSUM62 matrix, having a Gap Open penalty of 10.0 and a Gap Extend penalty of 0.5.

6. The chimeric protein according to any one of claims 1 to 4, wherein the first polypeptide is and the second polypeptide comprises an amino acid sequence with a sequence similarity of, at least, 80% to an amino acid sequence selected from the list consisting of SEQ ID NO: 14, SEQ ID NO: 42, SEQ ID NO: 15, SEQ ID NO: 43, SEQ ID NO: 17, SEQ ID NO: 45, SEQ ID NO: 28, SEQ ID NO: 56, SEQ ID NO: 29, and SEQ ID NO: 57, wherein sequence similarity is computed according algorithm EMBOSS Needle using the BLOSUM62 matrix, having a Gap Open penalty of 10.0 and a Gap Extend penalty of 0.5.

7. The chimeric protein according to any one of claims 1 to 4, wherein the first polypeptide is a phosphorylated HP1α, preferably wherein comprises an amino acid sequence with a sequence similarity of, at least, 80% to SEQ ID NO: 3, and the second polypeptide comprises an amino acid sequence with a sequence similarity of, at least, 80% to an amino acid sequence selected from the list consisting of SEQ ID NO: 5, SEQ ID NO: 33, SEQ ID NO: 14, SEQ ID NO: 42, SEQ ID NO 17, and SEQ ID NO: 45, wherein sequence similarity is computed according algorithm EMBOSS Needle using the BLOSUM62 matrix, having a Gap Open penalty of 10.0, a Gap Extend penalty of 0.5.

8. A polynucleotide comprising a nucleotide sequence encoding the chimeric protein according to any one of claims 1 to 7.

9. A host cell comprising the chimeric protein according to any one of claims 1 to 7, or the polynucleotide according to claim 8.

10. A composition comprising the chimeric protein according to any one of claims 1 to 7.

11. A hydrogel comprising the chimeric protein according to 7, or the composition according to claim 10, when said composition comprises the chimeric protein according to claim 7.

12. A filter system comprising the hydrogel according to claim 11 and a scaffold material, wherein the hydrogel is arranged on the surface of the scaffold material.

13. A kit comprising the chimeric protein according to any one of claims 1 to 7, the composition according to claim 10, the hydrogel according to claim 11, or the filter system according to claim 12.

14. Use of the chimeric protein according to any one of claims 1 to 7, the polynucleotide according to claim 8, the host cell according to claim 9, the composition according to claim 10, the hydrogel according to claim 11, the filter system according to claim 12 or the kit according to claim 13, for pathogen control in a system, preferably wherein the system is an aquatic system, or in a sample, preferably wherein the pathogen is an aquaculture pathogen.

15. Use of the chimeric protein according to any one of claims 1 to 7, the polynucleotide according to claim 8, the host cell according to claim 9, the composition according to claim 10, the hydrogel according to claim 11, the filter system according to claim 12, or the kit according to claim 13, for *in vitro* pathogen detection in a sample, preferably wherein the pathogen is an aquaculture pathogen.
